(19) Europäisches Patentamt
European Patent Office
Office européen des brevets

(11) EP 3 978 078 A1

(12) **EUROPEAN PATENT APPLICATION**
published in accordance with Art. 153(4) EPC

(43) Date of publication:
**06.04.2022 Bulletin 2022/14**

(21) Application number: **20814608.4**

(22) Date of filing: **29.05.2020**

(51) International Patent Classification (IPC):
***A61P 35/00*** *(2006.01)* ***A61K 47/10*** *(2017.01)*
***A61K 47/24*** *(2006.01)* ***A61K 47/65*** *(2017.01)*
***A61K 38/16*** *(2006.01)*

(52) Cooperative Patent Classification (CPC):
**A61K 38/16; A61K 47/10; A61K 47/24; A61K 47/65; A61P 35/00**

(86) International application number:
**PCT/JP2020/021301**

(87) International publication number:
**WO 2020/241819 (03.12.2020 Gazette 2020/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.05.2019 JP 2019100395**

(71) Applicants:
- **Tokyo Institute of Technology**
  **Tokyo 152-8550 (JP)**
- **Kawasaki Institute of Industrial Promotion**
  **Kawasaki-shi, Kanagawa 212-0013 (JP)**

(72) Inventors:
- **NISHIYAMA Nobuhiro**
  **Tokyo 152-8550 (JP)**
- **HONDA Yuto**
  **Tokyo 152-8550 (JP)**
- **NOMOTO Takahiro**
  **Tokyo 152-8550 (JP)**
- **TAKEMOTO Hiroyasu**
  **Tokyo 152-8550 (JP)**
- **MATSUI Makoto**
  **Tokyo 152-8550 (JP)**
- **KINOH Hiroaki**
  **Kawasaki-shi, Kanagawa 212-0013 (JP)**
- **KATAOKA Kazunori**
  **Kawasaki-shi, Kanagawa 212-0013 (JP)**
- **LIU Xueying**
  **Kawasaki-shi, Kanagawa 212-0013 (JP)**
- **DIRISALA Anjaneyulu**
  **Kawasaki-shi, Kanagawa 212-0013 (JP)**

(74) Representative: **Nederlandsch Octrooibureau**
**P.O. Box 29720**
**2502 LS The Hague (NL)**

(54) **COMPLEX, MEDICINE, THERAPEUTIC AGENT FOR CANCER, KIT AND CONJUGATE**

(57) A complex is provided comprising a conjugate in which a polymer having a boronic acid group and a compound having a diol structure are bonded, and a substance that is complexed with the conjugate.

EP 3 978 078 A1

FIG. 1

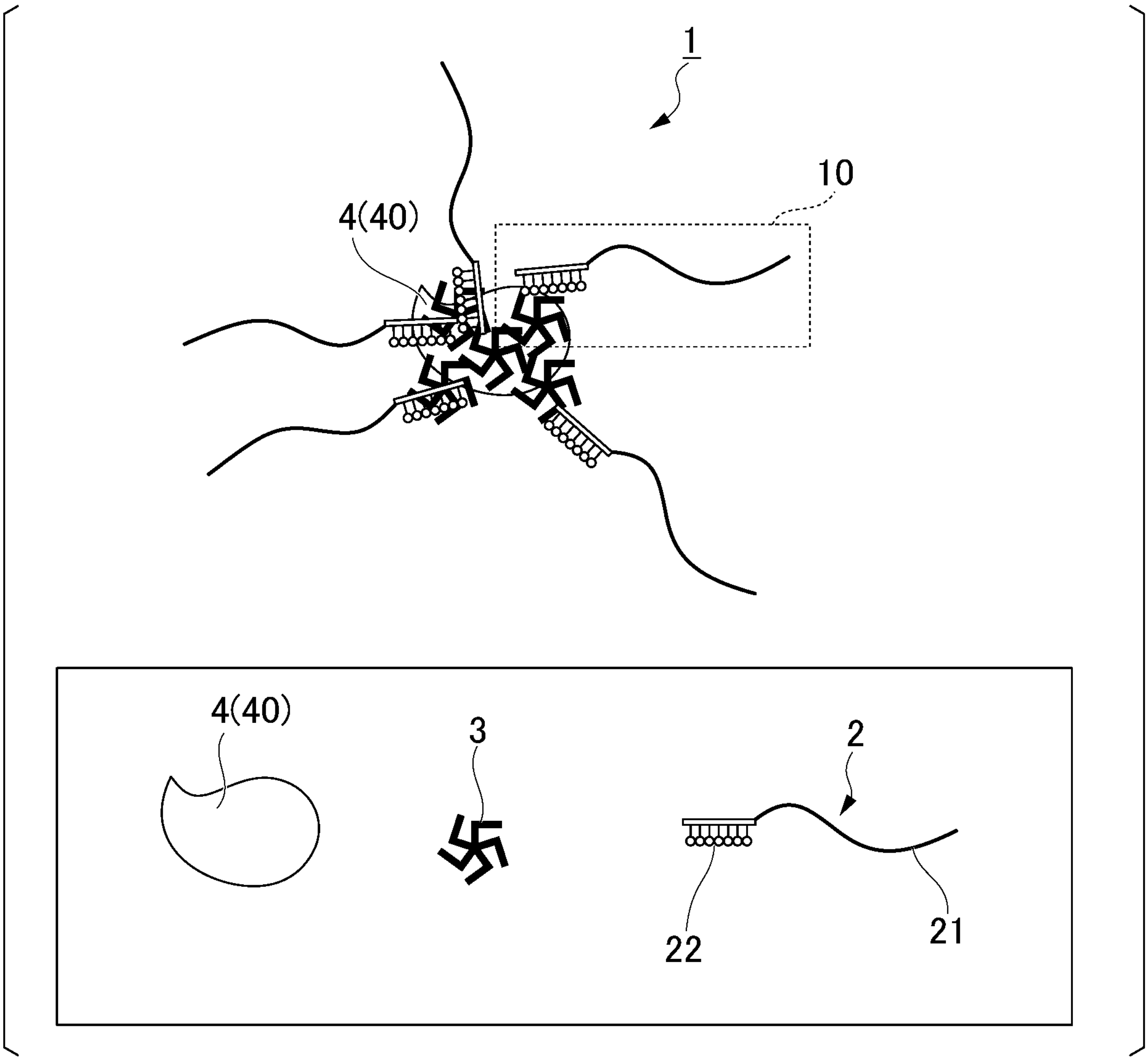

## Description

[Technical Field]

The present invention relates to a complex, a medicine, a therapeutic agent for cancer, a kit, and a conjugate.

Priority is claimed on Japanese Patent Application No. 2019-100395, filed May 29, 2019, the content of which is incorporated herein by reference.

[Background Art]

Biomedical products and other physiologically active proteins are highly expected as epoch-making therapeutic agents for intractable diseases such as cancer. However, proteins of which the size is smaller than the filtration threshold of renal glomeruli have poor blood retention since they are rapidly excreted from the body, and their stability in blood is not always sufficient to undergo enzymatic degradation in the blood. The reality is that the expected pharmacological effects have not been obtained from such proteins. Further, for the application of physiologically active proteins to cancer treatment, it may be required for the physiologically active proteins to be selectively accumulated in a tumor.

In order to improve blood retention and stability in blood, a PEG-modified protein that is chemically modified with a polyethylene glycol (PEG), which is a biocompatible polymer, has been clinically applied, and a certain effect has been obtained. However, there may be concerns that such PEG modification reduces the pharmacological activity.

In addition, Non-Patent Document 1 discloses a technique in which an antibody is used as a physiologically active protein, and an amino group contained in the antibody is modified with a negatively charged pH-responsive molecule to subsequently form a polyion complex (PIC) with a positively charged polymer compound. According to the above, it is said that the pH-responsive molecule is dissociated at the intracellular pH and the PIC disintegrates, whereby the antibody can be released specifically inside the cell.

On the other hand, a method of forming a complex with another molecule without chemically modifying the protein is also known.

Non-Patent Document 2 discloses a technique of encapsulating a protein in a catechol structure-introduced polymer. As a molecule having a catechol structure, for example, tannic acid is known.

It is known that tannic acid can bond to proteins to form a complex by hydrophobic interaction and hydrogen bonding (Non-Patent Documents 3 and 4). By bonding tannic acid to a protein or the like, it is possible to form a complex without utilizing chemical modification.

[Citation List]

[Non-Patent Documents]

[Non-Patent Document 1]
"Intracellular Delivery of Charge-Converted Monoclonal Antibodies by Combinatorial Design of Block/Homo Polyion Complex Micelles", A. Kim, Y. Miura, T. Ishii, O. F. Mutaf, N. Nishiyama, H. Cabral, and K. Kataoka, Biomacromolecules, 17(2), 446-453 (2016).
[Non-Patent Document 2]
"Self-assembled micellar nanocomplexes comprising green tea catechin derivatives and protein drugs for cancer therapy", J. E. Chung, S. Tan, S. J. Gao, N. Yongvongsoontorn, S. H. Kim, J. H. Lee, H. S. Choi, H. Yano, L. Zhuo, M. Kurisawa, and J. Y. Ying, Nat. Nanotech. 9 (11), 907-912(2014).
[Non-Patent Document 3]
"Formation of complexes between protein and tannic acid" J. P. Van Buren, and W. B. Robinson, J. Agric. Food Chem. 17(4), 772-777 (1969).
[Non-Patent Document 4]
"Gallic acid: Molecular rival of cancer" V. Sharad, S. Amit, and M. Abha, Env. Tox. and pharm. 35 (3), 473-485 (2013).

[Summary of Invention]

[Technical Problem]

However, in the method of Non-Patent Document 1, since the antibody is chemically modified, there is a concern that the pharmacological activity of the antibody may be decreased as in the case of the PEG modification.

In addition, there is no report that the complexation of the protein described in Non-Patent Documents 2 to 4 improved the blood retention and stability of the protein in blood and exhibited tumor accumulation.

The present invention has been made to solve the above-described problems, and an object of the present invention is to provide a complex having excellent blood retention and pH responsiveness.

[Solution to Problem]

That is, the present invention has the following aspects.

<1> A complex comprising:

a conjugate in which a polymer having a boronic acid group is bonded to a compound having a diol structure; and a substance that is complexed with the conjugate.

<2> The complex according to <1>, wherein the substance that is complexed with the conjugate is at least one selected from the group consisting of a protein, a virus, an inorganic particle, a nucleic acid, and a small molecule medicine.

<3> The complex according to <1> or <2>, wherein the complex comprises the conjugate in which a polymer having a boronic acid group is bonded to a compound having a diol structure, and a protein.

<4> The complex according to any one of <1> to <3>, wherein the compound having a diol structure is a polyphenol.

<5> The complex according to any one of <1> to <4>, wherein the compound having a diol structure is at least one selected from the group consisting of tannic acid, gallic acid, and derivatives thereof.

<6> The complex according to any one of <1> to <5>, wherein the polymer has two or more boronic acid groups.

<7> The complex according to any one of <1> to <6>, wherein the boronic acid group is a phenylboronic acid group which may have a substituent or a pyridylboronic acid group which may have a substituent.

<8> The complex according to any one of <1> to <7>, wherein the boronic acid group is a phenylboronic acid group represented by General Formula (I) or a pyridylboronic acid group represented by General Formula (II):

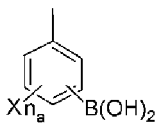

(I)

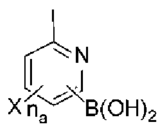

(II)

(in the formulae, X represents a halogen atom or a nitro group, and na is an integer of 0 to 4).

<9> The complex according to any one of <1> to <8>, wherein the polymer is at least one biocompatible polymer selected from the group consisting of a polyethylene glycol, an acrylic resin, a polyamino acid, a polyvinylamine, a polyallylamine, a polynucleotide, a polyacrylamide, a polyether, a polyester, a polyurethane, a polysaccharide, and copolymers thereof.

<10> The complex according to any one of <1> to <9>, wherein the polymer having a boronic acid group contains a first biocompatible polymer chain and a second biocompatible polymer chain that is different from the first biocompatible polymer chain.

<11> The complex according to <10>, wherein the second biocompatible polymer chain is a polyamino acid, and the boronic acid group is introduced into a side chain of the polyamino acid.

<12> The complex according to <10> or <11>, wherein the first biocompatible polymer chain is a polyethylene glycol.

<13> The complex according to any one of <10> to <12>, wherein the polymer having a boronic acid group contains a structure represented by General Formula (1) or (1-1),

$$A-L-B \quad (1) \qquad A-B \quad (1-1)$$

(in Formulae (1) and (1-1), A represents the first biocompatible polymer chain; L represents a linker part; and B represents the second biocompatible polymer chain having a boronic acid group and includes a repeating structure represented by the following (b2), or a repeating structure represented by (b1) and the repeating structure represented by (b2)),

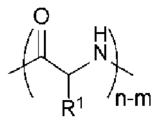
(b1)

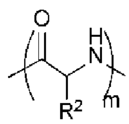
(b2)

(in Formulae (b1) and (b2), $R^1$ represents an amino acid side chain, $R^2$ is a structure in which the boronic acid group is introduced into an amino acid side chain, and n represents the total number of (b1) and (b2), n is an integer of 1 to 1,000, m is an integer of 1 to 1,000 (here, $m \leq n$), in a case where n - m is 2 or more, a plurality of $R^1$'s may be the same or different from each other, and in a case where m is 2 or more, a plurality of $R^2$'s may be the same or different from each other).

<14> The complex according to any one of <1> to <13>, wherein an average particle diameter of the complex determined by dynamic light scattering (DLS) or fluorescence correlation spectroscopy (FCS) is 5 nm or more and 200 nm or less.
<15> The complex according to any one of <1> to <14>, wherein a number average molecular weight of the polymer having a boronic acid group is 2,000 to 200,000.
<16> A medicine containing:
the complex according to any one of <1> to <15> as an active ingredient.
<17> A therapeutic agent for cancer, comprising:
the complex according to any one of <1> to <16> as an active ingredient.
<18> A kit comprising:
a polymer having a boronic acid group; and a compound having a diol structure.
<19> A conjugate in which a polymer having a boronic acid group is bonded to a compound having a diol structure.

[Advantageous Effects of Invention]

According to the present invention, it is possible to provide a complex having excellent blood retention and further having pH responsiveness.

[Brief Description of Drawings]

Fig. 1 is a schematic diagram showing an example of a schematic configuration of a complex of an embodiment.
Fig. 2 is a schematic diagram showing an example of a configuration of the complex of the embodiment in vivo.
Fig. 3 is a GPC curve of PEG-PLys$(TFA)_{20}$ prepared in Example.
Fig. 4 is a GPC curve of PEG-PLys$(TFA)_{40}$ prepared in the example.
Fig. 5 is a $^1$H NMR spectrum of PEG-$PLys_{20}$ prepared in Example.
Fig. 6 is a $^1$H NMR spectrum of PEG-$PLys_{40}$ prepared in Example.
Fig. 7 is a $^1$H NMR spectrum of PEG-P$[Lys(FPBA)_{10}]_{20}$ prepared in Example.
Fig. 8 is a $^1$H NMR spectrum of PEG-P$[Lys(FPBA)_{20}]_{40}$ prepared in Example.
Fig. 9 is a GPC curve of PEG-P$[Lys(FPBA)_{10}]_{20}$.
Fig. 10 is a GPC curve of PEG-P$[Lys(FPBA)_{20}]_{40}$.
Fig. 11 is a $^1$H NMR spectrum of PEG-FPBA.
Fig. 12 is a GPC curve of PEG-FPBA.
Fig. 13 is an FP spectrum of PEG-P$[Lys(FPBA_{10}/Cy5)]_{20}$.
Fig. 14 is a graph showing the particle diameter measurement results of GFP, GFP/TA, and a GFP/TA/boronic acid-introduced polymer.
Fig. 15 is a graph showing the particle diameter measurement results of GFP, GFP/TA, GFP/PEG-P$[Lys(FPBA)_{10}]_{20}$, and GFP/TA/PEG-P$[Lys(FPBA)_{10}]_{20}$.
Fig. 16 a graph showing the particle diameter measurement results of GFP/TA/PEG-P$[Lys(FPBA)_{10}]_{20}$ in a glucose solution.
Fig. 17 a graph showing the particle diameter measurement results of GFP/TA/PEG-P$[Lys(FPBA)_{10}]_{20}$ in an FBS solution.
Fig. 18 a graph showing the particle diameter measurement results of GFP/TA/PEG-P$[Lys(FPBA)_{10}]_{20}$ at various pH values.

Fig. 19 is a confocal microscope observation image showing the intracellular distribution of GFP/TA/PEG-P[Lys(FP-BA)$_{10}$]$_{20}$.

Fig. 20 is a graph showing the results of comparing blood retention between GFP, GFP/TA, and GFP/TA/PEG-P[Lys(FPBA)$_{10}$]$_{20}$ in a CT26 subcutaneous tumor model mouse.

Fig. 21 is a graph showing the results of comparing tumor accumulation between GFP, GFP/TA, and GFP/TA/PEG-P[Lys(FPBA)$_{10}$]$_{20}$ in a CT26 subcutaneous tumor model mouse.

Fig. 22 is a graph showing the results of comparing the blood retention of rose bengal, a rose bengal/TA complex, and a rose bengal ternary complex in a model mouse.

Fig. 23A is a graph showing the results of chronological changes of the oxidation of a TA solution and a TA/PEG-P[Lys(FPBA)$_{10}$]$_{20}$ solution, obtained by using absorbance measurement.

Fig. 23B is a photographic image of a TA solution and a TA/PEG-P[Lys(FPBA)$_{10}$]$_{20}$ solution after incubating for 24 hours.

Fig. 23C is a graph showing the results of the stability of a GFP ternary complex in solution, obtained from the measurements of particle diameter and fluorescence intensity.

Fig. 24 a graph showing the particle diameter measurement results of a GFP ternary complex in an ATP solution.

Fig. 25A is a graph showing the results of measuring the chronological activity change of βGal, a βGal/TA complex, and a βGal ternary complex, obtained by using GlycoGREEN-βGal.

Fig. 25B is a graph showing the results of measuring the maximum activity value of βGal, a βGal/TA complex, and a βGal ternary complex, obtained by using GlycoGREEN-βGal.

Fig. 26A is a graph showing the results of measuring the incorporation amounts of an Alexa647-βGal complex, an Alexa647-βGal/TA complex, and an Alexa647-βGal ternary complex incorporated into CT26 cells.

Fig. 26B is a graph showing the results of measuring the intracellular activity of βGal, a βGal/TA complex, and a βGal ternary complex using GlycoGREEN-βGal in CT26 cells.

Fig. 26C is a graph showing the results of dividing the intracellular activity of βGal, a βGal/TA complex in CT26 cells, and a βGal ternary complex by the incorporation amount, where the intracellular activity was measured using GlycoGREEN-βGal.

Fig. 27 is a graph showing the results of comparing blood retention and accumulation in each organ between Alexa647-βGal and an Alexa647-βGal ternary complex in a CT26 subcutaneous tumor model mouse.

Fig. 28 is a result of evaluating the gene expression efficiency of AAV, an AAV/TA complex, and an AAV ternary complex in CT26 cells.

Fig. 29 is a graph showing results of gene expression levels of AAV, an AAV/TA complex, and an AAV ternary complex in each organ of the CT26 subcutaneous tumor model mouse in a case where the gene expression level of AAV alone is set to 1.

Fig. 30A is a graph showing the results of measuring the amount of ALT in blood in a case where AAV, an AAV/TA complex, or an AAV ternary complex is administered to the CT26 subcutaneous tumor model mouse.

Fig. 30B is a graph showing the results of measuring the amount of AST in blood in a case where AAV, an AAV/TA complex, or an AAV ternary complex is administered to the CT26 subcutaneous tumor model mouse.

Fig. 31 is a graph showing the results of evaluating the change in gene expression efficiency of AAV, an AAV/TA complex, or an AAV ternary complex in CT26 cells due to the addition of an AAV antibody.

Fig. 32 is a graph showing the results of chronologically comparing blood retention of TUG1, a TUG1/TA complex, and a TUG1 ternary complex in a model mouse with an in vivo confocal laser scanning microscope.

[Description of Embodiments]

Hereinafter, a complex, a medicine, a therapeutic agent for cancer, a kit, and a conjugate in one embodiment of the present invention will be described.

<<Complex>>

The complex of the embodiment may be a complex comprising a conjugate in which a polymer having a boronic acid group is bonded to a compound having a diol structure and a substance (hereinafter, may be referred to as a "composite element") that is complexed with the conjugate, and the polymer may be a biocompatible polymer.

The complex of the embodiment is a complex comprising a conjugate in which a biocompatible polymer having a boronic acid group is bonded to a compound having a diol structure, and a substance that is complexed with the conjugate.

Fig. 1 is a schematic diagram showing an example of a schematic configuration of a complex of an embodiment. A complex 1 of the embodiment contains a conjugate 10 and a substance 40 is complexed with the conjugate 10.

Examples of the substance that is complexed with the conjugate 10 include at least one selected from the

group consisting of a protein, a virus, an inorganic particle, a nucleic acid, and a small molecule medicine.

In a case where the substance that is complexed with a conjugate is a protein, a protein complex can be exemplified as an embodiment of the complex.

The protein complex of the embodiment may be a complex comprising a conjugate in which a polymer having a boronic acid group is bonded to a compound having a diol structure, and a protein. In the present specification, the "diol structure" refers to a structure in which two hydroxyl groups are bonded to carbon atoms different from each other and may be a structure in which two hydroxyl groups are bonded to adjacent carbon atoms. The compound having a diol structure is not limited to an aliphatic compound.

In Fig. 1, in a case where the substance 40 that is complexed with the conjugate 10 is a protein 4, the protein complex 1 of the embodiment contains the conjugate 10 and the protein 4.

The conjugate 10 is a conjugate in which a polymer 2 having a boronic acid group is bonded to a compound 3 having a diol structure. For example, a diol structure represented by Formula (10a) and a boronic acid group represented by Formula (10b) can form a boronic acid diol bond represented by Formula (10c). That is, the conjugate 10 in the complex 1 of the embodiment may be a conjugate in which the polymer 2 having a boronic acid group and the compound 3 having a diol structure form a boronic acid diol bond.

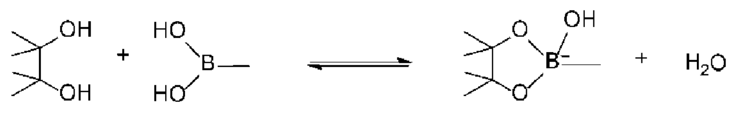

(10a) (10b) (10c)

While forming the conjugate 10 with the polymer 2 having a boronic acid group, the compound 3 having a diol structure can also bond to a composite element 40 such as a protein 4 to form a complex as shown in Fig. 1. It is conceived that the compound 3 having a diol structure and the protein 4 (the composite element 40) can be bonded by hydrophobic interaction and/or hydrogen bonding, and the complex formation is possible without chemically modifying the protein 4 (the composite element 40). That is, it appears that the polymer 2 having a boronic acid group is added to the protein 4 (the composite element 40) through the compound 3 having a diol structure (that is, through the portion derived from the compound 3 having a diol structure of the conjugate 10). As a result, in the complex 1 of the embodiment, a complex can be formed with the conjugate 10 without chemically modifying the composite element such as a protein.

From the complex formation mode described above, the complex of the embodiment may take a form in which a composite element such as a protein is used as a core and a conjugate is arranged around the composite element like a shell. More specifically, it is possible to take a form in which a composite element such as a protein is used as a core, a portion derived from a compound having a diol structure as a part of a conjugate is arranged around the core, and a polymer portion is further arranged on the outside thereof. As a result, the composite element is encapsulated and protected by the conjugate, and thus it is possible to suppress the involvement of the composite element such as a protein in an unintended biological reaction. An example of the unintended biological reaction is, for example, an immune response.

The compound having a diol structure has a property of easily interacting with a protein or the like, and thus in the related technology described in Non-Patent Documents 2 to 4, there is a possibility that an unintended interaction occurs in vivo due to the compound having a diol structure. On the other hand, in the complex of the embodiment, since a form in which the polymer portion is arranged outside the portion derived from the compound having a diol structure may taken, it is conceived that an unintended interaction in vivo can be suppressed as compared with the related art, and as a result, the stability of substance delivery is superior to a case where the compound having a diol structure is used as it is.

In the complex 1 of the embodiment, since the polymer 2 having a boronic acid group is bonded to the compound 3 having a diol structure to form a boronic acid diol bond, the boronic acid group and the diol structure may not be contained in the complex 1.

The boronic acid diol bond can be a reversible covalent bond. The bond between the boronic acid group and the diol structure is reversible depending on the pH condition, and the boronic acid diol bond is dissociated by the transition to a low pH to form again a diol structure (10a) and a boronic acid group (10b).

Fig. 2 is a schematic diagram showing an example of a configuration of the complex of the embodiment in vivo. Generally, it is said that the pH in blood is around 7.4, and the intracellular pH (in particular, in acidic organelles such as an endosome and a lysosome) is around 5.5. For example, in the complex 1 of the embodiment, in blood (pH: about 7.4), the conjugate 10 is complexed with the protein 4 (the composite element 40), whereby blood retention and stability in blood can be improved, and in the cell (pH : about 5.5) or the periphery of the tumor (pH : about 6.6), the boronic acid diol bond is dissociated, the polymer 2 having a boronic acid group is eliminated, and thus the protein 4 (the composite element 40) is released, whereby the original function of the protein 4 (the composite element 40) can be easily exhibited.

Further, it is known that the compound 3 having a diol structure such as a polyphenol is dissociated from the protein 4 in the cell.

As described above, the complex of the embodiment can have pH responsiveness. In the present specification, the pH responsiveness of a complex refers to a property by which a bond between the compound 3 having a diol structure and the polymer 2 having a boronic acid group in the conjugate constituting the complex is dissociated, depending on the surrounding pH environment. The pH responsiveness may be a property by which a bond between the compound 3 having a diol structure and the polymer 2 having a boronic acid group in the conjugate constituting the complex is dissociated as the pH is decreased.

The complex of embodiments can have ATP responsiveness. In the present specification, the ATP responsiveness which a complex may have refers to a property by which a bond between the compound 3 having a diol structure and the polymer 2 having a boronic acid group in the conjugate constituting the complex 10 is dissociated as the surrounding ATP concentration is increased.

In a case where the complex has ATP responsiveness, in blood (pH: about 7.4), the conjugate 10 is complexed with the protein 4 (the composite element 40), whereby blood retention and stability in blood can be improved, and in the cytoplasm, the boronic acid diol bond is dissociated, the polymer 2 having a boronic acid group is eliminated, and thus the protein 4 (the composite element 40) is released, whereby the original function of the protein 4 (the composite element 40) can be easily exhibited.

The bond and the dissociation can be measured, for example, by the alizarin red method. Regarding the alizarin red method, a method described in Examples described later can be used.

Alternatively, regarding the bond and the dissociation described above, the particle diameter of the complex particles (including those in which some or all components of the conjugate are dissociated) in a different pH environment is measured, for example, as described in Examples, and in a case where the particle size is smaller in a lower pH condition than in a certain pH condition, it can be determined that in such a pH condition, the bond between the compound 3 having a diol structure of the complex and the polymer 2 having a boronic acid group is dissociated and the complex has pH responsiveness.

The particle diameter can be confirmed by a known method, and as an example, the fluorescence correlation spectroscopy or the dynamic light scattering method described in Examples can be used.

Here, the particle diameter determined by the fluorescence correlation spectroscopy in the present specification is an arithmetic mean diameter based on the number of particle diameters determined by using the Einstein-Stokes equation.

Here, the particle diameter determined by the dynamic light scattering in the present specification is an arithmetic mean diameter based on the number of particle diameters determined by using the Einstein-Stokes equation.

The dissociation need not occur in the complexes of all embodiments, present in a pH environment. Whether or not the complex has pH responsiveness can be determined, for example, based on the value (for example, the average value) obtained by analyzing a plurality of complexes.

From the viewpoint of efficiently delivering a composite element to the target site in vivo, the complex of the embodiment preferably has pH responsiveness, for example, in which the composite element and the conjugate form a complex at a pH of 7.4, and the bond between the compound 3 having a diol structure and the polymer 2 having a boronic acid group is dissociated, for example, at pH of less than 7.4, pH of 6.6 or less, or a pH 5.5 or less.

From the viewpoint of efficiently delivering a protein to the target site in vivo, the complex of the embodiment preferably has pH responsiveness in which the protein and the conjugate form a complex, for example, at a pH of 7.4, and the bond between the compound 3 having a diol structure and the polymer 2 having a boronic acid group is dissociated, for example, at a pH less than 7.4, a pH of 6.6 or less, or a pH 5.5 or less.

The complex of the embodiment preferably has pH responsiveness in which a decrease in particle size can be confirmed, for example, at a pH less than 7.4, a pH of 6.6 or less, or a pH of 5.5 or less, as compared with pH conditions higher than the above pH (for example, a pH of 7.4).

As described above, the pH environment in blood is known to be about a pH of 7.4, the pH environment in the periphery of the tumor is known to be about a pH of 6.6, and the intracellular pH environment is about a pH of 5.5.

In the complex of the embodiment, having pH responsiveness in which a decrease in particle size can be confirmed at a pH of less than 7.4 as compared with a pH of 7.4 or more, since the complex is formed in blood and has excellent blood retention and stability in blood and the complex is dissociated to release a protein or the like at the delivery destination of the composite element such as a protein, such as the periphery of the tumor or the inside of the cell, the original function of the protein or the like is more effectively exhibited at the delivery destination.

In the complex of the embodiment, having pH responsiveness in which a decrease in particle size can be confirmed at a pH of 6.6 or less as compared with a pH of 7.4 or more, since the complex is formed in blood and has excellent blood retention and stability in blood and the complex is dissociated to release the composite element such as a protein in the periphery of the tumor or the inside of the cell, the original function of the composite element such as a protein can be more effectively exhibited in the periphery of the tumor and the inside of the cell.

In the complex of the embodiment, having pH responsiveness in which a decrease in particle size can be confirmed at a pH of 5.5 or less as compared with a pH of 7.4 or more, since the complex is formed in blood and has excellent blood retention and stability in blood and the complex is dissociated to release the composite element such as a protein in the inside of the cell, the original function of the composite element such as a protein can be more effectively exhibited in the inside of the cell.

Although those described above are exemplified as the pH related to the pH responsiveness of the complex of the embodiment, the pKa of the boronic acid group related to the pH responsiveness can be appropriately adjusted, for example, by modifying the structure to which the boronic acid group is bonded, and thus the pH related to the pH responsiveness of the complex of the embodiment is not limited to those exemplified above.

Further, the confirmation of the bond and dissociation between the compound 3 having a diol structure of the conjugate and the polymer 2 having a boronic acid group and the particle diameter described above is not limited to the measurement under the conditions described in Examples described later, and it can be appropriately determined depending on the environment in which the complex of the embodiment is used. Even in a case where the above dissociation is not confirmed under certain conditions, it is recommended to carry out measurement for a longer period of time or to confirm under conditions closer to the usage environment and delivery environment.

Further, the degree of pH responsiveness under the delivery environment (for example, the degree of decrease rate of the complex particle size) can be freely adjusted by adjusting the pKa of the boronic acid group related to the pH responsiveness. Further, even in a case where the degree of pH responsiveness (for example, the degree of decrease rate of the complex particle size) is poor in the delivery environment, the usefulness of the complex of the present embodiment is not denied. Rather, such a complex is conceived to be able to exhibit sustained release properties of the composite element such as a protein and may be suitably utilized for long-term substance delivery.

The average particle diameter of the complex of the embodiment is, for example, preferably 5 nm or more and 200 nm or less, more preferably 10 nm or more and 150 nm or less, and still more preferably 15 nm or more and 100 nm or less. The particle diameter of the complex can be measured by dynamic light scattering (DLS) or fluorescence correlation spectroscopy (FCS) under the measurement conditions described in Examples described later.

In a case where the particle diameter of the complex of the embodiment is in the above range, it is possible to suitably improve the blood retention and the accumulation of the complex in the tumor tissue and to prevent the accumulation thereof in the normal tissues such as the liver. As a result, the composite element such as a protein can be efficiently delivered to the tumor tissue.

Tumor accumulation of the complex is conceived to be exhibited by selective accumulation to a tumor, which utilizes enhanced vascular leakiness of a tumor, that is, an enhanced permeability and retention effect (an EPR effect), and a more excellent antitumor effect is achieved by selective delivery to the tumor.

The ratio of the conjugate 10 to the composite element 40 contained in the complex of the embodiment is not particularly limited; however, for example, one molecule of the composite element may form a complex with 1 or more conjugates, may form a complex with 2 or more conjugates, may form a complex with 5 or more conjugates, may form a complex with 1 to 100 conjugates, may form a complex with 2 to 50 conjugates, and may form a complex with 5 to 20 conjugates.

In a case where the complex of the embodiment contains the protein, the ratio of the conjugate 10 to the protein 4 contained in the complex of the embodiment is not particularly limited; however, for example, one molecule of the protein may form a complex with 1 or more conjugates, may form a complex with 2 or more conjugates, may form a complex with 5 or more conjugates, may form a complex with 1 to 100 conjugates, may form a complex with 2 to 50 conjugates, and may form a complex with 5 to 20 conjugates.

Hereinafter, details of each element included in the complex of the embodiment will be described.

(Compound having a diol structure)

The compound 3 having a diol structure according to the present embodiment forms a bond with a polymer having a boronic acid group and also is complexed with a composite element such as a protein, and thus contributes to the formation of the complex, so to speak, as a mediator between the two.

The compound having a diol structure according to the present embodiment is not particularly limited as long as it has one or more diol structures in the molecule, and from the viewpoint of bond stability, it preferably has one or more catechol structures and/or galloyl structures. A case where the compound has a catechol structure and/or a galloyl structure is preferable since the hydrophobic interaction with the benzene ring in the structure further promotes the complex formation with the composite element such as a protein.

As the catechol structure, a structure represented by Formula (3a) can be exemplified. As the galloyl structure, a structure represented by Formula (3b) can be exemplified. Among the structures shown below, the galloyl structure represented by Formula (3b) is preferable since hydrogen bonding with a hydroxyl group further promotes the complex formation with the composite element such as a protein.

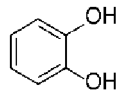 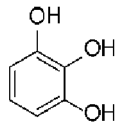

(3a) (3b)

The number of diol structures contained in the compound 3 according to the present embodiment is 1 or more, may be 2 or more, and may be 5 or more. The upper limit value of the number of diol structures in the compound according to the present embodiment is not particularly limited; however, it may be, as an example, 30 or less, 15 or less, and 13 or less. As an example of the numerical range of the above numerical values, the number of diol structures contained in the compound 3 according to the present embodiment may be an integer of 1 to 30, may be an integer of 2 to 15, and may be an integer of 5 to 13.

Considering the equilibrium state of dissociation and bonding in the diol structure, even in a case where one of the bonds of the diol structures is dissociated, the other diol structure can be bonded since the compound 3 has a plurality of (two or more) diol structures. As described above, as the number of diol structures in the compound having a diol structure according to the present embodiment is large, the apparent bonding force between the compound having a diol structure and the polymer having a boronic acid group is dramatically improved.

The bonding force between the polymer having a boronic acid group and the compound having a diol structure can be measured by, for example, the alizarin red method. Regarding the alizarin red method, a method described in Examples described later can be used.

Examples of the compound having a diol structure include those corresponding to polyphenols. Examples of the polyphenol include aromatic hydrocarbons having a structure in which two or more hydrogen atoms are substituted with a hydroxyl group. Natural polyphenols are known to be produced by plants. Examples of the polyphenol include gallic acid, catechins (catechin and a derivative thereof), epicatechins (epicatechin and a derivative thereof), proanthocyanidin, anthocyanidin, galloylated catechins (galloylated catechin and a derivative thereof), flavonoid, isoflavonoid, neoflavonoid, flavone, tannin, tannic acid, and derivatives thereof. The compound having a diol structure is preferably at least one selected from the group consisting of tannic acid, gallic acid, and derivatives thereof. Examples of the above derivative include compounds having a diol structure in which one or more hydrogen atoms or groups are substituted with another group (a substituent). Further, it may be a derivative obtained by adding or eliminating a hydrogen atom in the compound having a diol structure. Here, examples of the substituent include a hydroxyl group, an amino group, a monovalent chain-like saturated hydrocarbon group having 1 to 4 carbon atoms, and a halogen atom. Examples of the monovalent chain-like saturated hydrocarbon group having 1 to 4 carbon atoms include a methyl group, an ethyl group, a propyl group, and a butyl group. Examples of the halogen atom include a fluorine atom and a chlorine atom.

(Polymer having boronic acid group)

Hereinafter, the polymer 2 having a boronic acid group according to the present embodiment will be described.

The polymer may be a biocompatible polymer. The biocompatible polymer means a polymer that does not exhibit or hardly exhibits a remarkable deleterious action or adverse effect such as a strong inflammatory reaction or damage in a case of being administered to a living body.

The biocompatible polymer having a boronic acid group is not particularly limited as long as the effects of the present invention can be obtained, and examples thereof include a polyethylene glycol (PEG), an acrylic resin (a resin including a constitutional unit derived from a (meth)acrylic acid ester), a polyamino acid, a polyvinylamine, a polyallylamine, a polynucleotide, a polyacrylamide, a polyether, a polyester, a polyurethane, polysaccharides, and polymers obtained by introducing a boronic acid group into these copolymers. In a part of the biocompatible polymer having a boronic acid group, any group introduced in the process of the synthesis thereof may be contained. Examples of such a group include a part of a polymerization initiator or the like.

The dispersity (Mw/Mn) of the polymer is preferably 1.0 or more and less than 2.0, more preferably 1.0 to 1.5, and still more preferably 1.0 to 1.3. In order for the complex of the embodiment to more effectively exhibit excellent tumor accumulation, it is preferable that the dispersity of the polymer be within the above range.

In the present specification, as the number average molecular weight of the polymer, a value calculated from the ratio between the peak integral values based on the $^1H$ NMR spectrum can be adopted. Regarding the calculation method, the number average molecular weight of the polymer before being introduced with a boronic acid group can be calculated, for example, as described in Examples described later, by calculating the degree of polymerization of the monomer from the ratio of a peak integral value of a structure, which is derived from an initiator present at the terminal of the polymer chain, to a peak integral value of a structure, which is derived from the monomer of the calculation target portion, and adding the total molecular weight of the structures derived from the polymerized monomer to the molecular

weight of the structure derived from the initiator.

As the number average molecular weight of the polymer having a boronic acid group as well, which will be described later, a value calculated from the ratio between the peak integral values based on the $^1$H NMR spectrum can be adopted. Regarding the calculation method, the number average molecular weight thereof can be calculated, for example, as described in Examples described later, by calculating the number of conjugations of the boronic acid group from the ratio of a peak integral value of a structure, which is derived from an initiator present at the terminal of the polymer chain, to a peak integral value of a structure, which is derived from the boronic acid group of the calculation target portion, and adding the total molecular weight of the structures derived from the conjugated boronic acid group to the number average molecular weight of the polymer chain.

The polymer having a boronic acid group of the present embodiment preferably has a number average molecular weight (Mn) of 2,000 to 200,000 and, for example, may be 5,000 to 100,000, may be 10,000 to 50,000, or may be 12,000 to 45,000, where the number average molecular weight is calculated from $^1$H NMR.

In a case where the number average molecular weight of polymers having a boronic acid group is in the above range, it is possible to suitably improve the blood retention of the complex and the accumulation of the conjugate in the tumor tissue and to prevent the accumulation of the conjugate in the normal tissues such as the liver. As a result, the composite element such as a protein can be efficiently delivered to the tumor tissue.

Tumor accumulation of the complex is conceived to be exhibited by selective accumulation to a tumor, which utilizes enhanced vascular leakiness of a tumor, that is, an enhanced permeability and retention effect (an EPR effect), and a more excellent antitumor effect is achieved by selective delivery to the tumor.

Further, in the complex, the polymer having a boronic acid group may form a polymer micelle or may have a form of a polymer vesicle.

In the polymer having a boronic acid group of the present embodiment, the biocompatible polymer is preferably biodegradable.

The biodegradability means a property of being absorbed or degraded in vivo. The biodegradable biocompatible polymer is not particularly limited as long as the effects of the present invention can be obtained, and examples thereof include a polyamino acid, a polyester, a polynucleotide, and polysaccharides.

In the present specification, the description that a polymer having a boronic acid group is biodegradable means that at least a part of the polymer having a boronic acid group is biodegradable. As a result, a block copolymer of a polyamino acid, a polyester, a polynucleotide, polysaccharides, or the like with PEG, an acrylic resin (a resin including a constitutional unit derived from a (meth)acrylic acid ester), a polyacrylamide, a polyether, a polyurethane or the like also corresponds to the biodegradable biocompatible polymer.

In a case where a biodegradable polymer is used, it is possible to suppress the accumulation of the conjugate or complex in vivo and to reduce side effects.

In the present specification, the biostability means a property of being present in a living body without being immediately absorbed or immediately degraded in vivo. In a case where a polymer has biodegradability and biostability, it means that the biocompatible polymer is capable of being present in a living body until it is absorbed or degraded in vivo.

In the present specification, the description that a polymer is biostable means that at least a part of the polymer is biostable. As a result, a block copolymer of a polyamino acid, a polyester, a polynucleotide, polysaccharides, or the like with PEG, an acrylic resin (a resin including a constitutional unit derived from a (meth)acrylic acid ester), a polyacrylamide, a polyether, a polyurethane or the like also corresponds to the biostable biocompatible polymer.

The polymer having a boronic acid group may have the first biocompatible polymer chain and the second biocompatible polymer chain. Here, the first biocompatible polymer chain is different from the second biocompatible polymer chain, and the biocompatible polymer of the present embodiment can be provided as a block copolymer containing a first biocompatible polymer chain block and a second biocompatible polymer chain block. In addition, the biocompatible polymer according to the present embodiment can further contain another polymer chain in addition to the first biocompatible polymer chain and the second biocompatible polymer chain.

In the present embodiment, the "block copolymer" is a polymer to which a plurality of kinds of blocks (partial constitutional components in which the same kind of constitutional units are repeatedly bonded) are bonded. The blocks constituting the block copolymer may be two kinds or may be three kinds or more.

The first biocompatible polymer chain or the second biocompatible polymer chain is preferably a polyethylene glycol (PEG) from the viewpoints of excellent biocompatibility and versatility.

The first biocompatible polymer chain or the second biocompatible polymer chain is preferably a polyamino acid from the viewpoints of excellent biocompatibility and the balance between biostability and biodegradability.

The combination of the first biocompatible polymer chain and the second biocompatible polymer chain which are contained in the biocompatible polymer is preferably, for example, a combination in which the first biocompatible polymer chain is a polyethylene glycol and the second biocompatible polymer chain is a polyamino acid.

A method of producing a biocompatible polymer containing the first biocompatible polymer chain and the second biocompatible polymer chain is not particularly limited. For example, it can be produced by a method in which the first

biocompatible polymer chain is synthesized by a known polymerization reaction, and then a monomer of the second biocompatible polymer chain is polymerized to the first biocompatible polymer chain. The polymer chains obtained by the polymerization reaction may be each in a state of precursors (for example, those having a protective group), or precursors obtained by the polymerization reaction may be subjected to an ordinary treatment selected by those skilled in the art to produce the first biocompatible polymer chain and the second biocompatible polymer chain.

Alternatively, the first biocompatible polymer chain or a precursor thereof, provided as a polymer in advance, and the second biocompatible polymer chain or precursor thereof can be bonded by a known reaction. At that time, both the chains may be bonded by utilizing the bonding between reactive functional groups. In a case where precursors are used, the precursors are subjected to the same treatment as above, whereby the first biocompatible polymer chain and the second biocompatible polymer chain can be produced.

The polymer according to the embodiment is a polymer having a boronic acid group. The boronic acid group may have a structure represented by Formula (10b). From the viewpoint that a boronic acid diol bond can be efficiently formed even under pH conditions near neutrality such as the environment in vivo, the boronic acid group is preferably a phenylboronic acid group which may have a substituent or a pyridylboronic acid group which may have a substituent. As the phenylboronic acid group and the pyridylboronic acid group, those disclosed in the previous reports (PCT International Publication No. 2013/073697, Japanese Unexamined Patent Application, First Publication No. 2018-142115, and the like) can also be exemplified and incorporated herein.

From the viewpoint that a boronic acid diol bond is formed more efficiently and the above pH responsiveness can be more easily exhibited, the boronic acid group is preferably a phenylboronic acid group represented by General Formula (I) or a pyridylboronic acid group represented by General Formula (II).

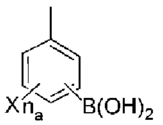

(I)

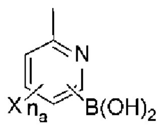

(II)

(in the formulae, X represents a halogen atom or a nitro group, and na is an integer of 0 to 4).

The halogen atom as X is an element belonging to Group 17 in the periodic table, such as F, Cl, Br, or I, and F is preferable.

The phenylboronic acid group represented by General Formula (I) is preferably a group represented by General Formula (1-1) or General Formula (1-2). The pyridylboronic acid group represented by General Formula (II) is preferably a group represented by General Formula (II-1).

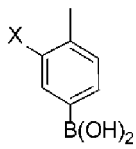

(I-1)

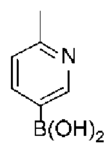

(II-1)

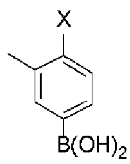

(I-2)

(In the formulae, X represents a halogen atom or a nitro group).

In General Formula (1-1) and General Formula (1-2), in a case where X is bonded to such a position, it is conceived that X effectively acts as an electron-withdrawing group and contribute to the stabilization of the boronic acid diol bond represented by Formula (10c). As a result, the boronic acid diol bond is easily formed even in a pH environment near neutrality in vivo, and thus the above pH responsiveness can be more easily exhibited.

The group represented by General Formula (1-1) is preferably a group represented by General Formula (I-1-1), and the group represented by General Formula (1-2) is preferably a group represented by General Formula (1-2-1). The group represented by General Formula (II-1) is preferably a group represented by General Formula (II-1-1).

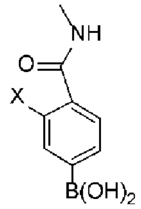
(I-1-1)

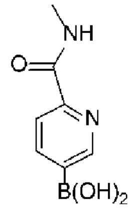
(II-1-1)

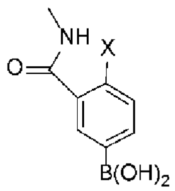
(I-2-1)

Since the groups represented by General Formula (I-1-1), General Formula (1-2-1), and General Formula (II-1-1) have an amide bond, they exhibit an action of reducing the apparent pKa of the boronic acid group.

In the polymer of the embodiment, one or more boronic acid groups may be introduced into the polymer, two or more thereof may be introduced thereinto, and five or more thereof may be introduced thereinto.

The upper limit value of the number of boronic acid groups in the polymer of the present embodiment is not particularly limited; however, it may be, as an example, 1,000 or less, 100 or less, or 50 or less. As an example of the numerical range of the above numerical values, the number of boronic acid groups contained in the polymer according to the present embodiment may be an integer of 1 to 1,000, may be an integer of 2 to 100, and may be an integer of 5 to 50.

In a case where the above number is equal to or larger than the lower limit, the action of forming the conjugate by the boronic acid group is satisfactorily exhibited, which is preferable.

Considering the equilibrium state of dissociation and bonding in the boronic acid group, even in a case where one of the bonds of the boronic acid groups is dissociated, the other boronic acid groups can be bonded since the polymer has a plurality of (two or more) boronic acid groups. As described above, as the number of boronic acid groups in the polymer according to the present embodiment increases, the apparent bonding force between the polymer having a boronic acid group and the compound having a diol structure is dramatically improved.

As a result, from the viewpoint of improving the bonding force between the above two, it is more preferable to use a combination of a compound having two or more diol structures and a polymer having two or more boronic acid groups. Examples of the number of two or more diol structures and boronic acid groups include those exemplified above.

The polymer having a boronic acid group can be obtained by introducing a boronic acid group into a polymer.

In the polymer of the present embodiment, the boronic acid group can be introduced into any position of the polymer. The boronic acid group may be introduced into the first biocompatible polymer chain and/or the second biocompatible polymer chain.

For example, a boronic acid group may be introduced by utilizing the bonding between the polymer and the "compound having a boronic acid group" through the functional groups that are reactive with each other. The reactive functional group may be one originally contained in the polymer, or may be modified or introduced.

In the bonding between the polymer and the compound having a boronic acid group, the compound having a boronic acid group and the polymer may each undergo a structural change necessary for bonding as long as the effects of the present invention are obtained.

For example, the compound having a boronic acid group may be bonded to a functional group contained in the polymer and may be bonded to a functional group contained in the first biocompatible polymer chain and/or the second biocompatible polymer chain.

For example, the compound having a boronic acid group may be bonded to a functional group of the side chain of the polymer and may be bonded to a functional group of the side chain of the first biocompatible polymer chain and/or the second biocompatible polymer chain.

The boronic acid group may be introduced into the side chain of the polymer through a divalent linking group. Examples of the divalent linking group include an amide bond, a carbamoyl bond, an alkyl bond, an ether bond, an ester bond, a thioester bond, a thioether bond, a sulfone amide bond, a urethane bond, a sulfonyl bond, a thymine bond, a urea bond, and a thiourea bond.

Here, the polymer into which a boronic acid group is introduced is preferably one having a cationic group in the side chain. Even in a case where the boronic acid group is introduced into a side chain of the polymer, the cationic

group of a side chain which remains without being introduced with the boronic acid group can stabilize the bonding of the conjugate by the interaction with the anionic group represented by Formula (10c).

Accordingly, the polymer according to the present embodiment may have a boronic acid group and a cationic group, and the first biocompatible polymer chain and/or the second biocompatible polymer chain may have a boronic acid group and a cationic group.

In a case where the polymer has a boronic acid group and a cationic group, the molar ratio of the boronic acid group to the cationic group (the cationic group : the boronic acid group) may be 10:1 to 1:10, may be 10:3 to 3:1, and may be 10:8 to 8:10.

The above cationic group is preferably an amino group. In a case where an amino group is contained in the side chain, the amino group can be coordinated with boron of the boronic acid in an aqueous medium, and the bonding of the conjugate can be further stabilized.

Examples of the biocompatible polymer chain having an amino group in the molecule include a polyamino acid, a polyacrylamide, a polyvinylamine, and a polyallylamine, and a polyamino acid is preferable. The polyamino acid preferably has a cationic group in the side chain and more preferably has an amino group in the side chain.

In a case where a biocompatible polymer into which a boronic acid group is introduced has an amino group, the amino group may be an amino group protected by a protective group.

In a case of using a biocompatible polymer chain that does not have an amino group or an amino group protected by a protective group, it is possible to introduce an amino group into the biocompatible polymer by a known method using the introduction of ethylenediamine and hydrazine, the Bechamp reduction, the direct amination method of a hydroxyl group, aminolysis, the Curtius transfer method.

In a case of forming a bond with the amino group, the compound having a boronic acid group is preferably one having a carboxyl group from the viewpoints of bond stability with the amino group and ease of synthesis. An amide bond is formed between the amino group of the biocompatible polymer to which a boronic acid group is introduced and the carboxyl group of the compound having a boronic acid group, and then the boronic acid group can be introduced into the biocompatible polymer. In addition, the formed amide bond also exhibits an action of reducing the apparent pKa of the boronic acid group.

As the compound having a boronic acid group and a carboxyl group, 4-carboxy-phenylboronic acid, 3-carboxy-4-fluorophenylboronic acid, 4-carboxy-2-fluorophenylboronic acid, 4-carboxy-3-fluorophenylboronic acid (FPBA), 3-carboxy-4-chlorophenylboronic acid, 4-carboxy-2-chlorophenylboronic acid, or 4-carboxy-3-chlorophenylboronic acid can be used.

Examples of the method of forming an amide bond between a carboxyl group and an amino group include subjecting a biocompatible polymer chain having an amino group and a compound having a boronic acid group and a carboxyl group to a condensation reaction in the presence of a condensing agent such as DMT-MM. In addition, in a case of a biocompatible polymer chain having an amino group protected by a protective group, the protective group can be deprotected by a known reaction to obtain a biocompatible polymer chain having an amino group, which subsequently can be subjected to the same condensation reaction.

In addition, the boronic acid group may be introduced into any one of the first biocompatible polymer chain or the second biocompatible polymer chain. For example, the boronic acid group can be introduced into the second biocompatible polymer chain. In Fig. 1, the biocompatible polymer 2 having a boronic acid group contains a second biocompatible polymer chain 22 having a boronic acid group and a first biocompatible polymer chain 21 having no boronic acid group. For example, the second biocompatible polymer chain has a side chain, and the boronic acid group may be introduced into a side chain of the second biocompatible polymer chain.

An example of the polymer having a boronic acid group according to the present embodiment includes a structure represented by General Formula (1) or (1-1).

$$A-L-B \quad (1) \qquad\qquad A-B \quad (1-1)$$

(In Formulae (1) and (1-1), A represents the first biocompatible polymer chain, L represents a linker part, and B represents the second biocompatible polymer chain having a boronic acid group.)

The linker part is preferably an alkylene group having 1 to 20 carbon atoms, more preferably a linear alkylene group having 1 to 20 carbon atoms, and still more preferably a linear alkylene group having 1 to 5 carbon atoms. One or more of -CH2- in the alkylene group may be each independently substituted with -CH=CH-, -O-, -CO-, -S-, -NH-, or -CONH-. Examples of the alkylene group include a methylene group, an ethylene group, a trimethylene group, a tetramethylene group, and a pentamethylene group.

The second biocompatible polymer chain is preferably polyamino acid.

In a case where the second biocompatible polymer chain is a polyamino acid and the boronic acid group is

introduced into the side chain of the polyamino acid, B in General Formula (1) or (1-1) is preferably as follows.

B represents the second biocompatible polymer chain having a boronic acid group, and the second biocompatible polymer chain preferably includes a repeating structure represented by the following (b2), or a repeating structure represented by (b1) and the repeating structure represented by (b2).

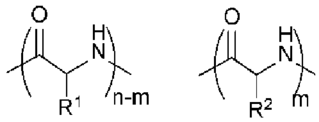

(b1) (b2)

(In Formulae (b1) and (b2), $R^1$ represents an amino acid side chain, $R^2$ is a structure in which the boronic acid group is introduced into an amino acid side chain, and n represents the total number of (b1) and (b2), n is an integer of 1 to 1,000, m is an integer of 1 to 1,000 (here, $m \leq n$), in a case where n - m is 2 or more, a plurality of $R^1$'s may be the same or different from each other, and in a case where m is 2 or more, a plurality of $R^2$'s may be the same or different from each other.)

The amino acid of $R^1$ and $R^2$ is preferably a naturally occurring amino acid, and examples thereof include valine, leucine, isoleucine, alanine, glycine, phenylalanine, tyrosine, tryptophan, methionine, cysteine, serine, threonine, glutamine, asparagine, lysine, arginine, histidine, aspartic acid, glutamine acid, and proline.

The amino acid side chain is used in the usual sense in the related art and refers to a structure other than the amino group and the carboxy group, involved in the amide bond of the polypeptide, and for example, is a hydrogen atom in a case of glycine, is a methyl group in a case of alanine, and is an isopropyl group in a case of valine.

In a case where the second biocompatible polymer chain includes a repeating structure represented by (b1) and a repeating structure represented by (b2), (b1) and (b2) may be randomly sequenced. m represents the total number of (b2) in the second biocompatible polymer chain, and n - m represents the total number of (b1) in the second biocompatible polymer chain. n - m may be 0 (that is, among (b1) and (b2), the second biocompatible polymer chain may have only (b2) introduced with a boronic acid group).

The second biocompatible polymer chain may be composed of a repeating structure represented by the above (b2), or a repeating structure represented by (b1) and a repeating structure represented by (b2).

Further, an amino acid side chain of $R^1$ and an amino acid side chain of $R^2$ may be the same or different from each other.

In the Formulae (b1) and (b2), n is an integer of 1 to 1,000, may be an integer of 10 to 500, and may be an integer of 15 to 100. In a case where the value of n is within the above range, the value of the molecular weight of the second biocompatible polymer chain becomes a suitable value, which is preferable.

In the Formulae (b1) and (b2), m is an integer of 1 to 1,000, may be an integer of 3 to 100, and may be an integer of 5 to 50. In a case where the above value m is equal to or larger than the lower limit, the action of forming the conjugate by the boronic acid group is satisfactorily exhibited, which is preferable.

Here, the numerical range of a case where n is larger than m is also exemplified; however, n and m may be the same number.

The mode of introduction of a boronic acid group into a polyamino acid is not particularly limited; however, a bonding between the amino acid side chain of the polyamino acid and the compound having a boronic acid group is preferable. Examples of the method of bonding a compound having a boronic acid group to an amino acid side chain of a polyamino acid include a method of forming an amide bond to a carboxyl group of an aspartic acid side chain or a glutamic acid side chain and a method of forming a disulfide bond to a thiol group in the side chain of cysteine. However, as described above, in a case where the second biocompatible polymer chain has an amino group in the side chain, the amino group can be coordinated with boron of the boronic acid in an aqueous medium, and the bonding of the conjugate can be further stabilized, and thus a method of forming an amide bond between the amino acid side chain having an amino group and the carboxyl group of the compound having a boronic acid group and a carboxyl group is preferable.

The amino acid side chain having an amino group may be an amino group of a natural amino acid side chain such as a lysine side chain, an arginine side chain, an asparagine side chain, or a glutamine side chain, and may be one obtained by introducing an amino group into any amino acid side chain, and a lysine side chain is preferable from the viewpoint of biocompatibility and the like.

The repeating structure represented by the above (b2) preferably contains a structure in which $R^2$ is a structure in which a boronic acid group is introduced into an amino acid side chain having cationic group, as a constitutional unit, more preferably contains a structure in which $R^2$ is a structure in which a boronic acid group is introduced into an amino acid side chain having an amino group, as a constitutional unit, and still more preferably contains a structure in which

$R^2$ is a structure in which a boronic acid group is introduced into a lysine side chain, as a constitutional unit.

In a case where n - m is 1 or more, the repeating structure represented by the above (b1) preferably contains a structure in which $R^1$ is an amino acid side chain having a cationic group, as a constitutional unit, more preferably contains a structure in which $R^1$ is an amino acid side chain having an amino group, as a constitutional unit, and still more preferably contains a structure in which $R^1$ is a lysine acid side chain, as a constitutional unit.

The first biocompatible polymer chain is preferably a polyethylene glycol.

In a case where the first biocompatible polymer chain is a polyethylene glycol and the second biocompatible polymer chain is a polyamino acid, the structure represented by General Formula (1) is preferably a structure represented by General Formula (1-2).

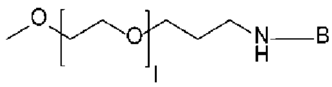

(1−2)

(In Formula (1-2), 1 is an integer of 1 to 1,500; B represents the second biocompatible polymer chain having a boronic acid group; and the second biocompatible polymer chain includes a repeating structure represented by the following (b2), or a repeating structure represented by (b1) and the repeating structure represented by (b2).)

In Formula (1-2), 1 is an integer of 1 to 1,500, may be an integer of 10 to 1,000, and may be an integer of 100 to 500.

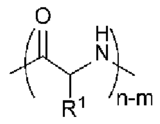

(b1)

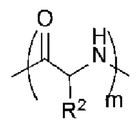

(b2)

(In Formulae (b1) and (b2), $R^1$, $R^2$, n, and m have the same meanings as described above.)

(Substance complexed with conjugate)

The substance that is complexed with a conjugate in the complex of the present embodiment is not particularly limited as long as it can be complexed with the conjugate to form the complex, and may be any substance.

The substance that is complexed with a conjugate can be complexed with the conjugate through a portion derived from a compound having a diol structure of the conjugate.

The fact that a substance can be complexed with the conjugate through a portion derived from a compound having a diol structure of the conjugate can be preliminarily confirmed, for example, in a case where both can form a complex in a composition containing the substance and a compound having a diol structure. For example, in a composition containing a protein and a polyphenol, in a case where both can form a complex, the protein is highly likely to be complexed with the conjugate through a portion of the conjugate derived from the polyphenol. The complexation can be determined by the fact that the particle size of the particles contained in the composition is larger than the particle size of the substance alone.

The fact that a substance is complexed with a conjugate can be evaluated, for example, by confirming that both can form a complex in a composition containing the substance and the conjugate. The formation of the complex can be determined by the fact that the particle size of the particles contained in the composition is larger than the particle size of the substance alone.

The size of the substance that is complexed with a conjugate is not particularly limited; however, as an example, the particle diameter of the substance may be 500 nm or less, 0.1 nm or more and 500 nm or less, may be 0.2 nm or more and 100 nm or less, and may be 0.3 nm or more and 50 nm or less. The particle diameter can be measured by dynamic light scattering (DLS) or fluorescence correlation spectroscopy (FCS) under the measurement conditions described in Examples described later.

As an example of the substance that is complexed with a conjugate, at least one selected from the group consisting of a protein, a virus, an inorganic particle, a nucleic acid, and a small molecule medicine can be exemplified. The substance included in the concept exemplified here may be included in a plurality of the above concepts.

- Protein

The protein as a composite element in the complex of the present embodiment is not particularly limited as long as it can be complexed with the conjugate to form a complex, and may be any protein.

Since the complex of the present embodiment can be suitably used for drug delivery in vivo due to having excellent blood retention, having pH responsiveness, and exhibiting tumor accumulation, the protein in the complex of the present embodiment is preferably a physiologically active protein. The physiologically active protein preferably has a pharmacological action and preferably contains a protein-type medicine.

Protein-type medicine is a medicine that contains a protein or a component containing a protein, as an active ingredient. Examples thereof include antibody medicines such as herceptin, avastin, and cyramza, various enzymes such as hyaluronidase, insulin, a cytokine, interferon, and a viral vector. Examples of the viral vector include a viral vector containing adeno-associated virus (AAV).

Further, in the present specification, the protein has a concept including a peptide. As the peptide, a membrane-permeable peptide can also be preferably exemplified.

The complex of the present embodiment preferably exhibits tumor accumulation, and the protein in the complex preferably has an antitumor effect.

Examples of the protein-type medicine having an antitumor effect include antibody medicine, interferon, and a viral vector.

• Virus

The virus as a composite element in the complex of the present embodiment is not particularly limited as long as it can be complexed with the conjugate to form a complex, and may be any virus.

Since the complex of the present embodiment can be suitably used for drug delivery in vivo due to having excellent blood retention, having pH responsiveness, and exhibiting tumor accumulation, the virus in the complex of the present embodiment is preferably a therapeutic virus that is used as a viral vector for the treatment (viral therapy) of a disease, and more preferably a cancer therapeutic virus that is used for the treatment of cancer.

The therapeutic virus may contain a nucleic acid having a pharmacological action in the viral vector or may contain a nucleic acid encoding a protein having a pharmacological action.

The therapeutic virus may contain a nucleic acid that is introduced for the treatment of a disease or may contain a nucleic acid that is introduced for the treatment of cancer.

The nucleic acid can contain an operably ligated promoter sequence in order to express the sequence contained in the nucleic acid.

Examples of therapeutic virus include various viruses or artificial viruses, which can be used as a virus vector for humans. Examples of the virus species of the virus vector include an adeno-associated virus, an adenovirus, a herpesvirus, a Sendai virus, a retrovirus, and a lentivirus.

Examples of the adeno-associated virus (AAV) include AAV1, AAV2, AAV3, AAV4, AAV5, AAV6, AAV7, AAV8, AAV9, AAV10, and AAV11.

The complex of the present embodiment preferably exhibits tumor accumulation, and the virus in the complex preferably has an antitumor effect.

• Inorganic particle

The inorganic particle as a composite element in the complex of the present embodiment is not particularly limited as long as it can be complexed with the conjugate to form a complex, and may be any inorganic particle.

The inorganic particle is a particle containing an inorganic material, and examples thereof include a metal particle such as a gold particle, a silver particle, a platinum particle, an iron particle, or titanium oxide particle; a silica particle; a semiconductor particle such as a quantum dot; and a carbon particle such as a carbon nanotube, or graphene.

The inorganic particle is preferably a nanoparticle. The nanoparticle is a particle having a particle diameter of 1 to 100 nm. The particle diameter of the particle can be measured by dynamic light scattering (DLS) or fluorescence correlation spectroscopy (FCS) under the measurement conditions described in Examples described later.

The inorganic particles may be particles further modified with at least one selected from the group consisting of the above-described protein, virus, nucleic acid, and small molecule medicine.

• Nucleic acid

The nucleic acid as a composite element in the complex of the present embodiment is not particularly limited as long as it can be complexed with the conjugate to form a complex, and may be any inorganic particle.

Since the complex of the present embodiment can be suitably used for drug delivery in vivo due to having excellent blood retention, having pH responsiveness, and exhibiting tumor accumulation, the nucleic acid in the complex of the present embodiment is preferably a physiologically active nucleic acid. The nucleic acid having physiological activity preferably has a pharmacological action and preferably contains a nucleic acid medicine.

The nucleic acid as a composite element in the complex of the present embodiment is preferably a nucleic acid medicine that is used for the treatment of a disease.

Examples of the nucleic acid medicine include various nucleic acids having physiological activity in the human body, and examples thereof include artificial nucleic acids such as DNA, RNA, and LNA. Examples of the kind of nucleic acid include a siRNA, a miRNA, antisense nucleic acid, an aptamer, and a ribozyme.

The complex of the present embodiment preferably exhibits tumor accumulation, and the nucleic acid in the complex preferably has an antitumor effect.

Examples of the nucleic acid having an antitumor effect include a taurine upregulated gene 1 (TUG1) antisense nucleic acid, a polo-like kinase 1(PLK1) siRNA, and a vascular endothelial growth factor (VEGF) siRNA.

• Small molecule medicine

The small molecule medicine as a composite element in the complex of the present embodiment is not particularly limited as long as it can be complexed with the conjugate to form a complex, and may be any small molecule medicine.

The complex of the present embodiment can be suitably used for drug delivery in vivo due to having excellent blood retention, having pH responsiveness, and exhibiting tumor accumulation.

The "small molecule medicine" in the present specification means a medicine having a molecular weight of 1000 or less, is preferably a medicine having a molecular weight of 500 or less, and, for example, may be a medicine having a molecular weight of 200 to 1,000 and may be a medicine having a molecular weight of 300 to 500. The molecular weight of pitavastatin, a therapeutic agent for dyslipidemia, used in Examples described later, is about 421.

The complex of the present embodiment preferably exhibits tumor accumulation, and the small molecule medicine in the complex preferably has an antitumor effect.

Examples of the small molecule medicine having an antitumor effect include anticancer agents such as bleomycin and a salt thereof, acoustic sensitizers such as rose bengal, photosensitizers such as chlorine e6, and radiosensitizers such as a boron cluster.

According to the complex of the present embodiment, a complex with a conjugate can be formed without chemically modifying a composite element such as a protein, and the blood retention is improved by having a higher molecular weight due to the high molecular weight of the conjugate. Further, the conjugate is obtained by bonding a polymer having a boronic acid group to a compound having a diol structure and has pH responsiveness by which the conjugate is dissociated depending on the pH environment of the target site.

As a result, the complex of the present embodiment is an epoch-making complex since it has excellent blood retention, and the conjugate is expected to be selectively dissociated at the target delivery destination to exhibit the function of the composite element such as protein.

<<Medicine>>

As one embodiment of the present invention, a medicine containing the complex of the embodiment as an active ingredient is provided. The complex of the embodiment can have a pharmacological effect on a disease. The present embodiment is suitable in a case where the composite element such as a protein in the complex of the present embodiment is an active ingredient and has a pharmacological action, and, for example, any protein having a pharmacological action, a protein-type medicine, a therapeutic virus, a nucleic acid, a nucleic acid medicine, or a small molecule medicine can be used.

As one embodiment of the present invention, a therapeutic agent for cancer containing the complex of the embodiment as an active ingredient is provided. As one embodiment of the present invention, a complex of an embodiment for the treatment of cancer is provided. As one embodiment of the present invention, the use of the complex of an embodiment for producing a therapeutic agent for cancer is provided. The complex of the embodiment can have a cancer therapeutic effect. The present embodiment is suitable in a case where the composite element such as a protein in the complex of the present embodiment is an active ingredient and has an antitumor effect, and, for example, various proteins, protein-type medicines, therapeutic viruses, nucleic acids, nucleic acid medicines, or small molecule medicines which are capable of exhibiting antitumor effect can be used.

Examples of the target disease on which a cancer therapeutic effect is expected include blood cancer and solid cancer, and in a case where the complex of the present embodiment has tumor accumulation, a suitable target disease is solid cancer. Examples of the human solid cancer include brain cancer, head and neck cancer, esophageal cancer, thyroid cancer, small cell cancer, non-small cell cancer, breast cancer, gastric cancer, gallbladder/bile duct cancer, lung cancer, liver cancer, hepatocellular carcinoma, pancreatic cancer, colon cancer, rectal cancer, ovarian cancer, chorionic epithelial cancer, uterine cancer, cervical cancer, renal/ureter cancer, bladder cancer, prostate cancer, penile cancer, testicular cancer, fetal cancer, Wilms cancer, skin cancer, malignant melanoma, neuroblastoma, osteosarcoma, Ewing

tumor, and soft tissue sarcoma.

Examples of therapeutic agent for cancer of the present embodiment include a tablet, a capsule, an elixir, and an oral agent that is orally used as a microcapsule drug, which are optionally coated with sugar.

Alternatively, examples thereof include aseptic solutions with water or another pharmaceutically acceptable liquid and those that are used parenterally in the form of an injection agent of a suspension preparation. Further, examples thereof include those formulated by combining pharmacologically acceptable carriers or media, specifically, sterile water, a physiological saline solution, vegetable oil, an emulsifier, a suspending agent, a surfactant, a stabilizer, a flavoring agent, an excipient, a vehicle, a preservative, and a bonder, and mixing them in the unit dosage form required for generally accepted pharmaceutical practice.

As additives that can be mixed with a tablet or a capsule, for example, the following can be used: bonders such as gelatin, cornstarch, gum tragacanth, gum arabic; excipients such as crystalline cellulose; swelling agents such as cornstarch, gelatin, and alginic acid; lubricants such as magnesium stearate; sweetening agents such as sucrose, lactose, saccharin; and flavoring agents such as peppermint, Akamono (Japanese azalea) oil, and cherry. In a case where the preparation unit form is a capsule, the above-described material can further contain a liquid carrier such as fat or oil. The sterile composition for injection can be prescribed according to ordinary pharmaceutical practice using a vehicle such as distilled water for injection.

Examples of the aqueous solution for injection include a physiological saline solution, an isotonic solution containing glucose and other adjuvants, for example, D-sorbitol, D-mannose, D-mannitol, and sodium chloride, and may be used in combination with a suitable dissolution auxiliary agent such as alcohol, specifically, ethanol or polyalcohol such as propylene glycol or a polyethylene glycol; and a nonionic surfactant such as polysorbate 80 (TM) or HCO-50.

Examples of the oily liquid include sesame oil and soybean oil and may be used in combination with benzyl benzoate or benzyl alcohol, as a dissolution auxiliary agent. In addition, it may also be blended with a buffer such as a phosphate buffer or a sodium acetate buffer, a soothing agent such as procaine hydrochloride, a stabilizer such as benzyl alcohol or phenol, and antioxidant. In general, an appropriate ampoule is filled with the prepared injection solution.

The administration to a patient can be carried out by, for example, intraarterial injection, intravenous injection, or subcutaneous injection, and also can be carried out intranasally, transbronchially, intramuscularly, transcutaneously, or orally by a method known to those skilled in the art. The dose varies depending on the body weight and the age of the patient, the administration method, and the like; however, those skilled in the art can appropriately select an appropriate dose. The dose and the administration method vary depending on the body weight and the age of the patient, the symptoms, and the like; however, they can be appropriately selected by those skilled in the art.

The therapeutic agent for cancer of the embodiment may further contain another anticancer agent and the like. With such a configuration, a synergistic effect on the cancer treatment can be expected.

<<Kit>>

The kit of the present embodiment is a kit including a polymer having a boronic acid group and a compound having a diol structure. The polymer may be a biocompatible polymer. The kit of the embodiment may include a biocompatible polymer having a boronic acid group and a compound having a diol structure.

The kit of the present embodiment can be used to form the complex of the above-described embodiment. The kit of the present embodiment may further include a substance (a composite element) which is complexed with a conjugate.

Other examples of the kit of the embodiment include a kit including the conjugate of the embodiment and a substance that is complexed with the conjugate.

Examples of the substance that is complexed with a conjugate include at least one selected from the group consisting of a protein, a virus, an inorganic particle, a nucleic acid, and a small molecule medicine.

Regarding the polymer having a boronic acid group, such as the compound having a diol structure or the substance that is complexed with at least one conjugate selected from the group consisting of a protein, a virus, an inorganic particle, a nucleic acid, and a small molecule medicine, those exemplified in «Complex» described above can be adopted, and the description thereof will be omitted here.

The kit of the present embodiment may further include a solution, a reagent such as a buffer, a reaction container, and an instruction manual.

The kit of the present embodiment can form the complex of the embodiment, containing the composite element such as any protein, by combining with the composite element of any one of the above protein and the like, and thus has excellent versatility.

<<Conjugate>>

The conjugate of the present embodiment is a conjugate containing a polymer having a boronic acid group and a compound having a diol structure. The polymer may be a biocompatible polymer. The conjugate of the embodiment

may be a conjugate containing a biocompatible polymer having a boronic acid group and a compound having a diol structure. The conjugate of the present embodiment can be used to form the complex of the above-described embodiment.

Regarding the polymer having a boronic acid group and the compound having a diol structure, those exemplified in «Complex» described above can be adopted, and the description thereof will be omitted here.

According to the conjugate of the embodiment, since the compound having a diol structure is bonded to the polymer, it is possible to suppress in vivo an unintended interaction of the compound having a diol structure, and the stability of substance delivery is superior to a case where the compound having a diol structure is used as it is.

According to the conjugate of the embodiment, since the compound having a diol structure is bonded to the polymer, it is possible to suppress the oxidation of the compound having a diol structure, and the stability of quality is superior to a case where the compound having a diol structure is used as it is.

[Examples]

Next, the present invention will be described in more detail by showing Examples, but the present invention is not limited to Examples below.

A solution or a complex obtained by adding substances X and Y may be denoted by an X/Y solution, an X/Y complex, or simply "X/Y". Similarly, a solution or a complex obtained by adding substances X, Y, and Z may be denoted by a X/Y/Z solution, a X/Y/Z complex, simply an "X/Y/Z", an X ternary complex, a ternary complex, or the like.

In addition, PEG-P[Lys(FPBA)$_m$]$_n$ may be simply denoted by a polymer.

1. Synthesis of PEG$_{10k}$-Poly[L-Lysine(Fluoro-Phenyl boronic acid)$_m$]$_n$

< 1.1. Overview>

The synthesis method of PEG$_{10k}$-Poly[L-Lysine(Fluoro-Phenyl boronic acid)$_m$]$_n$ (hereinafter, referred to as PEG-P[Lys(FPBA)$_m$]$_n$, in the synthesis scheme (1), n indicates the degree of polymerization of Lys, and m represents the number of FPBAs introduced) produced in Examples is described.

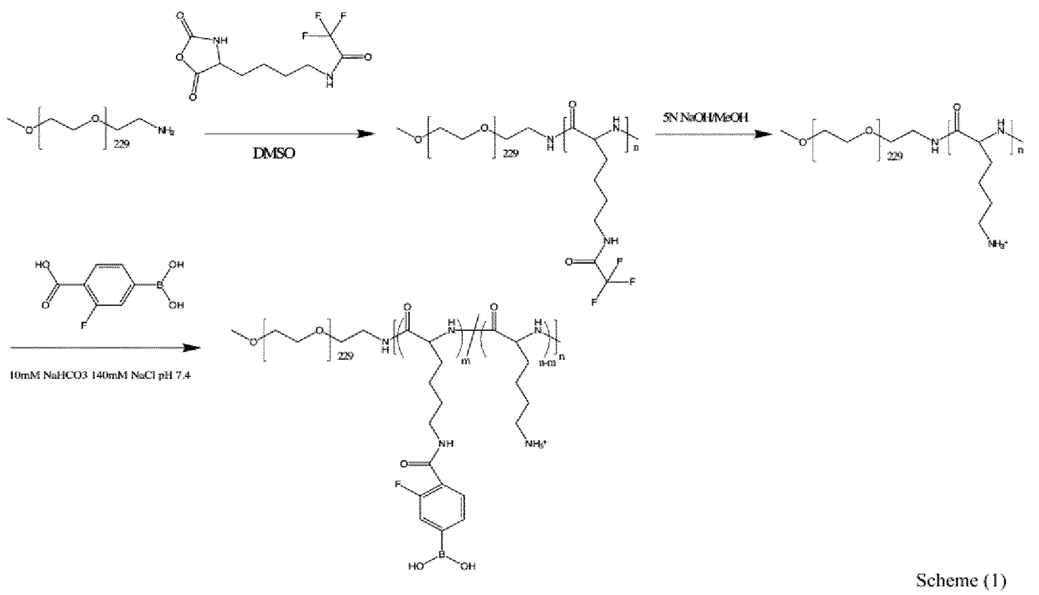


Scheme (1)

PEG-P[Lys(TFA)]$_n$ was synthesized by N-carboxyanhydride (NCA) polymerization using PEG$_{10k}$-$NH_2$ as an initiator and Lys(TFA)-NCA as a monomer. The TFA group of the side chain was deprotected under basic conditions to obtain PEG-PLys$_n$. Then, the carboxyl group of 3-carboxyl-4-fluoro-phenyl boronic acid (FPBA) was bonded to the amino group of PEG-PLys$_n$ to obtain PEG-P[Lys(FPBA)]$_n$.

<1.2. Reagent>

Regarding reagents and solvents which are not otherwise described, commercially available products were used as they were.

- α-Methoxy-ω-amino-poly(ethylene glycol) (PEG-$NH_2$) [Mn : 10K]: NOF Co, Inc.
- Benzene: Nacalai Tesque Inc.
- N-ε-Trifluoroacetyl-L-lysine-N-carboxy anhydride (Lys(TFA)-NCA): Chuo Kaseihin Co., Inc.
- Dimethyl sulfoxide(DMSO): Wako Pure Chemical Industries Co., Ltd. It was distilled in an argon atmosphere and used. (b.p.: 189°C)
- Diethyl ether: Kanto Chemical CO.,Inc.
- Methanol: Kanto Chemical CO.,Inc.
- 5 mol/L NaOH: Wako Pure Chemical Industries Co., Ltd.
- Dimethyl sulfoxide (DMSO): Nacalai Tesque Inc.
- 4-Carboxy-3-fluorophenylboronic acid (FPBA): Combi-Blocks
- Sodium hydrogen carbonate: Tokyo Chemical Industry Co., Ltd.
- D-Sorbitol: Tokyo Chemical Industry Co., Ltd.
- 4-(2-hydroxyethyl)-1-piperazineethanesulfonic acid (HEPES) : Dojinbo
- Sodium chloride(NaCl) : Wako pure chemical
- Cy5-NHS : Lumiprobe
- 1-Methyl-2-pyrrolidinone : Sigma Aldrich Co., llc.
- Lithium bromide: Sigma Aldrich Co., llc.

<1.3. Measuring apparatus>

- NMR (Nuclear Magnetic Resonance): BRUKER AVANCEIII400 (400 MHz, BRUKER BioSpin)
- GPC (Gel Permeation Chromatography): Jasco International Co., Ltd.

  Column: TSK-gel superAW3000 (Tosoh Corporation)
  Superdex 200 Increase 10/300 GL (GE Healthcare)
  Detector: RI-2031, UV-2030

- Fluorophotometer FP-8300 : Jasco International Co., Ltd.

<1.4. Synthesis method>

[Synthesis of PEG-P[Lys(TFA)]$_n$]

500 mg (0.100 mmol) of PEG-$NH_2$ was weighed and placed in a 300 mL two-necked eggplant flask, dissolved in 2.0 mL of benzene, and then freeze-dried. 321 mg (1.2 mmol, 24 equivalents) (n = 20) and 643 mg (2.4 mmol, 48 equivalents) (n = 40) of Lys(TFA)-NCA were weighed and placed in a 100 mL two-necked eggplant flask under an argon atmosphere. 5 mL of DMSO was added to PEG-$NH_2$. In addition, 10 mL DMSO was added to each Lys(TFA)-NCA to dissolve it. The Lys(TFA)-NCA solution was added to the PEG-$NH_2$ solution and stirred at room temperature for 72 hours under an argon atmosphere. Each reaction solution was added dropwise to 300 mL of diethyl ether and purified by reprecipitation. Then, the precipitate was dried under reduced pressure to obtain white solids of PEG-PLys(TFA) in yield amounts of 745 mg (n = 20) and 987 mg (n = 40) and yields of 91% (n = 20) and 86% (n = 40). GPC curves (acquired under the following conditions, column: TSK-gel superAW3000, eluent: NMP (50 mM LiBr), flow rate: 0.30 mL/min, detector: RI-2031, measurement temperature: 40°C) are shown in Fig.3 and Fig. 4.

[Deprotection of PEG-P[Lys(TFA)]$_n$]

Each of 500 mg (0.0194 mmol) of PEG-P[Lys(TFA)]$_{20}$ and 500 mg (0.0194 mmol) of PEG-P[Lys(TFA)]$_{40}$ was weighed and placed in a 50 mL eggplant flask, and added to a mixed solution of 2 mL of 5 M NaOH and 8 ml of methanol and stirred overnight at room temperature. The reaction solution was placed in a dialysis membrane (MWCO = 3.5 kDa) and dialyzed twice with 2 L of 0.1 M HCl and subsequently twice with 2 L of pure water. The solution was freeze-dried to obtain white solids of PEG-PLys$_n$ each in yield amounts of 367 mg (n = 20) and 331 mg (n = 40) and yields of 92% (n = 20) and 90% (n = 40). The $^1$H NMR spectrums (solvent: $D_2O$) are shown in Fig. 5 and Fig 6.

• $^1$H NMR spectrum of PEG-PLys$_{20}$

$^1$H NMR ($D_2O$ at 25 °C): δ 3.4-3.9 (909H, -$CH_2CH_2O$-), δ 1.25-1.99 (120H, - $CH_2CH_2CH_2CH_2NH_3$), δ 2.97

(40H, $-CH_2CH_2NH_3$), δ 4.30 (20H, -COCHNH-).

• $^1H$ NMR spectrum of PEG-$PLys_{40}$

$^1H$ NMR ($D_2O$ at 25°C): Attribution is the same as that of the above $^1H$ NMR spectrum of PEG-$PLys_{20}$.

[Bonding of FPBA to PEG-$PLys_n$]

Each of 100 mg (7.8 μmol) of PEG-$PLys_{20}$ and 100 mg (6.3 μmol) of PEG-$PLys_{40}$ was weighed and placed in a 50 mL eggplant flask and dissolved in 10 mL of 50 mM $NaHCO_3$ (pH 8.5). 61.2 mg (0.22 mmol) (n = 20) or 100 mg (0.36 mmol) (n = 40) of DMT-MM, 42 mg (0.23 mmol) (n = 20) or 69 mg (0.38 mmol) (n = 40) of D-Sorbitol, 14.3 mg (0.078 mmol) (n = 20) or 23.3 mg (0.13 mmol) (n = 40) of FPBA dissolved in 1 mL of methanol was added thereto and stirred overnight at room temperature. The reaction solution was placed in a dialysis membrane (MWCO = 3.5 kDa) and dialyzed twice with 2 L of 0.1 M NaOH, twice with 2 L of 0.1 M HCl, and subsequently twice with 2 L of pure water. The obtained solution was freeze-dried to obtain white solids of PEG-P$[Lys(FPBA)_m]_n$ in yield amounts of 126 mg (n = 20) and 144 g (n = 40) and yields of 87% (n = 20) and 80% (n = 40). $^1H$-NMR spectra (solvent: $D_2O$ with 180 mg/ml D-sorbitol) are shown in Fig. 7 and Fig. 8, and GPC curves (acquired under the following conditions, column: Superdex 200 increase 10/300 GL, eluent: 10 mM HEPES, 140 mM NaCl 500 mM, D-sorbitol (pH 7.4), flow rate: 0.75 mL/min, detector: UV-2030, measurement temperature: room temperature) are shown in Fig. 9 and Fig. 10.

• $^1H$ NMR spectrum of PEG-P[PEG-P$[Lys(FPBA)_{10}]_{20}$

$^1H$ NMR ($D_2O$ with 180 mg/mL of D-sorbitol at 25 °C): δ 0.87-2.22(120H, - $CH_2CH_2CH_2CH_2NH_3$) δ 7.00-7.70 (3H, $-C_6H_3FB(OH)_2$).

• $^1H$ NMR spectrum of PEG-P[PEG-P$[Lys(FPBA)_{20}]_{40}$

$^1H$ NMR ($D_2O$ with 180 mg/mL of D-sorbitol at 25 °C): Attribution is the same as that of the above $^1H$ NMR spectrum of PEG-P[PEG-P$[Lys(FPBA)_{10}]_{20}$.

[Introduction of Cy5 to PEG-P$[Lys(FPBA)_m]_n$]

15 mg (11 μmol) of PEG-P$[Lys(FPBA)_{10}]_{20}$ was individually weighed and placed in a 50 mL eggplant flask and dissolved in 10 mL of 50 mM $NaHCO_3$ (pH8.5). 8 mg (0.04 mmol) of D-Sorbitol and 0.7 mg (11 μmol) of Cy5-NHS dissolved in DMSO were added thereto and stirred overnight at room temperature. The reaction solution was dialyzed four times against pure water (Mwco: 3.5 kDa) and then freeze-dried. After removing unreacted Cy5-NHS with a PD-10 column (solvent: 1 M NaCl), dialysis (MWCO: 3.5 k Da) was performed three times in water. Finally, freeze-drying was performed to recover PEG-P[Lys($FPBA_{10}$/Cy5)]20. The yield was approximately 65%. The fluorescence spectrum (Ex: 560 nm) is shown in Fig. 13.

<1.5. Analysis >

[PEG-P[Lys(TFA)]]

From the GPC curves of the obtained polymers, it was determined that the Mw/Mn of PEG-P$[Lys(TFA)]_{20}$ is 1.25 and the Mw/Mn of PEG-P$[Lys(TFA)]_{40}$ is 1.29, and thus it was confirmed that the polymers have a narrow molecular weight distribution.

[PEG-$PLys_n$]

From the ratio between integrated values of a peak derived from the initiator [δ 3.4-3.9 (909H, $-CH_2CH_2O-$)] and a peak derived from Lys [δ 1.25-1.99 (120H, - $CH_2CH_2CH_2CH_2NH_3$), δ 2.97 (40H, $-CH_2CH_2NH_3$), δ 4.30 (20H, -COCHNH-)] in the $^1H$ NMR spectrum, the degree of polymerization of PLys was calculated to be DP = 20 and DP = 40.

[PEG-P$[Lys(FPBA)_m]_n$]

From the ratio between integrated values of a peak derived from PLys [δ 1.25-1.99 (120H, $-CH_2CH_2CH_2CH_2NH_3$)] and a peak derived from FPBA [δ 7.00-7.70 (3H, - $C_6H_3FB(OH)_2$)] in the $^1H$ NMR spectrum, it

was determined that the numbers of FPBA's introduced were 10 (n = 20) and 20 (n = 40), and the number average molecular weights thereof were Mn = 14,000 (n = 20) and Mn = 17,900 (n = 40). In addition, from the GPC curve, it was confirmed that the obtained polymer has a narrow molecular weight distribution having a single peak.

2. Synthesis of $PEG_{10k}$-FPBA

<2.1. Overview>

The synthesis method of a $PEG_{10k}$-Fluoro-Phenyl boronic acid (hereinafter referred to as PEG-FPBA, in the synthesis scheme (2)) produced in Examples is described.

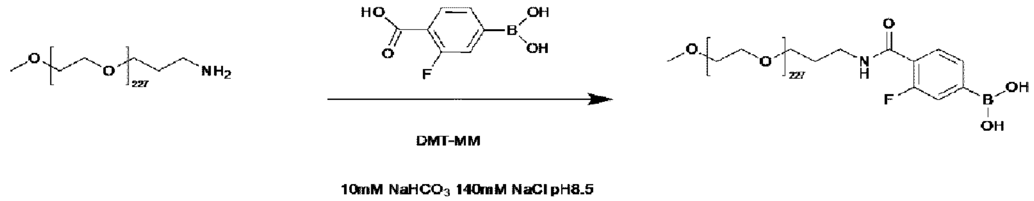


Scheme (2)

<2.2. Reagent>

Regarding reagents and solvents which are not otherwise described, commercially available products were used as they were.

- α-Methoxy-ω-amino-poly(ethylene glycol) ($PEG-NH_2$) [Mw : 10K]): NOF Co., Inc.
- 5 mol/L NaOH: Wako Pure Chemical Industries Co., Ltd.
- 4-Carboxy-3-fluorophenylboronic acid (FPBA): Combi-Blocks
- Sodium hydrogen carbonate: Tokyo Chemical Industry Co., Ltd.
- D-Sorbitol: Tokyo Chemical Industry Co., Ltd.
- 4-(2-hydroxyethyl)-1-piperazineethanesulfonic acid (HEPES) : Dojinbo
- Sodium chloride(NaCl) : Wako pure chemical

<2.3. Measuring apparatus>

- NMR (Nuclear Magnetic Resonance): BRUKER AVANCEIII400 (400 MHz, BRUKER BioSpin)
- GPC (Gel Permeation Chromatography): Jasco International Co., Ltd.

  Column: Superdex 200 Increase 10/300 GL (GE Healthcare)
  Detector: UV-2030

<2.4. Synthesis method>

[Bonding of FPBA to $PEG-NH_{2]}$

100 mg (0.01 mmol) of $PEG-NH_2$ was weighed and placed in a 50 mL eggplant flask, and dissolved in 50 mM $NaHCO_3$ (pH8.5). 13.8 mg (0.05 mmol) of DMT-MM, 27 mg (0.15 mmol) of D-Sorbitol, and 9.2 mg (0.05 mmol) of FPBA dissolved in 1 mL of methanol were added thereto and stirred overnight at room temperature. The reaction solution was placed in a dialysis membrane (MWCO = 3.5 kDa) and dialyzed twice with 2 L of 0.1 M NaOH, twice with 2 L of 0.1 M HCl, and subsequently twice with 2 L of pure water. The obtained solution was freeze-dried to obtain a white solid of $PEG-P[Lys(FPBA)_m]_n$ in a yield amount of 90 mg and a yield of 88%. A $^1$H-NMR spectrum is shown in Fig. 11, and a GPC curve (acquired under the following conditions, column: Superdex 200 increase 10/300 GL, eluent: 10 mM HEPES, 140 mM NaCl, 500 mM D-sorbitol (pH 7.4), flow rate: 0.75 mL/min, detector: UV-2030, measurement temperature: room temperature) are shown in Fig. 12.

- $^1H$ NMR spectrum of PEG-FPBA
- $^1H$ NMR spectrum of PEG-P[PEG-P[Lys(FPBA)$_{10}$]$_{20}$

$^1H$ NMR (d-DMSO at 25°C): δ 3.4-3.9 (909H, $-CH_2CH_2O-$), δ 7.00-7.70 (3H, - $C_6H_3FB(OH)_2$).

<2.5. Analysis>

[PEG-FPBA]

From the ratio between integrated values of a peak derived from PEG [δ 3.4-3.9 (909H, $-CH_2CH_2O-$)] and a peak derived from FPBA [δ 7.00-7.70 (3H, $-C_6H_3FB(OH)_2$)] in the $^1H$ NMR spectrum, the introduction rate of FPBA was determined to be 100 %. In addition, from the GPC curve, it was confirmed that the obtained polymer has a narrow molecular weight distribution having a single peak.

3. Evaluation of physicochemical properties of ternary complex

<3.1. Overview>

In a case where a ternary complex of protein, tannic acid (TA), and a boronic acid-introduced polymer is formed, the particle diameter increases. For this reason, the particle diameter was measured by fluorescence correlation spectroscopy using a green fluorescent protein (GFP) as a model protein. As Examples, PEG-P[Lys(FPBA)$_m$]$_n$ and PEG-FPBA were used. At the same time, the number of associations of PEG-P[Lys(FPBA)$_m$]$_n$ was evaluated using an ultracentrifuge. In addition, the particle diameter changes in a FBS solution and a glucose solution were also measured to evaluate the stability in the blood environment. Further, in order to check the pH responsiveness in the periphery of the tumor and the inside of the cell, the particle diameter change in a case where the pH was changed was also measured.

<3.2. Reagent>

Regarding reagents and solvents which are not otherwise described, commercially available products were used as they were.

- Green fluorescent protein (rGFP Protein, Mw: 33k Da): Clontech Laboratories, Inc.
- PEG-P[Lys(FPBA)$_{10}$]$_{20}$ (Mn = 14,000)
- PEG-P[Lys(FPBA)$_{20}$]$_{40}$ (Mn = 17,900)
- Tannic acid: (Mw = 1,701) Wako Pure Chemical., Ltd.
- D-PBS (-): Wako Pure Chemical., Ltd.
- 5 mol/L HCl: Wako Pure Chemical Industries Co., Ltd.

<3.3. Measuring apparatus>

- LSM710: Carl Zeiss Co., Ltd.
- CS 150GX: Hitachi
- Fluorophotometer FP-8300 : Jasco International Co., Ltd.

<3.4. Evaluation of complex formation due to addition of boronic acid-introduced polymer>

[Final concentrations of GFP, TA, PEG-FPBA, and PEG-P[Lys(FPBA)$_m$]$_n$]

- GFP: 0.5 μM
- Tannic acid: 40 μM (adjusted concentration: 82.5 μM)
- Structure derived from FPBA contained in PEG-P[Lys(FPBA)$_m$]$_n$(n = 20, m = 10, or n = 40, m = 20): 250 μM or 500 μM (calculated by FPBA concentration)
- PEG-FPBA: 250 μM or 500 μM

Each of these was adjusted by being dissolved in D-PBS (-).

The GFP solution and the tannic acid solution were mixed and centrifuged twice at 10,000 g × 5 minutes using an ultrafiltration membrane (MWCO: 10 kDa) to adjust a GFP/TA solution. Then, a PEG-P[Lys(FPBA)$_{10}$]$_{20}$ solution, a PEG-P[Lys(FPBA)$_{20}$]$_{40}$ solution, or a PEG-FPBA solution was added to a separate GFP/TA solution to adjust a GFP/TA/PEG-P[Lys(FPBA)$_m$]$_n$ (n = 20, m = 10, or n = 40, m = 20) solution or a GFP/TA/PEG-FPBA solution. Using a confocal microscope LSM710 (manufactured by Carl Zeiss AG), the particle diameter (the arithmetic mean diameter based on the number of particles) of the particles contained in each solution was measured by fluorescence correlation spectroscopy.

First, the diffusion time of the fluorescent molecule to be measured was calculated with a confocal microscope. Since the diffusion coefficient × diffusion time is constant, the diffusion time of Rhodamine 6G (diffusion coefficient: 4.14 × $10^{-10}$ $m^2$/sec, 25°C), the diffusion coefficient of which is known, was measured at the same time, and the diffusion coefficient of the fluorescent molecule to be measured was calculated. The calculated value was substituted into the Einstein-Stokes equation to calculate the particle diameter. The Einstein-Stokes equation is as follows.

$$D = K_B T / 6\pi\eta r$$

D: Diffusion coefficient
$K_B$: Boltzmann constant (1.38 × $10^{-23}$ $m^2 \cdot kg/s^2 \cdot K$)
T: Temperature (298 K)
η: Viscosity (0.00089 Pa·s)
r: Particle radius (nm)

The results are shown in Table 14.

The particle diameters of particles contained in the GFP/TA/PEG-FPBA solution and the GFP/TA/PEG-P[Lys(FPBA)$_m$]$_n$ (n = 20 or n = 40, m = 10 or m = 20) solution each increased as compared with the particle diameter of the particles contained in the GFP solution and the GFP/TA solution, suggesting the formation of the ternary complex of GFP, TA, and the boronic acid-introduced polymer.

Among the above, the particle diameter of the particles contained in the GFP/TA/PEG-P[Lys(FPBA)$_m$]$_n$ (n = 20, m = 10, or m = 40, m = 20) solution was significantly increased, suggesting the remarkable formation of the ternary complex thereof.

<3.5. Evaluation of ternary complex formation due to addition of tannic acid>

[Final concentrations of GFP, TA, and PEG-P[Lys(FPBA)$_{10}$]$_{20}$

- GFP: 0.5 μM
- Tannic acid: 40 μM (adjusted concentration: 82.5 μM)
- Structure derived from FPBA contained in PEG-P[Lys(FPBA)$_{10}$]$_{20}$: 250 μM (calculated by FPBA concentration)

Each of these was adjusted by being dissolved in D-PBS (-).

The GFP solution and the tannic acid solution were mixed and centrifuged twice at 10,000 g × 5 minutes using an ultrafiltration membrane (MWCO: 3.5 kDa) to adjust a GFP/TA solution. Then, a PEG-P[Lys(FPBA)$_{10}$]$_{20}$ solution was added to a separate GFP/TA solution to adjust a GFP/TA/PEG-P[Lys(FPBA)$_{10}$]$_{20}$ solution, and PEG-P[Lys(FPBA)$_{10}$]$_{20}$ was added to the GFP solution to adjust a GFP/PEG-P[Lys(FPBA)$_{10}$]$_{20}$ solution. Fig. 15 shows the results of measuring the particle diameter of the particles contained in each solution by fluorescence correlation spectroscopy using LSM710.

The particle diameter of the particles contained in the GFP/TA/PEG-P[Lys(FPBA)$_{10}$]$_{20}$ solution significantly increased as compared with the particle diameter of the particles contained in each of the GFP solution, the GFP/TA solution, and the GFP/PEG-P[Lys(FPBA)$_{10}$]$_{20}$ solution, and thus it was confirmed that the GFP/TA/PEG-P[Lys(FPBA)$_m$]$_n$ complex was formed by the ternary system thereof.

<3.6. Evaluation of complex formation in glucose>

[Final concentrations of GFP, TA, and PEG-P[Lys(FPBA)$_{10}$]$_{20}$

- GFP: 0.5 μM
- Tannic acid: 40 μM (adjusted concentration: 82.5 μM)
- Structure derived from FPBA contained in PEG-P[Lys(FPBA)$_{10}$]$_{20}$: 250 μM (calculated by FPBA concentration)

Each of these was adjusted by being dissolved in D-PBS (-) and D-PBS (-) solutions containing 0.1 mg/ml, 1.0 mg/ml, and 10.0 mg/ml glucose.

The GFP solution and the tannic acid solution were mixed and centrifuged twice at 10,000 g × 5 minutes using an ultrafiltration membrane (MWCO: 3.5 kDa) to adjust a GFP/TA solution. Then, PEG-P[Lys(FPBA)$_{10}$]$_{20}$ was added to a separate GFP/TA solution to adjust a GFP/TA/PEG-P[Lys(FPBA)$_{10}$]$_{20}$ solution at the above glucose concentration. Fig. 16 shows the results of subjecting the solutions to the measurement of the particle diameter of the particles contained in each solution by fluorescence correlation spectroscopy using LSM710.

The particle diameter of the particles contained in the GFP/TA/PEG-P[Lys(FPBA)$_{10}$]$_{20}$ solution did not change remarkably in the glucose solution of each concentration, and thus it was confirmed that the GFP/TA/PEG-P[Lys(FPBA)$_{10}$]$_{20}$ ternary complex is stable in glucose solution.

<3.7. Evaluation of complex formation in FBS>

[Final concentrations of GFP, TA, and PEG-P[Lys(FPBA)$_{10}$]$_{20}$

- GFP: 0.5 μM
- Tannic acid: 40 μM (adjusted concentration: 82.5 μM)
- Structure derived from FPBA contained in PEG-P[Lys(FPBA)$_{10}$]$_{20}$: 250 μM (calculated by FPBA concentration)

Each of these was adjusted by being dissolved in D-PBS (-) and then adjusted by adding FBS/D-PBS (-) to a mixed solution which had been obtained by mixing FBS/D-PBS (-) at the following volume ratio (5/95, 10/90, 30/70, 50/50, 75/25 (vol)) so that the concentration thereof was at the above final concentration.

The GFP solution and the tannic acid solution were mixed and centrifuged twice at 10,000 g × 5 minutes using an ultrafiltration membrane (MWCO: 10 kDa) to adjust a GFP/TA solution. Then, PEG-P[Lys(FPBA)$_{10}$]$_{20}$ was added to a separate GFP/TA solution to adjust a GFP/TA/PEG-P[Lys(FPBA)$_{10}$]$_{20}$ solution at the above FBS concentration. Fig. 17 shows the results of subjecting the solutions to the measurement of the particle diameter of the particles contained in each solution by fluorescence correlation spectroscopy using LSM710.

The particle diameter of the particles contained in the GFP/TA/PEG-P[Lys(FPBA)$_{10}$]$_{20}$ solution did not change remarkably in the FBS solution of each concentration, and thus it was confirmed that the GFP/TA/PEG-P[Lys(FPBA)$_{10}$]$_{20}$ ternary complex is stable in FBS solution.

<3.8. Evaluation of pH responsiveness of complex>

[Final concentrations of GFP, TA, and PEG-P[Lys(FPBA)$_{10}$]$_{20}$

- GFP: 0.5 μM
- Tannic acid: 40 μM (Preparation concentration: 82.5 μM)
- Structure derived from FPBA contained in PEG-P[Lys(FPBA)$_{10}$]$_{20}$: 250 μM (calculated by FPBA concentration)

Each of these was adjusted by being dissolved in pH 7.4 D-PBS (-), pH 6.6 D-PBS (-), and pH 5.5 D-PBS (-), which had been adjusted with HCl.

The GFP solution and the tannic acid solution were mixed and centrifuged twice at 10,000 g × 5 minutes using an ultrafiltration membrane (MWCO: 10 kDa) to adjust a GFP/TA solution. Then, PEG-P[Lys(FPBA)$_{10}$]$_{20}$ was added to a separate GFP/TA solution to adjust a GFP/TA/PEG-P[Lys(FPBA)$_{10}$]$_{20}$ solution in the above pH. Fig. 18 shows the results of subjecting the solutions to the measurement of the particle diameter of the particles contained in each solution by fluorescence correlation spectroscopy using LSM710.

The particle diameter of the particles contained in the GFP/TA/PEG-P[Lys(FPBA)$_{10}$]$_{20}$ solution adjusted to a pH of 6.6 decreased as compared with particle diameter of the particles contained in the GFP/TA/PEG-P[Lys(FPBA)$_{10}$]$_{20}$ adjusted to a pH of 7.4. In addition, the particle diameter of the particles contained in the GFP/TA/PEG-P[Lys(FPBA)$_{10}$]$_{20}$ solution adjusted to a pH of 5.5 was equivalent to the particle diameter of GFP, and thus it was confirmed that the

GFP/TA/PEG-P[Lys(FPBA)$_{10}$]$_{20}$ ternary complex has the pH responsiveness by which the formation state of the complex is changed depending on the pH, since it is conceived that GFP/TA/PEG-P[Lys(FPBA)$_{10}$]$_{20}$] is eliminated at the pH (pH 6.6) in the periphery of the tumor and the pH (pH 5.5) in the inside of the cell.

<3.9. Evaluation of number of associations of PEG-P[Lys(FPBA$_{10}$/Cy5)]$_{20}$ in complex>

[Final concentrations of GFP, TA, and PEG-P[Lys(FPBA$_{10}$/Cy5)]$_{20}$]

- GFP: 0.5 μM
- Tannic acid: 40 μM (Preparation concentration: 82.5 μM)
- Structure derived from FPBA contained in PEG-P[Lys(FPBA$_{10}$/Cy5)]$_{20}$: 250 μM (calculated by FPBA concentration)

Each of these was adjusted by being dissolved in D-PBS (-).

The GFP solution and the tannic acid solution were mixed and centrifuged twice at 10,000 g × 5 minutes using an ultrafiltration membrane (MWCO: 10 kDa) to adjust a GFP/TA solution. Then, the PEG-P[Lys(FPBA)$_{10}$/Cy5]$_{20}$ solution was added to a separate GFP/TA solution to adjust a GFP/TA/PEG-P[Lys(FPBA)$_{10}$/Cy5]$_{20}$ solution. A precipitate was generated by ultracentrifuging the solution with an ultracentrifuge (CS 150GX) at 50,000 g × 1 h. The precipitate selectively contains the GFP/TA/PEG-P[Lys(FPBA$_{10}$/Cy5)]$_{20}$ complex having a large sedimentation coefficient. The precipitate was dissolved in 1 ml of D-PBS (-), subjected to the measurement of the fluorescence spectrum (Ex: 640 nm / Em: 680 nm) to calculate the concentration, whereby the number of associations of PEG-P[Lys(FPBA$_{10}$/Cy5)]$_{20}$ per GFP molecule was measured. The results are shown in Table 1.

[Table 1]

| | PEG-FPBA | PEG-P[Lys(FPBA$_{10}$/Cy5)]$_{20}$ |
|---|---|---|
| Number of associations | N.D. | 8.9 |

In the GFP/TA/PEG-P[Lys(FPBA$_{10}$/Cy5)]$_{20}$ complex, it was confirmed that 8.9 pieces, on average, of PEG-P[Lys(FPBA$_{10}$/Cy5)]$_{20}$ were associated with one GFP molecule.

4. Evaluation of bonding force between tannic acid and PEG-P[Lys(FPBA)$_m$]$_n$ by alizarin red method

<4.1. Overview>

The bonding force was evaluated by the alizarin red method, which has been established as a method for quantifying the bonding force between boronic acid and the diol structure. The principle of the alizarin red method is briefly shown below by taking the method carried out in present Examples as an example.

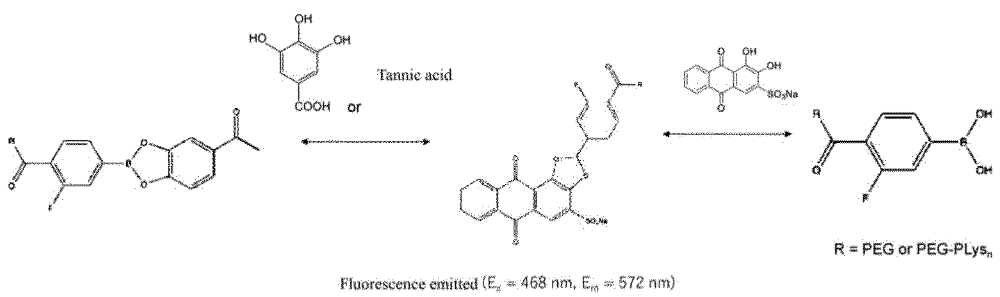


<4.2. Reagent>

- Green fluorescent protein (rGFP Protein): Clontech Laboratories, Inc.
- PEG-P[Lys(FPBA)$_{10}$]$_{20}$ (Mn = 14,000)

- PEG-P[Lys(FPBA)$_{20}$]$_{40}$ (Mn = 17,900)
- Tannic acid: Wako Pure Chemical Industries Co., Ltd.
- D-PBS(-): Wako Pure Chemical Industries Co., Ltd.
- Gallic acid: Wako Pure Chemical., Ltd.
- Alizarin Red S: Wako Pure Chemical Industries Co., Ltd.

<4.3. Measuring apparatus>

- Fluorophotometer FP-8300: Jasco International Co., Ltd.

<4.4. Evaluation of complex formation>

[Final concentrations of GFP, TA, PEG-FPBA, and PEG-P[Lys(FPBA)$_{10}$]$_{20}$]

- Solution A: ARS ($9.0 \times 10^{-6}$ M)
- Solution B: ARS ($9.0 \times 10^{-6}$ M) + PEG-FPBA (FPBA concentration: $2.0 \times 10^{-3}$ M)
- Solution C: ARS ($9.0 \times 10^{-6}$ M) + PEG-P[Lys(FPBA)$_{10}$]$_{20}$ (FPBA concentration: $2.0 \times 10^{-3}$ M)
- Solution D: ARS ($9.0 \times 10^{-6}$ M) + PEG-FPBA (FPBA concentration: $2.0 \times 10^{-3}$ M) + tannic acid (diol concentration = $5.0 \times 10^{-4}$ M)
- Solution E: ARS ($9.0 \times 10^{-6}$ M) + PEG-FPBA ($2.0 \times 10^{-3}$ M) + gallic acid (diol concentration = $5.0 \times 10^{-4}$ M)
- Solution F: ARS ($9.0 \times 10^{-6}$ M) + PEG-P[Lys(FPBA)$_{10}$]$_{20}$ (FPBA concentration: $2.0 \times 10^{-3}$ M) + tannic acid (diol concentration = $5.0 \times 10^{-4}$ M)
- Solution G: ARS ($9.0 \times 10^{-6}$ M) + PEG-P[Lys(FPBA)$_{10}$]$_{20}$ (FPBA concentration: $2.0 \times 10^{-3}$ M) + gallic acid (diol concentration = $5.0 \times 10^{-4}$ M)

Solution A, and Solution B or Solution C were mixed in various ratios and the fluorescence measurement was performed (Ex = 468 nm, Em = 572 nm) using a disposable cell. After substituting the obtained fluorescence intensity and the concentration of each FPBA into the following expression (1) and creating a calibration curve by the least squares method, the equilibrium constant $K_0$ of the ARS-FPBA system was calculated from the slope of the calibration curve. Next, Solution B or Solution C was mixed with Solution D, E, F, or G containing each diol compound in various ratios in the combination of B + D, B + E, C + F, and C + G, and the fluorescence measurement was performed (Ex = 468 nm, Em = 572 nm) using a disposable cell. After substituting the obtained fluorescence intensity and the concentration of each diol compound into the following expression (2) and creating a calibration curve by the least squares method, the equilibrium constant $K_1$ of each diol compound-BPA system was calculated from the slope of the calibration curve. The equilibrium constant obtained from the calibration curve is shown in Table 2 as a relative equilibrium constant.

$$\frac{1}{\Delta I_f} = \frac{1}{[\mathrm{L}_0]\,K_0} \cdot \frac{1}{\Delta I_{fc}} + \frac{1}{\Delta I_{fc}} \quad \cdots\cdots\cdots (1)$$

$$\frac{[\mathrm{S}_0]}{P} = \frac{\mathrm{K}_0}{\mathrm{K}_1}\mathrm{Q} + 1 \qquad \mathrm{Q} = \frac{\Delta I_{fc}}{\Delta I_f} - 1$$

$$P = [\mathrm{L}_0] - \frac{1}{Q\,K_0} - \frac{[I_0]}{Q+1} \quad \cdots\cdots\cdots (2)$$

$[L_0]$ = Concentration of FPBA added

[So] = Concentration of diol added

$[I_0]$ = Concentration of ARS

$$\Delta I_f = I_{fs} - I_{fa}$$

$\Delta I_{fc}$ = Reciprocal of intercept of graph created in ARS-FPBA system

$I_{fs}$ - Fluorescence intensity of ARS-FPBA
$I_{fa}$ - Fluorescence intensity of solution A
$K_0$ =Equilibrium constant of ARS-FPBA

$K_I$ = Equilibrium constant of diol compound - equilibrium constant of FPBA

[Table 2]

| Galloyl group | PEG-FPBA | PEG10k-P[Lys(FPBA)$_{10}$]$_{20}$ |
|---|---|---|
| Gallic acid | 2,030 | 5,112 |
| Tannic acid (TA) | 1,877 | 8,827 |

The bonding constant between gallic acid and PEG-P[Lys(FPBA)$_{10}$]$_{20}$ was high as compared with the bonding constant between gallic acid and PEG-FPBA by 2.5 times. In addition, the bonding constant between tannic acid and PEG-P[Lys(FPBA)$_{10}$]$_{20}$ was high as compared with the bonding constant between tannic acid and PEG-FPBA by 5 times.

5. Evaluation in cultured cell

<5.1. Overview>

The intracellular distribution of GFP was observed with a confocal microscope to confirm the intracellular incorporation pathway.

<5.2. Reagent and cell line>

Regarding reagents which are not otherwise described, commercially available products were used as they were.

- Green fluorescent protein (rGFP Protein, Mw: 33k Da): Clontech Laboratories, Inc.
- PEG-P[Lys(FPBA)$_{10}$]$_{20}$ (Mn = 14,000)
- Tannic acid: (Mw = 1,701) Wako Pure Chemical., Ltd.
- D-PBS (-): Wako Pure Chemical., Ltd.
- Roswell Park Memorial Institute medium (RPMI): Sigma Aldrich Co., 11c.
- Fetal bovine serum (FBS): Biosera Inc.
- Trypsin-EDTA solution: Sigma life science Co., Ltd.
- Penicillin/streptomycin: Sigma life science Co., Ltd.
- 5 mol/L HCl: Wako Pure Chemical Industries Co., Ltd.
- CT26 cell (mouse colon carcinoma cell line): American Type Culture Collection
- LysoTracker (registered trade name) red DND-99: Thermo Fisher Scientific Inc.
- Hoechst 33342: Thermo Fisher Scientific Inc.
- Paraformaldehyde: Nacalai Tesque Inc.

It was used as a 4% a paraformaldehyde/D-PBS (-) solution.

<5.3. Measuring apparatus>

- Countess: Thermo Fisher Scientific Inc.
- LSM710: Carl Zeiss Co., Ltd.

<5.4. Observation of intracellular distribution of GFP with confocal microscope>

[Final concentrations of GFP, TA, and PEG-P[Lys(FPBA)$_{10}$]$_{20}$

- GFP: 0. 5 μM
- Tannic acid: 40 μM (adjusted concentration: 82.5 μM)
- Structure derived from FPBA contained in PEG-P[Lys(FPBA)$_{10}$]$_{20}$: 250 μM (calculated by FPBA concentration)

Each of these was adjusted by being dissolved in D-PBS (-).

The GFP solution and the tannic acid solution were mixed and centrifuged twice at 10,000 g × 5 minutes using an ultrafiltration membrane (MWCO: 10 kDa) to adjust a GFP/TA solution. Then, the PEG-P[Lys(FPBA)$_{10}$]$_{20}$ solution was added to a separate GFP/TA solution to adjust a GFP/TA/PEG-P[Lys(FPBA)$_{10}$]$_{20}$ solution.

[Observation with confocal microscope]

CT26 cells were seeded in on 35 $mm^2$ glass base dish at 5.0 × $10^4$ cells/dish and pre-cultured at 37°C and under 5% $CO_2$ for 24 hours. 200 μL of the above GFP/TA/PEG-P[Lys(FPBA)$_{10}$]$_{20}$ solution was added to each dish and incubated for 6 hours. After washing with 1 mL of D-PBS (-), 1 mL of a 100 nM LysoTracker (registered trade name) red DND-99/(D-PBS (-): RPMI = 1:9) solution was added thereto followed by incubating for 30 min. After washing with 1 mL of D-PBS (-), the mixture was incubated with 4% a paraformaldehyde/D-PBS (-) solution for 4 minutes. After washing with 1 mL of D-PBS (-), 1 mL of a 5.0 μg/mL Hoechst/D-PBS (-) solution was added thereto followed by incubating for 5 min. After washing twice with 1 mL of D-PBS (-), 2 mL of RPMI was added thereto and observed with CLSM. The obtained results are shown in Fig. 19.

Since GFP was localized in endosomes/lysosomes, it was suggested that GFP was incorporated into the cell by endocytosis.

5. Effect on subcutaneous tumor model mouse (blood retention and tumor accumulation)

<5.1. Overview>

The pharmacokinetics of the GFP/TA/PEG-P[Lys(FPBA)$_{10}$]$_{20}$ complex in a CT26 (mouse colon cancer cell) subcutaneous tumor model mouse were evaluated.

<5.2. Reagent, cell, and animal>

- Green fluorescent protein (rGFP Protein, Mw: 33k Da): Clontech Laboratories, Inc.
- PEG-P[Lys(FPBA)$_{10}$]$_{20}$ (Mn = 14,000)
- Tannic acid: (Mw = 1,701) Wako Pure Chemical Industries Co., Ltd.
- CT-26 cell (mouse colon carcinoma cell line): American Type Culture Collection
- BALB/c mice: Charles River Japan Inc.
- GFP ELISA Kit (ab171581): abcam

An Extraction Buffer, a 96well plate, antibody solution, a Wash Buffer, 3,3',5,5'-tetramethylbenzidine (TMB), and a Stop solution are included.

<5.3. Apparatus/equipment>

- Countess: Thermo Fisher Scientific Inc.
- iMark: BioRAD

<5.4. Pharmacokinetics of GFP/TA/PEG-P[Lys(FPBA)$_{10}$]$_{20}$ complex>

To evaluate the blood retention and the tumor accumulation of GFP, GFP/TA, and GFP/TA/PEG-P[Lys(FPBA)$_{10}$]$_{20}$, GFP/TA/PEG-P[Lys(FPBA)$_{10}$]$_{20}$ was intravenously injected into a CT26 subcutaneous tumor model mouse, and the GFP content of blood and tumor after a certain period of time was measured by ELISA.

[Final concentrations of GFP, TA, and PEG-P[Lys(FPBA)$_{10}$]$_{20}$

- GFP: 2.2 μM
- Tannic acid: 350 μM
- Structure derived from FPBA contained in PEG-P[Lys(FPBA)$_{10}$]$_{20}$: 1 mM (calculated by FPBA concentration)

Each of these was adjusted by being dissolved in D-PBS (-).
The GFP solution and the tannic acid solution were mixed and centrifuged twice at 10,000 g × 5 minutes using an ultrafiltration membrane (MWCO: 10 kDa) to adjust a GFP/TA solution. Then, the PEG-P[Lys(FPBA)$_{10}$]$_{20}$ solution was added to a separate GFP/TA solution to adjust a GFP solution, a GFP/TA solution, and a GFP/TA/PEG-P[Lys(FPBA)$_{10}$]$_{20}$ solution.

[Preparation of CT26 subcutaneous tumor model mouse]

100 μl of a CT26 cell suspension (1.0 × $10^6$ cells/ml) was subcutaneously injected into a BALB/c mouse.

[Evaluation of pharmacokinetics]

100 μl of the prepared solution described above was intravenously administered to the tail vein of a model mouse of which the tumor size reached about 200 $mm^3$. Two, six, and twenty-four hours after the sample administration, dissection was carried out to recover blood and various organs, Cell Extraction Buffer of 5 times the weight thereof was added thereto, and incubated on ice for 20 minutes. Then, centrifugation was performed at 18,000 g × 20 minutes at 4°C, the supernatant was diluted with Cell Extraction Buffer, 50 μl of the diluted supernatant was added to a 96-well plate of the GFP ELISA Kit, antibody solution was added thereto, and the plate was shaken at 1 h × 400 rpm at room temperature. Next, after washing 3 times with 350 μl of Wash Buffer, 100 μl of TMB was added, and the plate was shaken at room temperature for 10 minutes, and then the Stop solution was added. Then, the absorbance at 450 nm was measured with a plate reader, the concentration of GFP was calculated from the calibration curve, and then the pharmacokinetics was evaluated. The results are shown in Fig. 20 and Fig. 21.
The concentrations of GFP and GFP/TA in blood were about 5.0% and 1.5%, 2 hours and 6 hours after administration, showing the rapid disappearance from the blood, whereas GFP/TA/PEG-P[Lys(FPBA)$_{10}$]$_{20}$ had a concentration of 15% in blood 6 hours after administration and 3.8% 24 hours after administration, which were significantly high. Furthermore, GFP/TA/PEG-P[Lys(FPBA)$_{10}$]$_{20}$ had a tumor accumulation 2.5 times, 5.5 times, and 10 times more than GFP at 2,6, and 24 hours later. From these results, it was shown that the protein delivery system composed of GFP/TA/PEG-P[Lys(FPBA)$_{10}$]$_{20}$ achieves tumor accumulation and retention in addition to the improvement of blood retention.

6. Confirmation of formation of ternary complex encapsulating various substances

<6.1. Overview>

Ternary complexes were formed using not only the GFP protein but also a small molecule medicine, a peptide, an adeno-associated virus, an inorganic particle, a nucleic acid, and the like, and the particle diameter change was measured. As the measurement method, dynamic light scattering (DLS) or fluorescence correlation spectroscopy (FCS) was used.

<6.2. Reagent>

Regarding reagents and solvents which are not otherwise described, commercially available products were used as they were.

- PEG-P[Lys(FBPA)$_{10}$]$_{20}$ (Mn = 14,000)
- Tannic acid: (Mw = 1,701) Wako Pure Chemical Industries Co., Ltd.
- D-PBS(-): Wako Pure Chemical Industries Co., Ltd.
- Bleomycin sulfate (simply abbreviated as bleomycin): 1513.6 g/mol, Tokyo Chemical Industry Co., Ltd.
- Rose bengal: 973.67 g/mol, Tokyo Chemical Industry Co., Ltd.
- Chlorin e6: 596.7 g/mol, Cayman Chemical Co., Ltd.
- Pitavastatin calcium (simply abbreviated as pitavastatin): 880.98 g/mol, Wako Pure Chemical Industries Co., Ltd.
- Gelonin: Mw about 30 kDa, Enzo Life Sciences, Inc.
- Pseudomonas exotoxin A (PE): Mw about 60kDa, Sigma Aldrich Co., 11c.
- Green fluorescent protein (rGFP Protein, Mw: 33k Da): Clontech Laboratories, Inc.
- β-D-galactosidase (βGal): Mw 540 kDa, Wako Pure Chemical Industries Co., Ltd.
- FITC-LC-Antennapedia Peptide (simply abbreviated as Peptide): 2748.3 g/mol, Anaspec Inc.

  - AAV9-CMV-Luc (simply abbreviated as AAV): SignaGen Laboratories.

- Gold nanoparticle (AuNP): particle diameter: 15 nm, 0.050 mg/mL, 2.3 nM, BBI Solutions.
- Alexa Fluor647-TUG1 (TUG1 antisense nucleic acid, simply abbreviated as TUG1): 8,058.7 g/mol, GeneDesign, Inc.

<6.3. Measuring apparatus>

(Fluorescence correlation spectroscopy)

- LSM710: Carl Zeiss Co., Ltd.
  Temperature: 25°C, measurement time: 10 seconds, number of integrations: 10 times

(Dynamic light scattering method)

- Zetasizer Nano ZS (Zetasizer): Malvern Instruments
  Temperature: 25 °C, measurement time: 10 seconds, number of integrations: 10 times

The method for measuring dynamic light scattering is as follows. Dynamic Light Scattering (DLS Zetasizer Nano ZS (manufactured by Malvern Instruments) was used to carry out DLS measurement at a detection angle of 173° and a temperature of 25 °C. A He-Ne laser (633 nm) was used as the incident beam. Each complex solution was added to a small glass cuvette (capacity: 12 μL, ZEN2112, manufactured by Malvern Instruments). The data obtained from the decay rate in the photon correlation function was analyzed by the Cumulant method, and then the hydrodynamic diameter (the arithmetic mean diameter based on the number of complexes) of each complex was calculated by the above Einstein-Stokes equation.

<6.4. Evaluation of bleomycin ternary complex formation>

[Final concentrations of bleomycin, TA, and PEG-P[Lys(FPBA)$_{10}$]$_{20}$]

- Bleomycin: 0.02 mg/mL
- Tannic acid: 0.2 mg/mL
- PEG-P[Lys(FBPA)$_{10}$]$_{20}$: 3.3 mg/mL

Each of these was adjusted by being dissolved in D-PBS (-).

The bleomycin solution and the tannic acid solution were mixed to adjust a bleomycin/TA solution. Then, PEG-P[Lys(FPBA)$_{10}$]$_{20}$ was added to the bleomycin/TA solution to adjust a bleomycin/TA/PEG-P[Lys(FPBA)$_{10}$]$_{20}$ (a bleomycin ternary complex) solution. Table 3 shows the results of particle diameter measurement using Zetasizer.

The particle diameter of the bleomycin ternary complex was clearly increased as compared with the particle diameter of bleomycin alone, confirming the formation of the bleomycin ternary complex.

<6.5. Evaluation of rose bengal ternary complex formation>

[Final concentrations of rose bengal, TA, and PEG-P[Lys(FPBA)$_{10}$]$_{20}$]

- Rose bengal: 0.02 mg/mL
- Tannic acid: 0.2 mg/mL
- PEG-P[Lys(FBPA)$_{10}$]$_{20}$: 3.3 mg/mL

Each of these was adjusted by being dissolved in D-PBS (-).
The structure of rose bengal used in Examples is shown below.

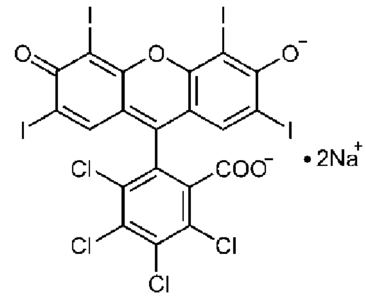

The rose bengal solution and the tannic acid solution were mixed to adjust a rose bengal/TA solution. Then, PEG-P[Lys(FPBA)$_{10}$]$_{20}$ was added to the rose bengal/TA solution to adjust a rose bengal/TA/PEG-P[Lys(FPBA)$_{10}$]$_{20}$ (a rose bengal ternary complex) solution. Table 3 shows the results of particle diameter measurement using Zetasizer.
The particle diameter of the rose bengal ternary complex was clearly increased as compared with the particle diameter of rose bengal alone, confirming the formation of the rose bengal ternary complex.

<6.6. Evaluation of Chlorin e6 ternary complex formation>

[Final concentrations of Chlorin e6, TA, and PEG-P[Lys(FPBA)$_{10}$]$_{20}$]

- Chlorin e6: 1 $\mu$M
- Tannic acid: 15 $\mu$M
- PEG-P[Lys(FBPA)$_{10}$]$_{20}$: 30 $\mu$M

Each of these was adjusted by being dissolved in D-PBS (-).
The Chlorin e6 solution and the tannic acid solution were mixed to adjust a Chlorin e6/TA solution. Then, PEG-P[Lys(FPBA)$_{10}$]$_{20}$ was added to the Chlorin e6/TA solution to adjust a Chlorin e6/TA/PEG-P[Lys(FPBA)$_{10}$]$_{20}$ (a Chlorin e6 ternary complex) solution. Table 3 shows the results of particle diameter measurement by FCS using LSM710.
The particle diameter of the Chlorin e6 ternary complex was clearly increased as compared with the particle diameter of Chlorin e6 alone, confirming the formation of the Chlorin e6 ternary complex.

<6.7. Evaluation of pitavastatin ternary complex formation>

[Final concentrations of pitavastatin, TA, and PEG-P[Lys(FPBA)$_{10}$]$_{20}$]

- Pitavastatin: 0.02 mg/mL
- Tannic acid: 0.2 mg/mL
- PEG-P[Lys(FBPA)$_{10}$]$_{20}$: 3.3 mg/mL

Pitavastatin was adjusted by being dissolved D-PBS (-) containing 5% THF, and TA and PEG-P[Lys(FBPA)$_{10}$]$_{20}$

were adjusted by being dissolved in D-PBS (-).

The pitavastatin solution and the tannic acid solution were mixed to adjust a pitavastatin/TA solution. Then, PEG-P[Lys(FPBA)$_{10}$]$_{20}$ was added to the pitavastatin/TA solution to adjust a pitavastatin/TA/PEG-P[Lys(FPBA)$_{10}$]$_{20}$ (a pitavastatin ternary complex) solution. Table 3 shows the results of particle diameter measurement using Zetasizer.

The pitavastatin (measured value: 4586 nm) that had aggregated in the pitavastatin solution had a particle diameter of 60.2 nm in the pitavastatin/TA/PEG-P[Lys(FPBA)$_{10}$]$_{20}$ solution, confirming the formation of the pitavastatin ternary complex.

<6.8. Evaluation of Gelonin ternary complex formation>

[Final concentrations of Gelonin, TA, and PEG-P[Lys(FPBA)$_{10}$]$_{20}$]

- Gelonin: 0.5 μM
- Tannic acid: 82.5 μM
- PEG-P[Lys(FBPA)$_{10}$]$_{20}$: 50 μM

Each of these was adjusted by being dissolved in D-PBS (-).

The Gelonin solution and the tannic acid solution were mixed and centrifuged twice at 10,000 g × 5 minutes using an ultrafiltration membrane (MWCO: 3.5 kDa) to adjust a Gelonin/TA solution. Then, PEG-P[Lys(FPBA)$_{10}$]$_{20}$ was added to the Gelonin/TA solution to adjust a Gelonin/TA/PEG-P[Lys(FPBA)$_{10}$]$_{20}$ solution (a Gelonin ternary complex solution). Table 3 shows the results of particle diameter measurement using Zetasizer.

The particle diameter of the Gelonin ternary complex was clearly increased as compared with the particle diameter of Gelonin alone, confirming the formation of the Gelonin ternary complex.

<6.9. Evaluation of PE ternary complex formation>

[Final concentrations of PE, TA, and PEG-P[Lys(FPBA)$_{10}$]$_{20}$]

- PE: 0.25 μM
- Tannic acid: 82.5 μM
- PEG-P[Lys(FBPA)$_{10}$]$_{20}$: 50 μM

Each of these was adjusted by being dissolved in D-PBS (-).

The PE solution and the tannic acid solution were mixed and centrifuged twice at 10,000 g × 5 minutes using an ultrafiltration membrane (MWCO: 3.5 kDa) to adjust a PE/TA solution. Then, PEG-P[Lys(FPBA)$_{10}$]$_{20}$ was added to the PE/TA solution to adjust a PE/TA/PEG-P[Lys(FPBA)$_{10}$]$_{20}$ solution (a PE ternary complex solution). Table 3 shows the results of particle diameter measurement using Zetasizer.

The particle diameter of the PE ternary complex was clearly increased as compared with the particle diameter of PE alone, confirming the formation of the PE ternary complex.

<6.10. Evaluation of rGFP ternary complex formation>

Table 3 shows the results of particle diameter measurement carried out in the same manner as in the evaluation of complex formation in the section of 3.4.

<6.11. Evaluation of βGal ternary complex formation>

[Final concentrations of βGal, TA, and PEG-P[Lys(FPBA)$_{10}$]$_{20}$]

- βGal: 0.1 mg/mL
- Tannic acid: 0.37mg/mL
- PEG-P[Lys(FBPA)$_{10}$]$_{20}$: 3.95 mg/mL

Each of these was adjusted by being dissolved in D-PBS (-).
The βGal solution and the tannic acid solution were mixed to adjust a βGal/TA solution. Then, PEG-P[Lys(FPBA)$_{10}$]$_{20}$ was added to the βGal/TA solution to adjust a βGal/TA/IPEG-P[Lys(FPBA)$_{10}$]$_{20}$ solution (a βGal ternary complex solution). Table 3 shows the results of particle diameter measurement using Zetasizer.
The particle diameter of the βGal ternary complex was clearly increased as compared with the particle diameter of βGal alone, confirming the formation of the βGal ternary complex.

<6.12. Evaluation of Peptide ternary complex formation>

[Final concentrations of Peptide, TA, and PEG-P[Lys(FPBA)$_{10}$]$_{20}$]

- Peptide: 1 μM
- Tannic acid: 8 μM
- PEG-P[Lys(FBPA)$_{10}$]$_{20}$: 15 μM

Each of these was adjusted by being dissolved in D-PBS (-).
The Peptide solution and the tannic acid solution were mixed to adjust a Peptide/TA solution. Then, PEG-P[Lys(FPBA)$_{10}$]$_{20}$ was added to the Peptide/TA solution to adjust a Peptide/TA/PEG-P[Lys(FPBA)$_{10}$]$_{20}$ solution (a Peptide ternary complex) solution. Table 3 shows the results of particle diameter measurement by FCS using LSM710.
The particle diameter of the Peptide ternary complex was clearly increased as compared with the particle diameter of Peptide alone, confirming the formation of the Peptide ternary complex.

<6.13. Evaluation of AAV ternary complex formation>

[Final concentrations of AAV, TA, and PEG-P[Lys(FPBA)$_{10}$]$_{20}$]

- AAV: 2.0 × $10^{10}$vL/mL
- Tannic acid: 2.04 × $10^{-4}$ mg/mL
- PEG-P[Lys(FBPA)$_{10}$]$_{20}$: 0.0022 mg/mL

Each of these was adjusted by being dissolved in D-PBS (-).
The AAV solution and the tannic acid solution were mixed to adjust an AAV/TA solution. Then, PEG-P[Lys(FPBA)$_{10}$]$_{20}$ was added to the AAV/TA solution to adjust an AAV/TA/PEG-P[Lys(FPBA)$_{10}$]$_{20}$ solution (an AAV ternary complex solution). Table 3 shows the results of particle diameter measurement using Zetasizer.
The particle diameter of the AAV ternary complex was clearly increased as compared with the particle diameter of AAV alone, confirming the formation of the AAV ternary complex.

<6.14. Evaluation of AuNP ternary complex formation>

[Final concentrations of AuNP, TA, and PEG-P[Lys(FPBA)$_{10}$]$_{20}$]

- AuNP: 1.0 nM
- Tannic acid: 1 μM
- PEG-P[Lys(FBPA)$_{10}$]$_{20}$: 2 μM

Each of these was adjusted by being dissolved in pure water.
The AuNP solution and the tannic acid solution were mixed and centrifuged twice at 10,000 g × 5 minutes using an ultrafiltration membrane (MWCO: 10 kDa) to adjust an AuNP/TA solution. Then, PEG-P[Lys(FPBA)$_{10}$]$_{20}$ was added to the AuNP/TA solution to adjust an AuNP/TA/PEG-P[Lys(FPBA)$_{10}$]$_{20}$ solution (an AuNP ternary complex solution). Table 3 shows the results of particle diameter measurement using Zetasizer.
The particle diameter of the AuNP ternary complex was clearly increased as compared with the particle diameter of AuNP alone, confirming the formation of the AuNP ternary complex.

<6.15. Evaluation of TUG1 ternary complex formation>

[Final concentrations of TUG1, TA, and PEG-P[Lys(FPBA)$_m$]$_n$]

- TUG1: 100 nM
- Tannic acid: 5 μM
- PEG-P[Lys(FBPA)$_{10}$]$_{20}$: 10 μM

Each of these was adjusted by being dissolved in D-PBS (-).

The TUG1 solution and the tannic acid solution were mixed to prepare a TUG1/TA solution. Then, PEG-P[Lys(FPBA)$_{10}$]$_{20}$ was added to the TUG1/TA solution to adjust a TUG1/TA/PEG-P[Lys(FPBA)$_{10}$]$_{20}$ solution (a TUG1 ternary complex) solution. Table 3 shows the results of particle diameter measurement by FCS using LSM710.

The particle diameter of the TUG1 complex was clearly increased as compared with the particle diameter of TUG1 alone, confirming the formation of the TUG1 complex.

[Table 3]

| Type | Application | Encapsulated substance | Particle diameter of encapsulated substance (nm) | Particle diameter of complex (nm) | Polydiversity (PDI) |
|---|---|---|---|---|---|
| Small Drug | Anticancer agent | Bleomycin sulfate | 0.6 | 68.5* | 0.19 |
| | Acoustic sensitizer | Rose bengal | 0.7 | 73.2* | 0.14 |
| | Photosensitizer | Chlorin e 6 | 1.2 | 21.6** | - |
| | Therapeutic agent for dyslipidemia | Pitavastatin calcium | Aggregated | 60.2* | 0.29 |
| Protein | Cytoplasmic responsive toxin | Gelonin | 4.1 | 23.5* | 0.24 |
| | Intra-lysosomal responsive toxin | Pseudomonas exotoxin A | 5.6 | 26.8* | 0.19 |
| | Fluorescence protein | GFP | 3.9 | 17.9** | - |
| | Enzyme | β-D-galactosidase | 13.9 | 42.8* | 0.18 |
| Peptide | Membrane-permeable peptide | FITC-LC-Antennapedia Peptide | 2.0 | 41.5** | - |
| Virus | Therapeutic virus | AAV9 | 29.1 | 46.3* | 0.41 |
| Inorganic | Inorganic particle | Gold nanoparticle | 15.2 | 43.4* | 0.09 |
| RNA | Nucleic acid | Alexa Fluor647-TUG1 | 3.3 | 26.2** | - |

* Measurement by dynamic light scattering method
** Measurement by fluorescence correlation spectroscopy

7. Evaluation of functionality of rose bengal ternary complex

<7.1. Overview>

Blood retention was evaluated by the rose bengal ternary complex by an animal experiment.

<7.2. Reagent>

Regarding reagents which are not otherwise described, commercially available products were used as they were.

- Rose bengal: (973.67 g/mol) Tokyo Chemical Industry Co., Ltd.
- PEG-P[Lys(FBPA)$_{10}$]$_{20}$ (Mn = 14,000)
- Tannic acid: (Mw = 1,701) Wako Pure Chemical Industries Co., Ltd.
- D-PBS(-): Wako Pure Chemical Industries Co., Ltd.
- BALB/c mice: Charles River Japan Inc.
- Passive Lysis Buffer: Promega corporation.

<7.3. Measuring apparatus>

- Guava (registered trade name) easyCyte Flow Cytometry (FCM): Merck Millipore
- Spark: Tecan Group Ltd.

<7.4. Pharmacokinetics of rose bengal ternary complex>

[Final concentrations of rose bengal, TA, and PEG-P[Lys(FPBA)$_{10}$]$_{20}$]

- Rose bengal: 0.1 mg/mL
- Tannic acid: 1.0 mg/mL
- PEG-P[Lys(FBPA)$_{10}$]$_{20}$: 16.5 mg/mL

Each of these was adjusted by being dissolved in D-PBS (-).

The rose bengal solution and the tannic acid solution were mixed to adjust a rose bengal/TA solution. Then, PEG-P[Lys(FPBA)$_{10}$]$_{20}$ was added to the rose bengal/TA solution to adjust a rose bengal solution, a rose bengal/TA complex solution, and a rose bengal/TA/PEG-P[Lys(FPBA)$_{10}$]$_{20}$ (a rose bengal ternary complex) solution.

[Evaluation of pharmacokinetics]

200 μl of the prepared solution described above was intravenously administered to the tail vein of the model mouse. One to three hours after the sample administration, dissection was carried out to recover blood, which was subsequently subjected to centrifugation at 5,000 g × 10 minutes at 20°C to recover 100 μl of a plasma component, and 700 μl of Passive Lysis Buffer was added thereto. Then, the fluorescence intensities (Ex/Em: 520 nm/570 nm) of the plasma component and the administered sample were measured with Spark to evaluate the blood retention. The results are shown in Fig. 22. In the figure, rose bengal/TA/PEG-P[Lys(FPBA)$_{10}$]$_{20}$ is denoted by a rose bengal/TA/polymer.

The concentrations of the rose bengal and the rose bengal/TA complex in blood 3 hours after administration were each about 0.22% and 1.02%. On the other hand, the concentration of the rose bengal ternary complex in blood was 2.2% 3 hours after administration, which was about 10 times the concentration of rose bengal alone. From this result, it was shown that the rose bengal ternary complex achieved an improvement in blood retention as compared with rose bengal alone and rose bengal/TA.

8. Evaluation of functionality of GFP ternary complex

<8.1. Overview>

The functionality of the GFP ternary complex was evaluated. Specifically, it is an evaluation of the stability related to the oxidation of tannic acid in a solution and an evaluation of the responsiveness of adenosine triphosphate (ATP), which is an intracellular molecule of the GFP ternary complex.

<8.2. Reagent>

Regarding reagents and solvents which are not otherwise described, commercially available products were used as they were.

- Green fluorescent protein (rGFP Protein, Mw: 33k Da): Clontech Laboratories, Inc.
- PEG-P[Lys(FBPA)$_{10}$]$_{20}$ (Mn = 14,000)
- Tannic acid: (Mw = 1,701) Wako Pure Chemical Industries Co., Ltd.
- D-PBS(-): Wako Pure Chemical Industries Co., Ltd.
- Adenosine triphosphate: Wako Pure Chemical Industries Co., Ltd.

<8.3. Measuring apparatus>

- LSM710: Carl Zeiss Co., Ltd.
- Fluorophotometer FP-8300 : Jasco International Co., Ltd.
- Absorptiometer (V-650, JASCO, Tokyo, Japan)

<8.4. Evaluation of TA oxidation suppression by adding boronic acid-introduced polymer>

[Final concentrations of TA and PEG-P[Lys(FPBA)$_{10}$]$_{20}$]

- Tannic acid: 0.5 mg/mL
- PEG-P[Lys(FPBA)$_{10}$]$_{20}$: 2.2 mg/mL

Each of these was adjusted by being dissolved in D-PBS (-).

The tannic acid solution and the PEG-P[Lys(FPBA)$_{10}$]$_{20}$ solution were mixed to adjust a TA/PEG-P[Lys(FPBA)$_{10}$]$_{20}$ solution.

[Evaluation of TA oxidation suppression in solution]

After incubating the prepared TA solution and the TA/PEG-P[Lys(FPBA)$_{10}$]$_{20}$ solution at 37°C for a predetermined time, the absorbance increase at the absorption wavelength (380 nm) derived from quinone, which is an oxidized product of TA, was measured with an absorption spectrometer. The obtained results are shown in Fig. 23A, and a photographic image of the solutions after 24 hours is shown in Fig. 23B.

From the results shown in Fig. 23A and Fig. 23B, it is shown that in the TA/PEG-P[Lys(FPBA)$_{10}$]$_{20}$ solution, the chronological increase in absorbance and the change in color after 24 hours are significantly suppressed as compared with the TA solution. From the above, it can be seen that the addition of PEG-P[Lys(FPBA)$_{10}$]$_{20}$ significantly suppresses the oxidation of tannic acid.

<8.5. Evaluation of chronological stability of GFP ternary complex>

[Final concentrations of GFP, TA, and PEG-P[Lys(FPBA)$_{10}$]$_{20}$

- GFP: 0.5 μM

- Tannic acid: 82.5 μM
- FBPA of PEG-P[Lys(FPBA)$_{10}$]$_{20}$: 250 μM

Each of these was adjusted by being dissolved in D-PBS (-).
The GFP solution and the tannic acid solution were mixed and centrifuged twice at 10,000 g × 5 minutes using an ultrafiltration membrane (MWCO: 3.5 kDa) to adjust a GFP/TA solution. Then, the PEG-P[Lys(FPBA)$_{10}$]$_{20}$ solution was added to a GFP/TA solution to adjust a GFP/TA/PEG-P[Lys(FPBA)$_{10}$]$_{20}$ complex (a GFP ternary complex) solution.

[Evaluation of stability of GFP ternary complex]

The prepared GFP ternary complex was incubated at 37°C for a predetermined time, and then the particle diameter was measured by FCS using LSM710, and the obtained results are shown in Fig. 23C. In addition, the results of measuring the fluorescence intensity using FP-8300 are also shown in Fig. 23C.
No significant change in particle diameter and fluorescence intensity was observed even after 24 hours, and thus it can be seen that the GFP ternary complex stably forms the complex.

<8.6. Evaluation of stability of GFP ternary complex in ATP solution>

[Final concentrations of GFP, TA, and PEG-P[Lys(FPBA)$_{10}$]$_{20}$

- GFP: 0.5 μM
- Tannic acid: 82.5 μM
- FBPA of PEG-P[Lys(FPBA)$_{10}$]$_{20}$: 250 μM

Each of these was adjusted by being dissolved in D-PBS (-).
The GFP solution and the tannic acid solution were mixed and centrifuged twice at 10,000 g × 5 minutes using an ultrafiltration membrane (MWCO: 3.5 kDa) to adjust a GFP/TA solution. Then, the PEG-P[Lys(FPBA)$_{10}$]$_{20}$ solution was added to a GFP/TA solution to adjust a GFP/TA/PEG-P[Lys(FPBA)$_{10}$]$_{20}$ complex (a GFP ternary complex) solution.

[Evaluation of stability of GFP ternary complex in ATP solution]

The prepared GFP ternary complex was mixed with an ATP solution having a predetermined concentration, and then the particle diameter was measured by FCS using LSM710. The results are shown in Table 24.
A significant decrease in particle diameter was observed in association with the increase in ATP concentration, and thus it was confirmed that the GFP ternary complex has ATP responsiveness by which the bond between the tannic acid and PEG-P[Lys(FPBA)$_{10}$]$_{20}$ is dissociated in association with the increase in ATP concentration.

9. Evaluation of functionality of βGal ternary complex formation

<9.1. Overview>

The functionality of the βGal ternary complex was evaluated. Specifically, it is an evaluation of the enzymatic activity of the βGal ternary complex in the solution and in the cell and an evaluation of the pharmacokinetics by an animal experiment.

<9.2. Reagent>

Regarding reagents which are not otherwise described, commercially available products were used as they were.

- β-D-galactosidase (βGal): Mw 540 kDa, Wako Pure Chemical Industries Co., Ltd.
- PEG-P[Lys(FBPA)$_{10}$]$_{20}$ (Mn = 14,000)
- Tannic acid: (Mw = 1,701) Wako Pure Chemical Industries Co., Ltd.
- Alexa Fluor647-NHS: Mw = 1,250, Thermo Fisher Scientific Inc.
- D-PBS(-): Wako Pure Chemical Industries Co., Ltd.
- GlycoGREEN (registered trade name)-βGal: GORYO Chemical, Inc.

- Roswell Park Memorial Institute medium (RPMI): Sigma Aldrich Co., 11c.
- Fetal bovine serum (FBS): Biosera Inc.
- Trypsin-EDTA solution (Trp): Sigma life science Co., Ltd.
- Penicillin / streptomycin (PS): Sigma life science Co., Ltd.
- CT26 cell (mouse colon carcinoma cell line): American Type Culture Collection
- BALB/c mice: Charles River Japan Inc.
- Passive Lysis Buffer: Promega corporation.
- Dimethyl sulfoxide(DMSO): Wako Pure Chemical Industries Co., Ltd.

<9.3. Measuring apparatus>

- LSM710: Carl Zeiss Co., Ltd.
- Fluorophotometer FP-8300: Jasco International Co., Ltd.
- Absorptiometer V-650: Jasco International Co., Ltd.

<9.4. Evaluation of βGal ternary complex activity in solution>

[Final concentrations of βGal, TA, and PEG-P[Lys(FPBA)$_{10}$]$_{20}$]

- βGal: 0.1 mg/mL
- Tannic acid: 0.37mg/mL
- PEG-P[Lys(FBPA)$_{10}$]$_{20}$: 3.95 mg/mL

Each of these was adjusted by being dissolved in D-PBS (-).

The βGal solution and the tannic acid solution were mixed to adjust a βGal/TA solution. Then, PEG-P[Lys(FPBA)$_{10}$]$_{20}$ was added to the βGal/TA solution to adjust a βGal/TA/IPEG-P[Lys(FPBA)$_{10}$]$_{20}$ solution (a βGal ternary complex solution).

[Final concentration of GlycoGREEN-βGal]

- GlycoGREEN-βGal 1 mM

It was adjusted by being dissolved in DMSO.

[Evaluation of βGal ternary complex activity in solution]

1 μl of 1 mM GlycoGREEN-βGal solution was added to 20 μl of each of the prepared the βGal solution, the βGal/TA solution, and the βGal ternary complex solution, and the fluorescence (Ex/Em: 480/510 nm) detected in a case where GlycoGREEN-βGal enzymatically reacted with βGal was chronologically measured using FP-8300. The results obtained are shown in Fig. 25A. The maximum fluorescence intensity of each solution is shown in Fig. 25B. In the figure, βGal/TA/PEG-P[Lys(FPBA)$_{10}$]$_{20}$ is denoted by β-galactosidase/TA/polymer.

From the results shown in Fig. 25A and 25B, it was revealed that the enzymatic reaction of the βGal ternary complex is suppressed as compared with the enzymatic reaction of βGal alone, and the encapsulation of βGal in the complex reduces the apparent enzyme activity of galactosidase.

<9.5. Evaluation of intracellular βGal ternary complex activity>

[Introduction of Alexa647 to βGal complex]

- βGal: 10 mg

- Alexa Flour647-NHS: 0.12 mg

10 mg of βGal was weighed in a 20 mL vial bottle and dissolved in 10 ml of 50 mM $NaHCO_3$ (pH8.0). 0.12 mg of Alexa Flour647-NHS dissolved in DMSO was added thereto, and the mixture was stirred at room temperature for 4 hours. After performing ultrafiltration (MWCO: 10 kDa) twice with D-PBS (-) on the reaction solution, unreacted Alexa Flour 647-NHS was removed with a PD-10 column (solvent: D-PBS (-)), and then ultrafiltration (MWCO: 10 kDa) was performed twice again using D-PBS (-) to recover Alexa Flour647-modified βGal (Alexa647-βGal) in a solution state. Then, the βGal concentration was calculated from the absorbance at 280 nm, which is the absorption wavelength derived from a protein.

[Final concentrations of βGal, TA, and PEG-P[Lys(FPBA)$_{10}$]$_{20}$]

- βGal: 0.1 mg/mL
- Alexa647-βGal: 0.1 mg/mL
- Tannic acid: 0.37mg/mL
- PEG-P[Lys(FBPA)$_{10}$]$_{20}$: 3.95 mg/mL

Each of these was adjusted by being dissolved in D-PBS (-).
The βGal solution and the tannic acid solution were mixed to adjust a βGal/TA solution. Then, PEG-P[Lys(FPBA)$_{10}$]$_{20}$ was added to the βGal/TA solution to adjust each of a βGal solution, a βGal/TA solution, and a βGal/TA/PEG-P[Lys(FPBA)$_{10}$]$_{20}$ solution (a βGal ternary complex solution).
The same operation was performed using Alexa647-βGal instead of βGal to prepare each of an Alexa647-βGal solution, an Alexa647-βGal/TA complex solution, and an Alexa647-βGal/TApGal/TA/PEG-P[Lys(FPBA)$_{10}$]$_{20}$ solution (an Alexa647-βGal ternary complex solution).

[Final concentration of GlycoGREEN-βGal]

- GlycoGREEN-βGal 1 mM

It was adjusted by being dissolved in DMSO.

[Evaluation of amount of βGal ternary complex incorporated into cell]

FBS and PS each were mixed with RPMI to 10 wt% and 2 wt% to adjust a cell medium. CT26 cells were suspended in a cell medium to prepare a cell suspension of $1.25 \times 10^5$ cells/ml. 400 μl of this cell suspension was seeded on a 24-well plate ($5.0 \times 10_4$ cells per well) and incubated at 37°C for 24 hours. After removing the medium, the cells were washed once with D-PBS (-), and then 400 μl of each solution adjusted using Alexa647-βGal was added to the cells, and the cells were incubated at 37°C for 6 hours. After incubating for a predetermined time, the solution was removed, washing was carried out twice with D-PBS (-), 150 μl of Trp was added and incubated at 37°C for 7 minutes, and then 150 μl of D-PBS (-) + 10% FBS was added to measure the fluorescence intensity (Ex/Em: 642/664 nm) derived from Alexa647 with a flow cytometer (FCM) and to evaluate the cellular incorporation amount of each sample. The results obtained are shown in Fig. 26A.

[Evaluation of βGal ternary complex activity in cells]

CT26 cells were suspended in a cell medium to prepare a cell suspension of $1.25 \times 10^5$ cells/ml. 400 μ1 of this cell suspension was seeded on a 24-well plate ($5.0 \times 10_4$ cells per well) and incubated at 37°C for 24 hours. After removing the medium, washing was carried out once with D-PBS (-), and then 400 μl of each solution adjusted using βGal was added to the cells, and the cells were incubated at 37°C for 6 hours. After incubating for a predetermined time, the solution was removed, washing was carried out twice with D-PBS (-), and then 400 μl of GlycoGREEN-βGal prepared to 1 μM was added to the cells, and the cells were incubated for 30 minutes. Then, after removing the solution and washing twice with D-PBS (-), 150 μl of Trp was added and the cells were incubated at 37°C for 7 minutes, and then 150 μ1 of D-PBS (-) + 10% FBS was added to measure the fluorescence intensity (Ex/Em = 488 nm/525 nm) derived from the activity, which is detected in a case where GlycoGREEN-βGal is enzymatically reacted with βGal, with a flow

cytometer (FCM). The obtained results are shown in Fig. 26B. In addition, Fig. 26C shows the results of dividing the obtained fluorescence intensity derived from the activity by the fluorescence intensity corresponding to the intracellular incorporation amount.

From the results shown in Fig. 26C, it was revealed that the intracellular activity of the βGal ternary complex is equivalent to that of βGal alone.

<9.6. Pharmacokinetics of βGal ternary complex>

[Final concentrations of Alexa647-βGal, TA, and PEG-P[Lys(FPBA)$_{10}$]$_{20}$]

- Alexa647-βGal: 0.5 mg/mL
- Tannic acid: 1.85 mg/mL
- PEG-P[Lys(FBPA)$_{10}$]$_{20}$: 19.74 mg/mL

Each of these was adjusted by being dissolved in D-PBS (-).

The Alexa647-βGal solution and the tannic acid solution were mixed to adjust a Alexa647-βGal/TA solution. Then, PEG-P[Lys(FPBA)$_{10}$]$_{20}$ was added to the Alexa647-βGal/TA solution to adjust an Alexa647-βGal solution and an Alexa647-βGal/TA/PEG-P[Lys(FPBA)$_{10}$]$_{20}$ solution (an Alexa647-βGal ternary complex solution).

[Preparation of CT26 subcutaneous tumor model mouse]

100 μl of a CT26 cell suspension (1.0 × $10^6$ cells/ml) was subcutaneously injected into a Balb/c mouse.

[Evaluation of pharmacokinetics]

200 μl of the prepared solution described above was intravenously administered to the tail vein of a model mouse of which the tumor size reached about 200 mm$^3$. Six hours after the sample administration, dissection was carried out to recover blood and organs. The blood was subjected to centrifugation at 5,000 g × 10 minutes at 20°C to recover 100 μl of a plasma component, and 700 μl of Passive Lysis Buffer was added thereto. Each organ was weighed, Passive Lysis Buffer of 8 times the weight of the organ was added thereto and homogenized. Then, centrifugation was carried out at 10,000 g × 5 minutes, and the fluorescence intensity (Ex/Em: 640 nm/680 nm) of the supernatant solution was measured by TECAN to evaluate blood retention and pharmacokinetics. The results are shown in Fig. 27. In the figure, Alexa647-βGal is denoted by β-galactosidase, and Alexa647-βGal/TA/PEG-P[Lys(FPBA)$_{10}$]$_{20}$ is denoted by a β-galactosidase/TA/polymer.

The blood retention and the tumor accumulation of the Alexa647-βGal ternary complex were each improved by 4 times and 15 times as compared with Alexa647-βGal. On the other hand, the accumulation of the Alexa647-βGal ternary complex in the liver, kidney, and lung, which are normal tissues, was 1.4 times, 5.0 times, and 0.2 times, as compared with Alexa647-βGal, which was significantly suppressed as compared with the accumulation in the tumor.

10. Evaluation of functionality of AAV ternary complex formation

<10.1. Overview>

The functionality of the AAV ternary complex was evaluated. Specifically, the gene expression efficiency of the AAV ternary complex was evaluated by the cell experiment and an animal experiment.

<10.2. Reagent>

Regarding reagents which are not otherwise described, commercially available products were used as they were.

- AAV9-CMV-Luc (AAV): SignaGen Laboratories.
- PEG-P[Lys(FBPA)$_{10}$]$_{20}$ (Mn = 14,000)
- Tannic acid: (Mw = 1,701) Wako Pure Chemical Industries Co., Ltd.
- D-PBS(-): Wako Pure Chemical Industries Co., Ltd.
- PBS (+): Wako Pure Chemical Industries Co., Ltd.

- Roswell Park Memorial Institute medium (RPMI): Sigma Aldrich Co., llc.
- Fetal bovine serum (FBS): Biosera Inc.
- Trypsin-EDTA solution (Trp): Sigma life science Co., Ltd.
- Penicillin / streptomycin (PS): Sigma life science Co., Ltd.
- CT26 cell (mouse colon carcinoma cell line): American Type Culture Collection
- BALB/c mice: Charles River Japan Inc.
- Passive Lysis Buffer: Promega corporation.

    - Luciferase Assay System (Luciferin solution): Promega corporation.

- Anti-AAV-9, Mouse-Mono: PROGEN

<10.3. Measuring apparatus>

- GloMax Multi Detection System: Promega corporation.
- Fuji DRI-CHEM NX500: Fuji Film

< 10.4. Evaluation of gene expression efficiency of AAV ternary complex by cell experiment>

[Final concentrations of AAV, TA, and PEG-P[Lys(FPBA)$_{10}$]$_{20}$]

- AAV9-CMV-Luc (simply abbreviated as AAV): 2.0 $\times$ $10^{10}$ vL/mL
- Tannic acid: 2.0 $\times$ $10^{-4}$ mg/mL
- PEG-P[Lys(FBPA)$_{10}$]$_{20}$: 2.2 $\times$ $10^{-3}$ mg/mL

Each of these was adjusted by being dissolved in D-PBS (-).

The AAV solution and the tannic acid solution were mixed to adjust an AAV/TA solution. Then, PEG-P[Lys(FPBA)$_{10}$]$_{20}$ was added to the AAV/TA solution to adjust an AAV solution, an AAV/TA solution, and an AAV/TA/PEG-P[Lys(FPBA)$_{10}$]$_{20}$ solution (an AAV ternary complex solution).

[Evaluation of amount of AAV ternary complex incorporated into cell]

FBS and PS each were mixed with RPMI to 10 wt% and 2 wt% to adjust a cell medium. CT26 cells were suspended in cell medium to prepare a cell suspension of 2.0 $\times$ $10^5$ cells/ml. 25 $\mu$l of this cell suspension was seeded on a 96-well plate (5.0 $\times$ $10^3$ cells per well), 25 $\mu$l of each adjusted solution was added thereto, and the cells were incubated at 37°C for 72 hours. After incubating for a predetermined time, the solution was removed, washing was carried out once with D-PBS (+), 50 $\mu$l of Passive Lysis Buffer was added and the cells were incubated at 37°C for 15 minutes, and then 20 $\mu$l of each cell suspension was transferred to a white plate 96F (MS-8096W, Sumitomo Bakelite Co., Ltd.) for luminescence measurement, 100 $\mu$l of a Luciferin solution was added thereto using the GloMax Multi Detection System, and the luminescence intensity was measured. The obtained results are shown in Fig. 28. In the figure, AAV/TA/PEG-P[Lys(FPBA)$_{10}$]$_{20}$ is denoted by an AAV/TA/polymer.

From the results shown in Fig. 28, it was revealed that the Luc gene expression efficiency in a case where the AAV ternary complex is used is remarkably improved as compared with the case where AAV alone is used.

< 10.5. Evaluation of gene expression efficiency of AAV ternary complex by an animal experiment>

[Final concentrations of AAV, TA, and PEG-P[Lys(FPBA)$_{10}$]$_{20}$]

- AAV9-CMV-Luc (simply abbreviated as AAV): 2.0 $\times$ $10^{12}$ vL/mL
- Tannic acid: 2.0 $\times$ $10^{-2}$ mg/mL
- PEG-P[Lys(FBPA)$_{10}$]$_{20}$: 2.2 $\times$ $10^{-1}$ mg/mL

Each of these was adjusted by being dissolved in D-PBS (-).

The AAV solution and the tannic acid solution were mixed to adjust an AAV/TA solution. Then, PEG-P[Lys(FP-BA)$_{10}$]$_{20}$ was added to the AAV/TA solution to adjust an AAV solution, an AAV/TA solution, and an AAV/TA/PEG-P[Lys(FPBA)$_{10}$]$_{20}$ solution (an AAV ternary complex solution).

[Preparation of CT26 subcutaneous tumor model mouse]

100 μl of a CT26 cell suspension ($1.0 \times 10^6$ cells/ml) was subcutaneously injected into a Balb/c mouse.

[Evaluation of pharmacokinetics]

100 μl of the prepared solution described above was intravenously administered to the tail vein of a model mouse of which the tumor size reached about 200 mm$^3$. Two weeks after the sample administration, dissection was carried out to recover blood and organs. Each organ was weighed, Passive Lysis Buffer of 1 to 3 times the weight of organ was added thereto and homogenized. Then, 100 μl of the Luciferin solution was added to 20 μl of the homogenized suspension, and the gene expression level of each organ was measured using the GloMax Multi Detection System. Regarding the Luc gene expression level of each organ, Fig. 29 shows the results of the gene expression ratio where the Luc gene expression level of AAV alone in each organ is set to 1. In the figure, AAV/TA/PEG-P[Lys(FPBA)$_{10}$]$_{20}$ is denoted by an AAV/TA/polymer.

From the results of the gene expression ratio in each organ shown in Fig. 29, it was seen that the gene expression ratios of the AAV ternary complex in normal tissues such as liver, kidney, and heart were each 0.80 times, 0.02 times, and 0.27 times, as compared with AAV alone, which were significantly suppressed. On the other hand, the gene expression ratio of the AAV ternary complex in the tumor was improved 6.16 times as compared with AAV alone.

The separately collected blood was centrifuged at 5,000 g × 10 minutes at 20°C, a plasma component was recovered, and ALT and AST were measured using Fuji DRI-CHEM NX500 to evaluate liver toxicity. The obtained results are shown in Fig. 30A and Fig. 30B.

From the results shown in Fig. 30A and Fig. 30B, no liver toxicity was observed in the AAV ternary complex although liver toxicity was observed in the AAV/TA complex due to the increase in ALT and AST.

< 10.6. Evaluation of suppression of gene expression efficiency in case of using AAV9 antibody of AAV ternary complex by cell experiment>

[Final concentrations of AAV, TA, and PEG-P[Lys(FPBA)$_{10}$]$_{20}$]

- AAV9-CMV-Luc (simply abbreviated as AAV): $2.0 \times 10^{10}$ vL/mL
- Tannic acid: $2.0 \times 10^{-4}$ mg/mL
- PEG-P[Lys(FBPA)$_{10}$]$_{20}$: $2.2 \times 10^{-3}$ mg/mL
- Anti-AAV-9, Mouse-Mono (simply abbreviated as AAV antibody): diluted $10^5$ or $10^7$ times

Each of these was adjusted by being dissolved in D-PBS (-).

An AAV/TA solution was adjusted by mixing the AAV solution and the tannic acid solution. Then, PEG-P[Lys(FP-BA)$_{10}$]$_{20}$ was added to adjust an AAV solution, an AAV/TA solution, and an AAV/TA/PEG-P[Lys(FPBA)$_{10}$]$_{20}$ solution (an AAV ternary complex solution).

[Evaluation of amount of AAV ternary complex incorporated into cell]

CT26 cells were suspended in RPMI to prepare a cell suspension of $2.0 \times 10^5$ cells/ml. 25 μl of this cell suspension was seeded on a 96-well plate ($5.0 \times 10^3$ cells per well), 25 μl of each adjusted AAV solution and 1 μl of the AAV antibody solution were added thereto, and the cells were incubated at 37°C for 48 hours. After incubating for a predetermined time, the solution was removed, washing was carried out once with D-PBS (+), 50 μl of Passive Lysis Buffer was added and the cells were incubated at 37°C for 15 minutes, and then 20 μl of each cell suspension was transferred to a white plate 96F (MS-8096W, Sumitomo Bakelite Co., Ltd.) for luminescence measurement, 100 μl of a Luciferin solution was added thereto using the GloMax Multi Detection System, and the luminescence intensity was measured. The obtained results are shown in Fig. 31. At that time, the calculation was carried out by setting the luminescence intensity in a case where only each AAV solution sample was incubated with CT26 cells without adding the AAV antibody to 100%. In the figure, AAV/TA/PEG-P[Lys(FPBA)$_{10}$]$_{20}$ is denoted by an AAV/TA/polymer.

From the results shown in Fig. 31, it was revealed that the gene expression efficiency of AAV alone and the AAV/TA complex decreases by adding the AAV9 antibody as compared with the case where the AAV9 antibody is not added, but the gene expression efficiency of the AAV ternary complex does not decrease even in a case where the AAV9 antibody is added.

11. Evaluation of pharmacokinetics of TUG1 ternary complex

<11.1. Overview>

The functionality of the TUG1 ternary complex was evaluated. Specifically, the blood retention of the TUG1 ternary complex was evaluated by an animal experiment.

<11.2. Reagent and cell line>

Regarding reagents which are not otherwise described, commercially available products were used as they were.

- Alexa647-TUG1 (simply abbreviated as TUG1): 8.058.7 g/mol, GeneDesign, Inc.
- PEG-P[Lys(FBPA)$_{10}$]$_{20}$ (Mn = 14,000)
- Tannic acid: (Mw = 1,701) Wako Pure Chemical Industries Co., Ltd.
- D-PBS(-): Wako Pure Chemical Industries Co., Ltd.
- BALB/c mice: Charles River Japan Inc.
- Passive Lysis Buffer: Promega corporation.

<11.3. Measuring apparatus>

- Nikon A1R: Nikon Corporation
- ECLIPSE FN1: Nikon Corporation
- Spark: Tecan Group Ltd.

Nikon AIR and ECLIPSE FN1 were combined and used as an in vivo confocal laser scanning microscope.

<11.4. Measurement of blood retention of TUG1 by in vivo confocal laser scanning microscope>

[Final concentrations of TUG1, TA, and PEG-P[Lys(FPBA)$_{10}$]$_{20}$]

- TUG1: 6.25 μM
- Tannic acid: 312.5 μM
- PEG-P[Lys(FBPA)$_{10}$]$_{20}$: 625 μM

Each of these was adjusted by being dissolved in D-PBS (-).

The TUG1 solution and the tannic acid solution were mixed to prepare a TUG1/TA solution. Then, PEG-P[Lys(FPBA)$_{10}$]$_{20}$ was added to the TUG1/TA solution to adjust a TUG1/TA/PEG-P[Lys(FPBA)$_{10}$]$_{20}$ solution (a TUG1 ternary complex) solution.

[Measurement of blood retention of TUG1 by in vivo confocal laser scanning microscope]

200 μl of the prepared solution described above was intravenously administered to the tail vein of the model mouse. Then, the blood retention of TUG1 was measured for a predetermined time using an in vivo confocal laser scanning microscope. The obtained results are shown in Fig. 32 and Table 4. In the figure, TUG1/TA/PEG-P[Lys(FP-BA)$_{10}$]$_{20}$ is denoted by a TUG1/TA/polymer.

[Table 4]

| Samples | 10% time (min) |
|---|---|
| TUG1 | 28.3 |
| TUG1/TA | 49.6 |
| TUG1/TA/polymer | 171.4 |

The obtained results showed that the blood retention of the TUG1 ternary complex is dramatically extended as compared with TUG1 and TUG1/TA.

<11.5. Measurement of blood retention of TUG1 by blood sampling>

[Final concentrations of TUG1, TA, and PEG-P[Lys(FPBA)$_{10}$]$_{20}$]

- TUG1: 6.25 nM
- Tannic acid: 312.5 μM
- PEG-P[Lys(FBPA)$_{10}$]$_{20}$: 625 μM

Each of these was adjusted by being dissolved in D-PBS (-).

The TUG1 solution and the tannic acid solution were mixed to prepare a TUG1/TA solution. Then, PEG-P[Lys(FPBA)$_{10}$]$_{20}$ was added to the TUG1/TA solution to adjust a TUG1/TA/PEG-P[Lys(FPBA)$_{10}$]$_{20}$ solution (a TUG1 ternary complex) solution.

[Measurement of blood retention of TUG1 by blood sampling]

200 μl of the prepared solution described above was intravenously administered to the tail vein of the model mouse. Three hours after the sample administration, dissection was carried out to recover blood. The blood was subjected to centrifugation at 5,000 g × 10 minutes at 20°C to recover 100 μl of a plasma component, and 700 μl of Passive Lysis Buffer were added thereto. Then, the fluorescence intensities (Ex/Em: 640 nm/680 nm) of the solution and the sample were measured with Spark to evaluate the blood retention of TUG1. The results are shown in Table 5.

[Table 5]

| | Blood retention | % ID/g tissue |
|---|---|---|
| TUG1 | 1 | 0.11 |
| TUG1/TA | 1.65 | 0.18 |
| TUG1/TA/polymer | 40.7 | 4.41 |

The obtained results showed that the blood retention of the TUG1 ternary complex is about 40 times higher as compared with TUG1 and TUG1/TA and the blood retention of the TUG1 ternary complex is dramatically extended.

12. Summary

In the present Examples, in order to improve blood retention and stability in blood of a physiologically active protein, a ternary protein delivery system in which a boronic acid-introduced polymer is further added to a complex formed from a protein and tannic acid was constructed. GFP/TA/PEG-P[Lys(FPBA)$_{10}$]$_{20}$ using GFP as a model protein showed pH responsiveness and ATP responsiveness. It was confirmed that GFP/TA/PEG-P[Lys(FPBA)$_{10}$]$_{20}$ forms a stable complex in the blood environment (pH: about 7.4). In addition, it was also confirmed that PEG-P[Lys(FPBA)$_{10}$]$_{20}$ bonds to tannic acid with a high bonding force as compared with PEG-FPBA. Further, as a result of measuring the intracellular distribution measured, lysosomes and GFP were co-localized, suggesting that GFP was incorporated by endocytosis. It was shown that in the protein delivery system composed of GFP/TA/PEG-P[Lys(FPBA)$_{10}$]$_{20}$, tumor accumulation and retention are improved in addition to the improvement of blood retention as compared with GFP alone and GFP/TA.

It was also confirmed that the above protein delivery system can encapsulate molecules other than proteins and can form a ternary complex, as shown in Table 3. In addition, it was shown that the in vivo pharmacokinetics of the encapsulated substance can be improved by using this delivery system. In TUG1 (nucleic acid) encapsulated in the ternary complex, remarkable blood retention was observed as compared with TUG1 alone and TUG1/TA. In rose bengal (a small molecule medicine), remarkable blood retention was observed as compared with rose bengal alone and rose bengal/TA.
When the intracellular activity of βGal (protein) encapsulated in the ternary complex was evaluated, it exhibited an activity equivalent to the activity of βGal alone. In addition, when the gene expression efficiency of cells was measured using AAV (a viral vector) encapsulated in the ternary complex, it was confirmed that the ternary complex improves the expression efficiency of the introduced gene.
Each of the configurations and the combination thereof in each embodiment are examples, and additions, omissions, substitutions, and other modifications can be made without departing from the spirit or scope of the present invention. In addition, the present invention is not to be considered as being limited by each Example and is only limited by the claims (CLAIMS).

[Reference Signs List]

1: Complex
2: Polymer having boronic acid group
3: Compound having diol structure
40: Substance that is complexed with conjugate (a composite element)
4: Protein
10: Conjugate

**Claims**

**1.** A complex comprising:

a conjugate in which a polymer having a boronic acid group is bonded to a compound having a diol structure; and
a substance that is complexed with the conjugate.

**2.** The complex according to Claim 1,
wherein the substance that is complexed with the conjugate is at least one selected from the group consisting of a protein, a virus, an inorganic particle, a nucleic acid, and a small molecule medicine.

**3.** The complex according to Claim 1 or 2,
wherein the complex comprises the conjugate in which a polymer having a boronic acid group is bonded to a compound having a diol structure, and a protein.

**4.** The complex according to any one of Claims 1 to 3,
wherein the compound having a diol structure is a polyphenol.

**5.** The complex according to any one of Claims 1 to 4,
wherein the compound having a diol structure is at least one selected from the group consisting of tannic acid, gallic acid, and derivatives thereof.

**6.** The complex according to any one of Claims 1 to 5,
wherein the polymer has two or more boronic acid groups.

**7.** The complex according to any one of Claims 1 to 6,
wherein the boronic acid group is a phenylboronic acid group which may have a substituent or a pyridylboronic acid group which may have a substituent.

**8.** The complex according to any one of Claims 1 to 7,
wherein the boronic acid group is a phenylboronic acid group represented by General Formula (I) or a pyridylboronic

acid group represented by General Formula (II):

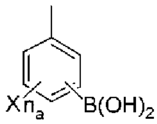

(I)

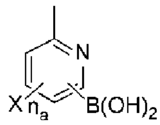

(II)

(in the formulae, X represents a halogen atom or a nitro group, and na is an integer of 0 to 4).

**9.** The complex according to any one of Claims 1 to 8,
wherein the polymer is at least one biocompatible polymer selected from the group consisting of a polyethylene glycol, an acrylic resin, a polyamino acid, a polyvinylamine, a polyallylamine, a polynucleotide, a polyacrylamide, a polyether, a polyester, a polyurethane, a polysaccharide, and copolymers thereof.

**10.** The complex according to any one of Claims 1 to 9,
wherein the polymer having a boronic acid group contains a first biocompatible polymer chain and a second biocompatible polymer chain that is different from the first biocompatible polymer chain.

**11.** The complex according to Claim 10,
wherein the second biocompatible polymer chain is a polyamino acid, and the boronic acid group is introduced into a side chain of the polyamino acid.

**12.** The complex according to Claim 10 or 11,
wherein the first biocompatible polymer chain is a polyethylene glycol.

**13.** The complex according to any one of Claims 10 to 12,
wherein the polymer having a boronic acid group contains a structure represented by General Formula (1) or (1-1),

$$A{-}L{-}B \quad (1) \qquad A{-}B \quad (1-1)$$

(in Formulae (1) and (1-1), A represents the first biocompatible polymer chain; L represents a linker part; and B represents the second biocompatible polymer chain having a boronic acid group and includes a repeating structure represented by the following (b2), or a repeating structure represented by (b1) and the repeating structure represented by (b2)),

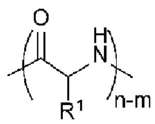

(b1)

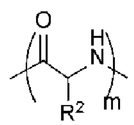

(b2)

(in Formulae (b1) and (b2), $R^1$ represents an amino acid side chain, $R^2$ is a structure in which the boronic acid group is introduced into an amino acid side chain, and n represents the total number of (b1) and (b2), n is an integer of 1 to 1,000, m is an integer of 1 to 1,000 (here, $m \leq n$), in a case where n - m is 2 or more, a plurality of $R^1$'s may be the same or different from each other, and in a case where m is 2 or more, a plurality of $R^2$'s may be the same or different from each other).

**14.** The complex according to any one of Claims 1 to 13,
wherein an average particle diameter of the complex determined by dynamic light scattering (DLS) or fluorescence correlation spectroscopy (FCS) is 5 nm or more and 200 nm or less.

**15.** The complex according to any one of Claims 1 to 14,
wherein a number average molecular weight of the polymer having a boronic acid group is 2,000 to 200,000.

**16.** A medicine containing:
the complex according to any one of Claims 1 to 15 as an active ingredient.

**17.** A therapeutic agent for cancer, comprising:
the complex according to any one of Claims 1 to 16 as an active ingredient.

**18.** A kit comprising:

a polymer having a boronic acid group; and
a compound having a diol structure.

**19.** A conjugate in which a polymer having a boronic acid group is bonded to a compound having a diol structure.

FIG. 1

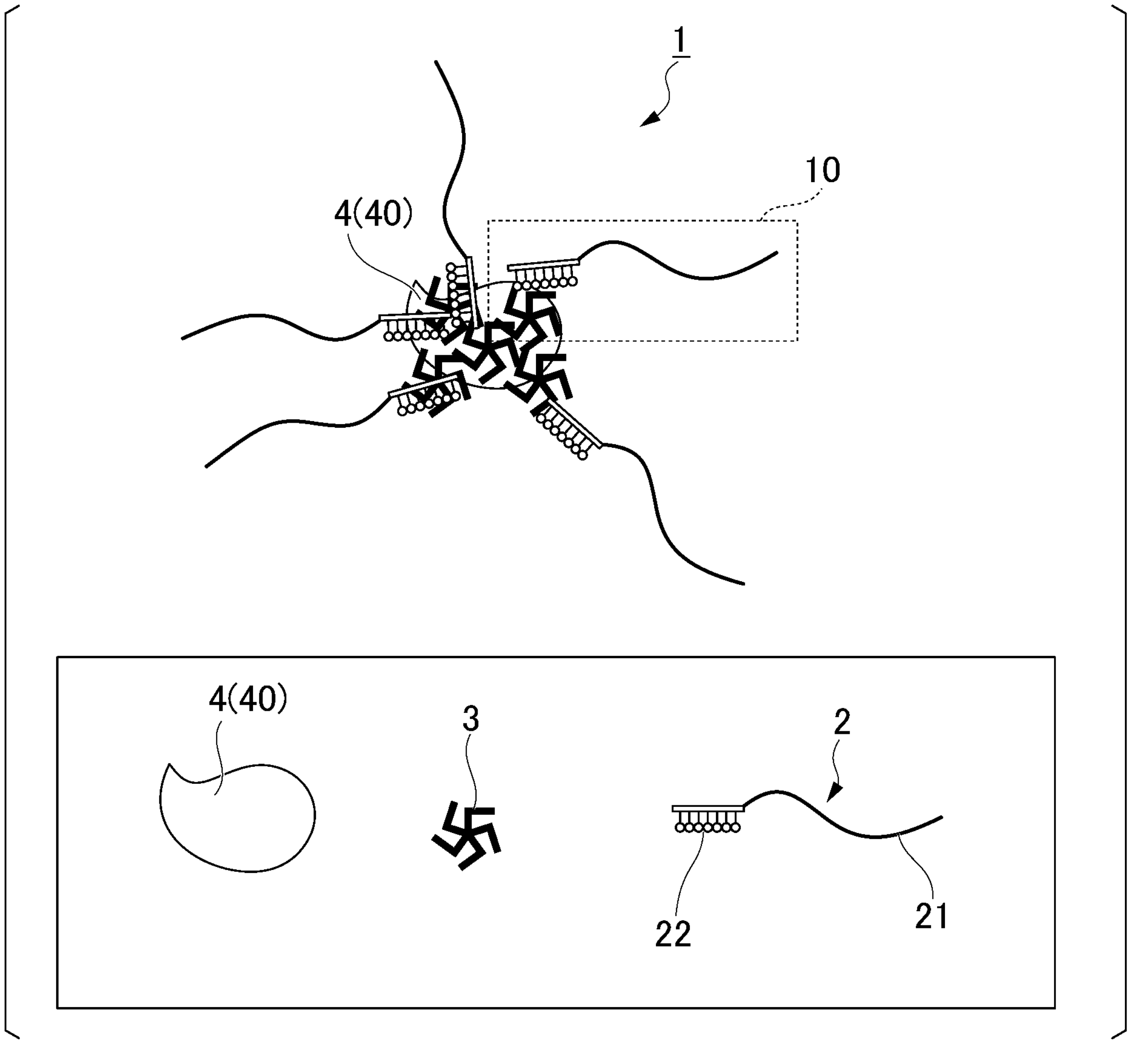


FIG. 2

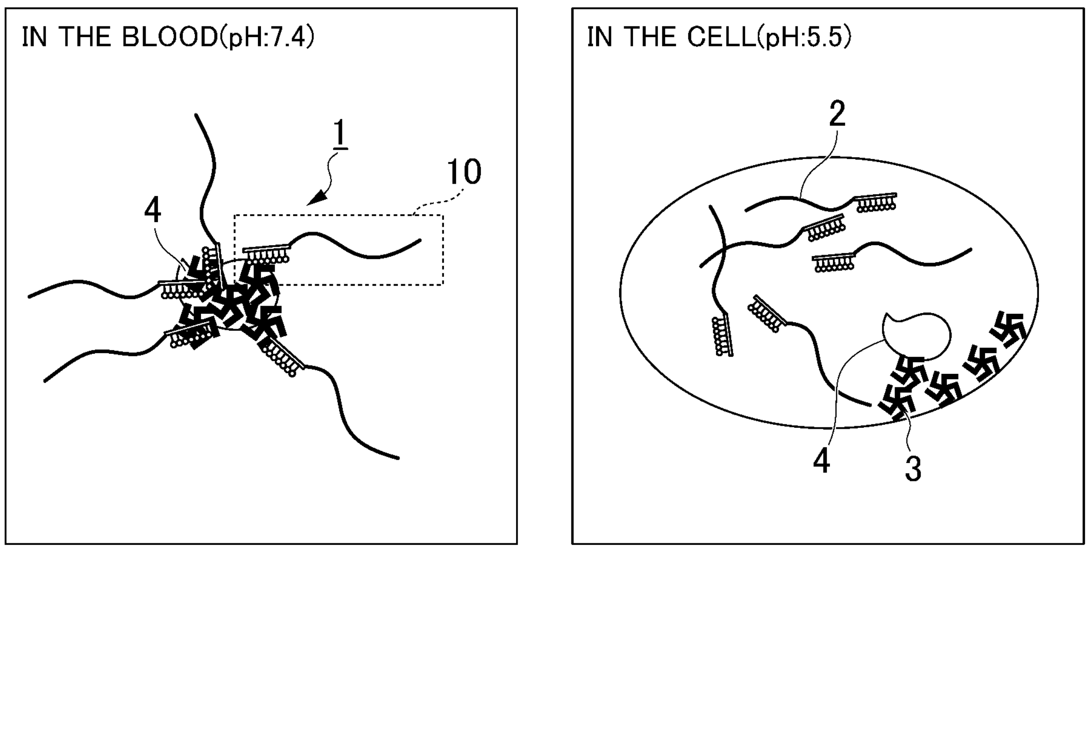

FIG. 3

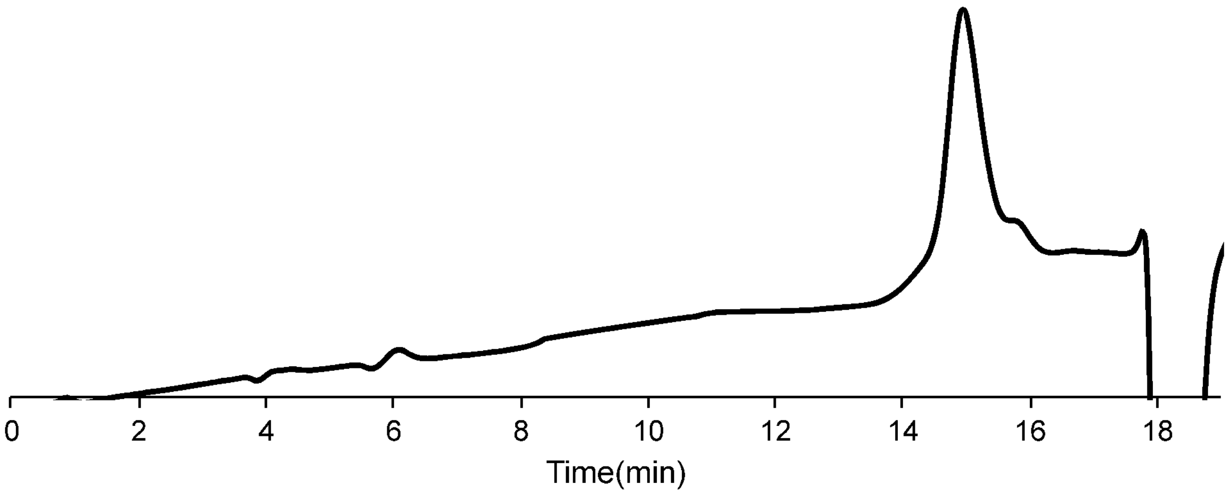


FIG. 4

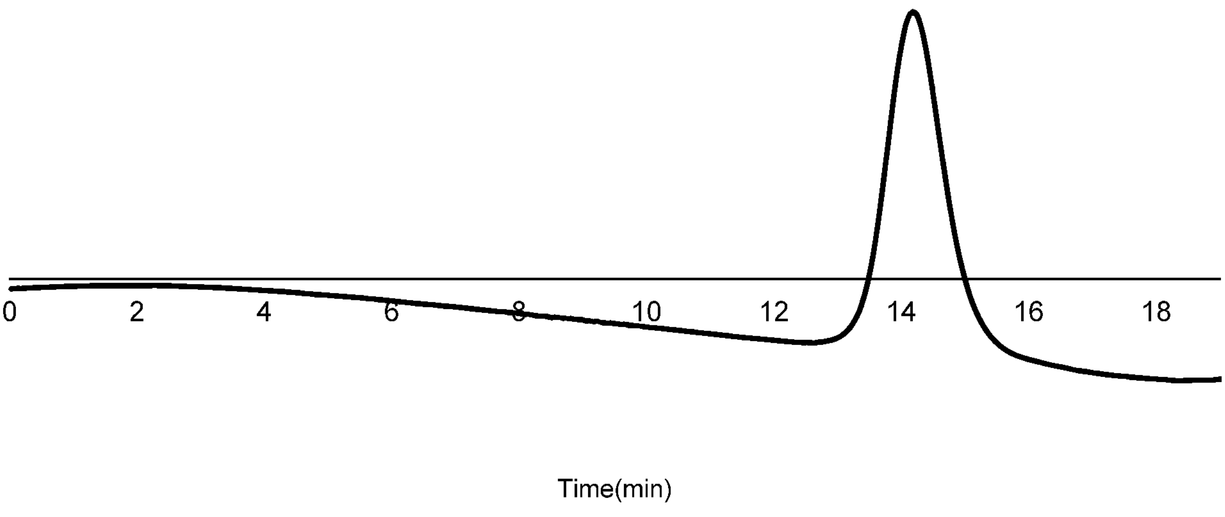

FIG. 5

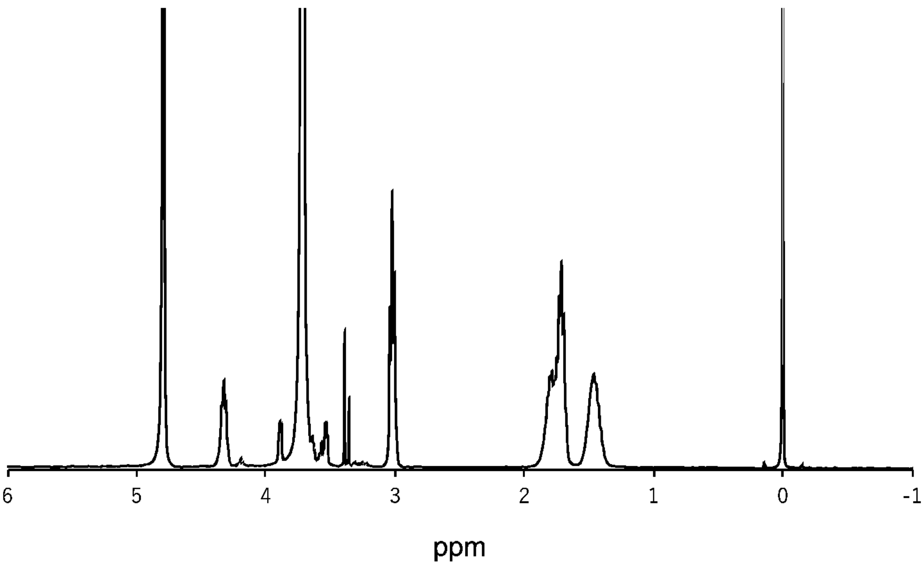


FIG. 6

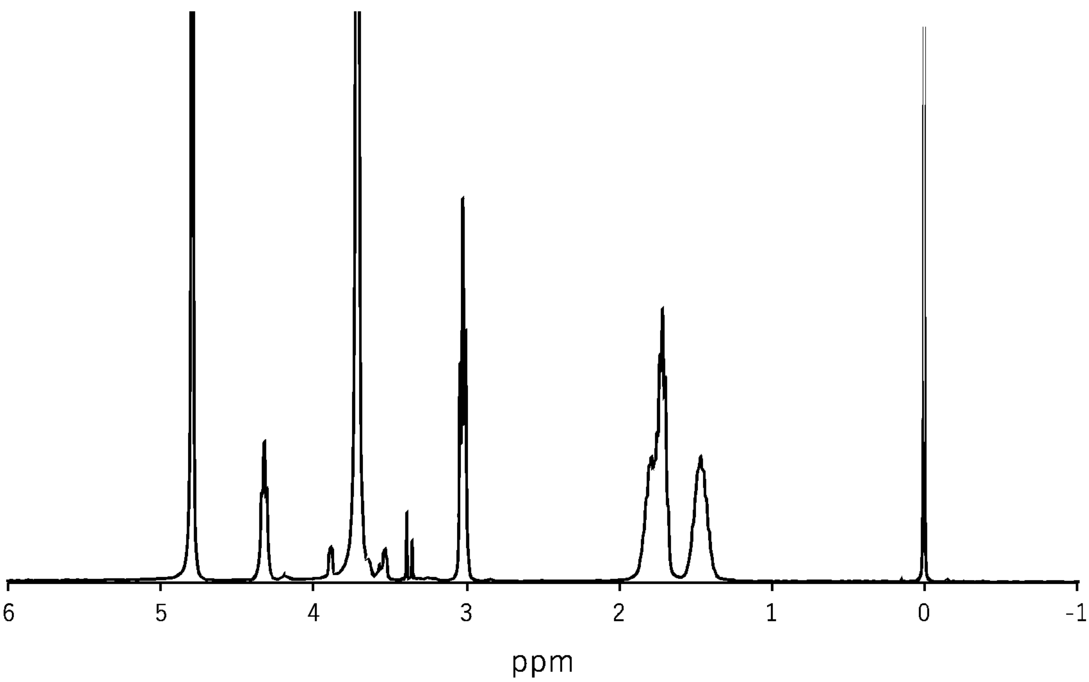

FIG. 7

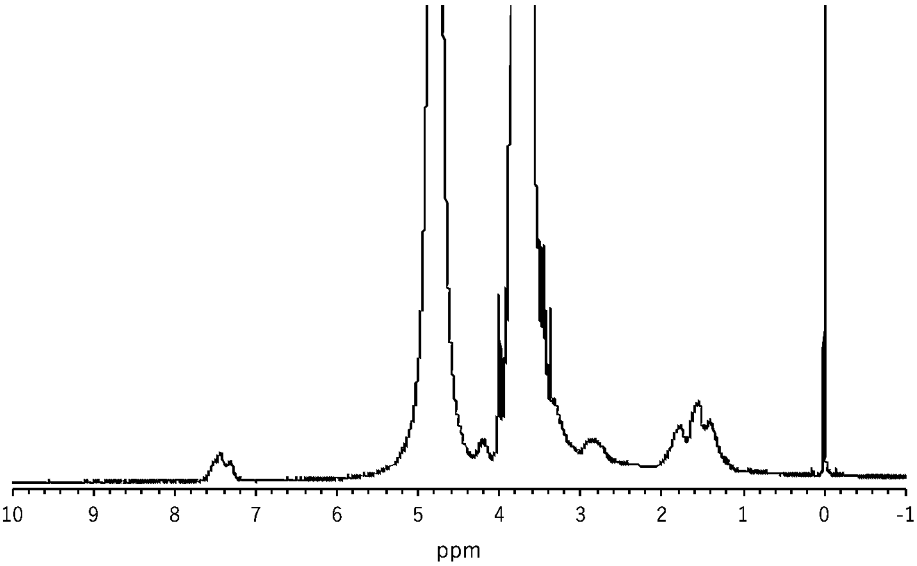


FIG. 8

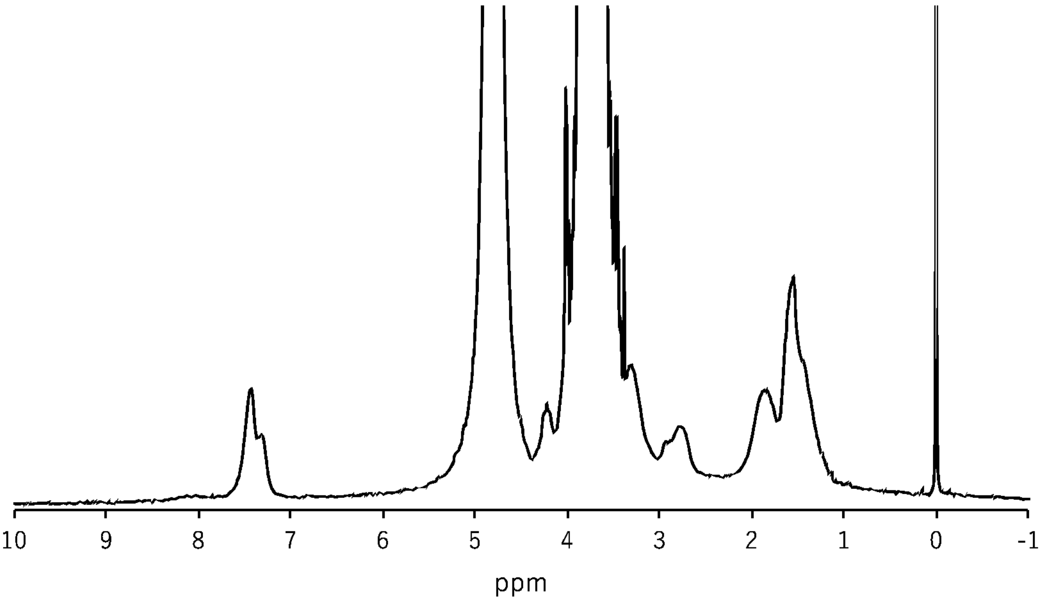

FIG. 9

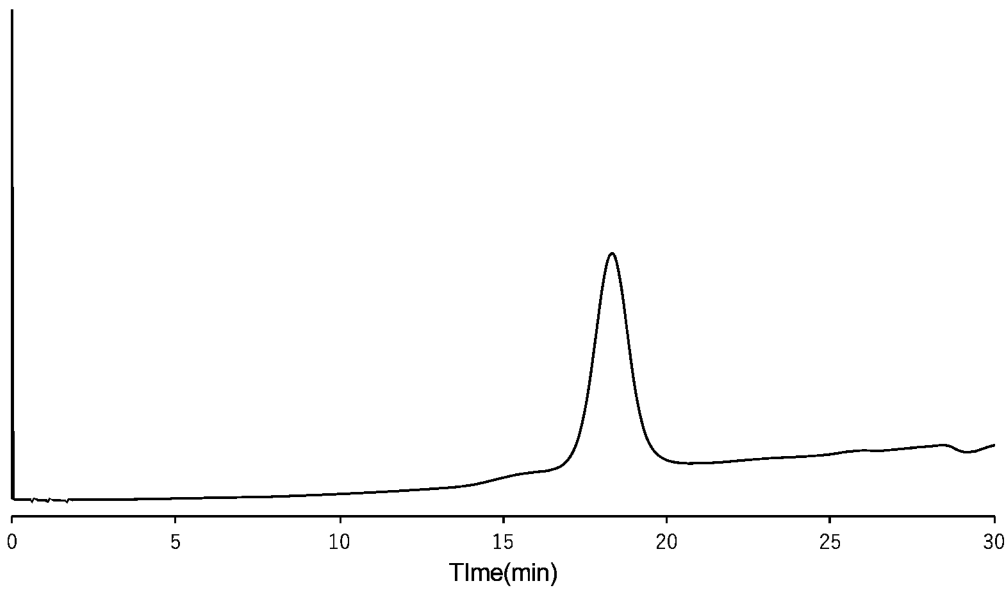


FIG. 10

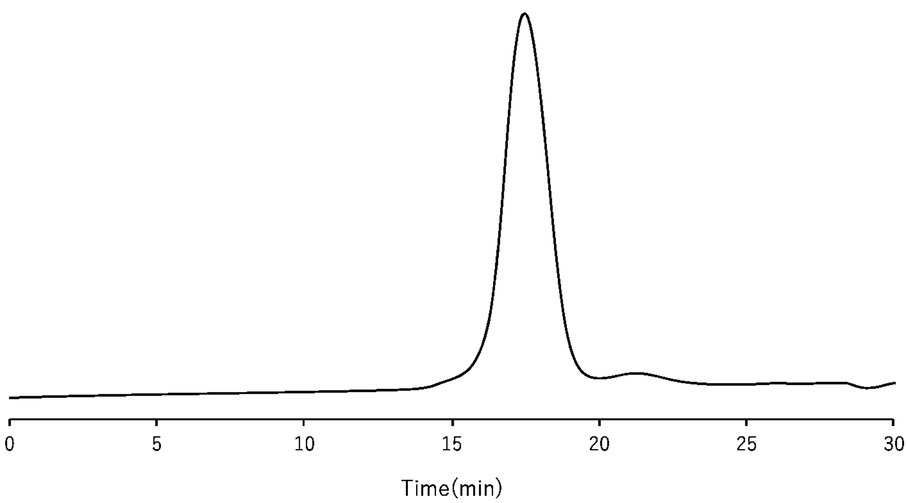

FIG. 11

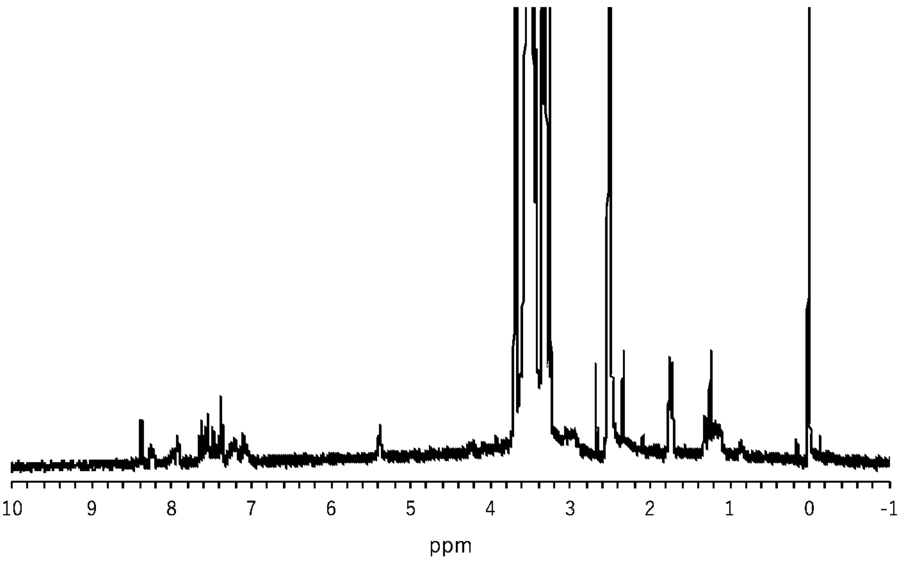


FIG. 12

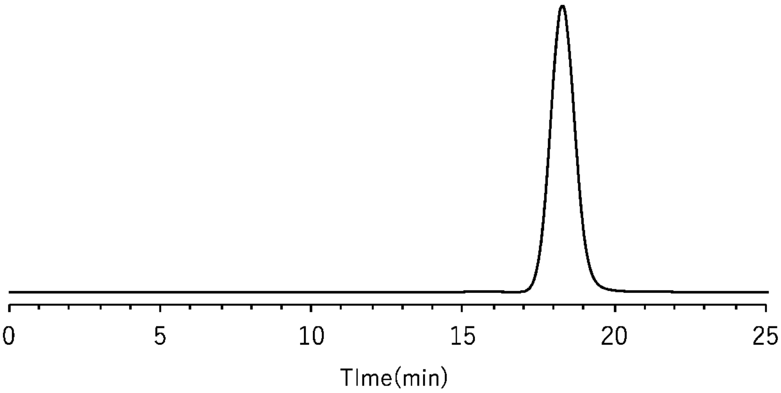

# FIG. 13

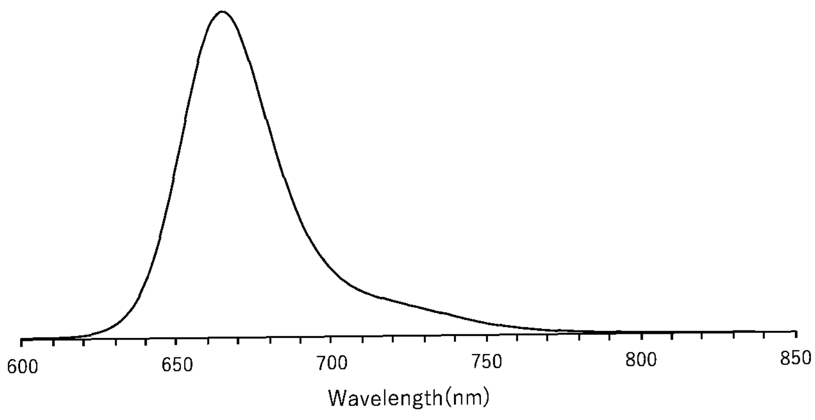
600
650
700
750
800
850
Wavelength(nm)

# FIG. 14

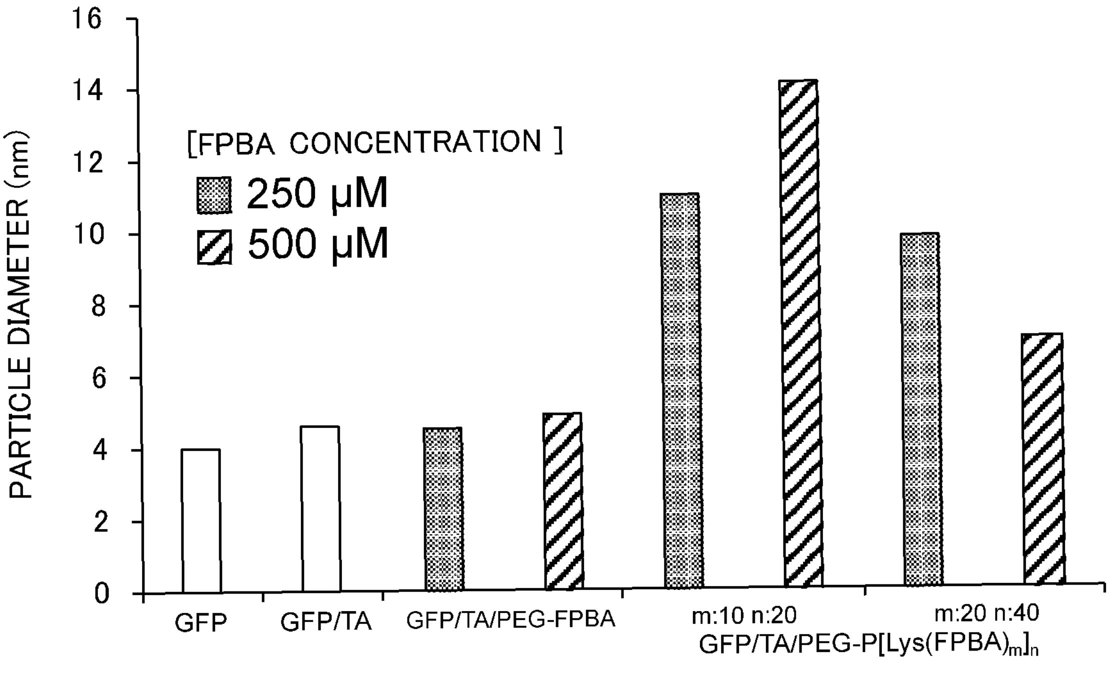
PARTICLE DIAMETER (nm)
0
2
4
6
8
10
12
14
16
[FPBA CONCENTRATION ]
250 μM
500 μM
GFP
GFP/TA
GFP/TA/PEG-FPBA
m:10 n:20
m:20 n:40
GFP/TA/PEG-P[Lys(FPBA)m]n

FIG. 15

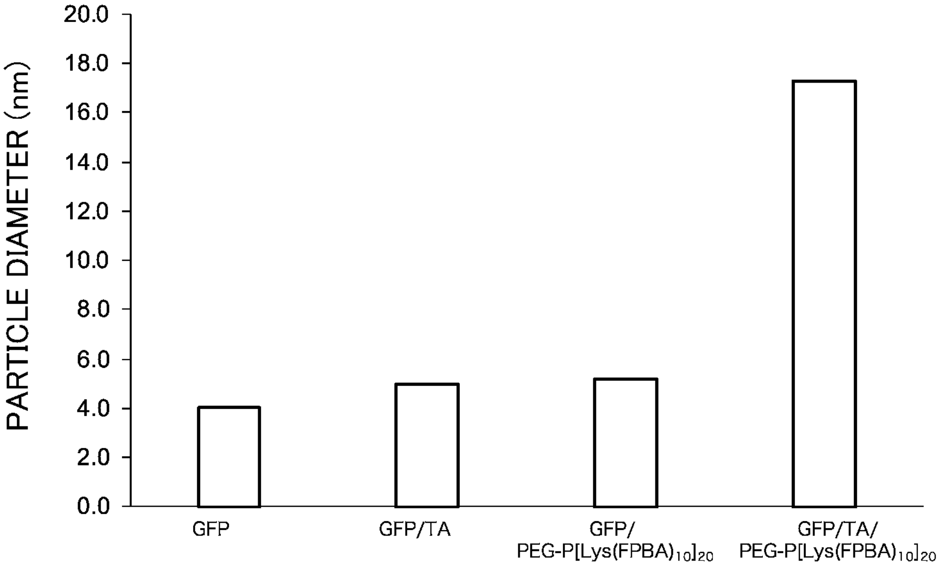
PARTICLE DIAMETER (nm)
20.0
18.0
16.0
14.0
12.0
10.0
8.0
6.0
4.0
2.0
0.0
GFP
GFP/TA
GFP/ PEG-P[Lys(FPBA)10]20
GFP/TA/ PEG-P[Lys(FPBA)10]20

FIG. 16

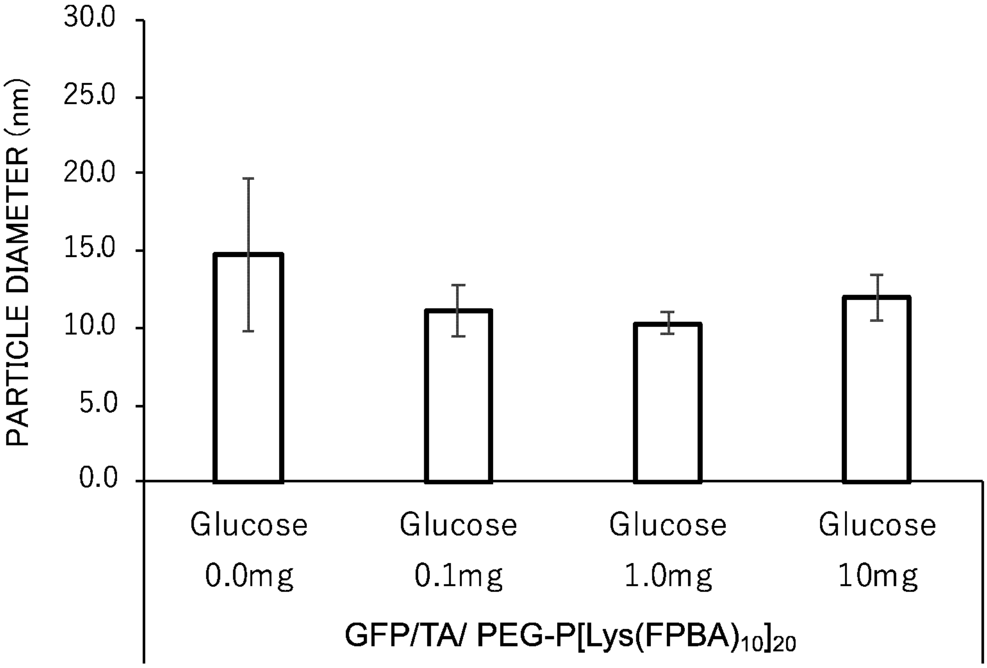
PARTICLE DIAMETER (nm)
30.0
25.0
20.0
15.0
10.0
5.0
0.0
Glucose 0.0mg
Glucose 0.1mg
Glucose 1.0mg
Glucose 10mg
GFP/TA/ PEG-P[Lys(FPBA)10]20

FIG. 17

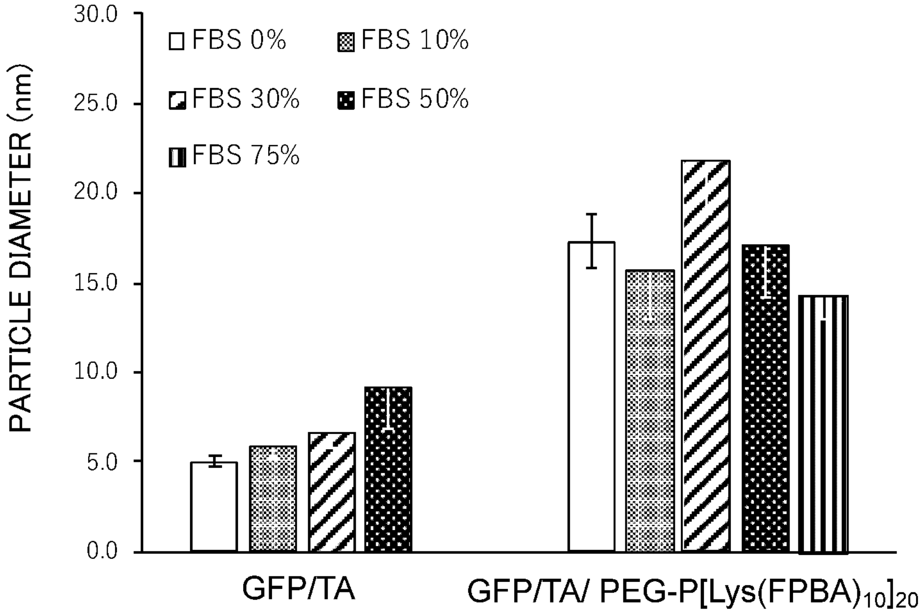
30.0
25.0
20.0
15.0
10.0
5.0
0.0
PARTICLE DIAMETER (nm)
FBS 0%
FBS 10%
FBS 30%
FBS 50%
FBS 75%
GFP/TA
GFP/TA/ PEG-P[Lys(FPBA)10]20

FIG. 18

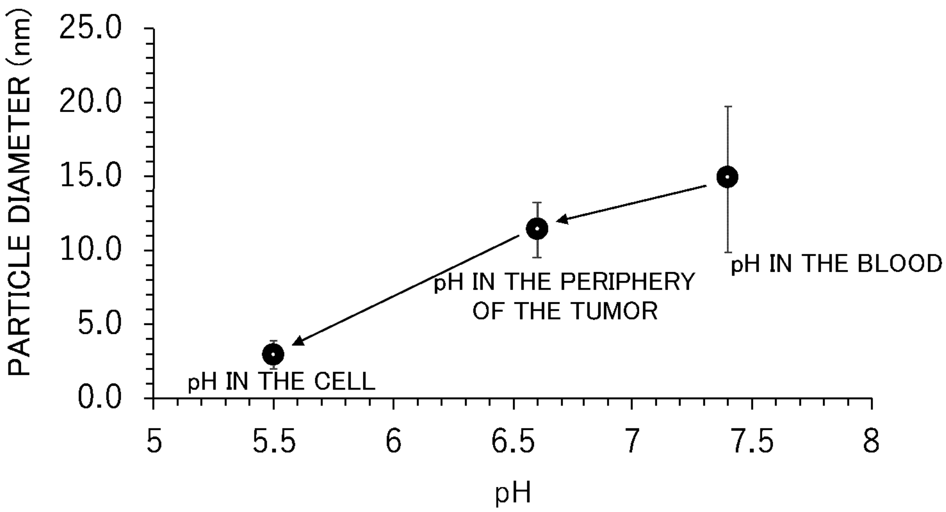
25.0
20.0
15.0
10.0
5.0
0.0
PARTICLE DIAMETER (nm)
pH IN THE BLOOD
pH IN THE PERIPHERY
OF THE TUMOR
pH IN THE CELL
5
5.5
6
6.5
7
7.5
8
pH

FIG. 19

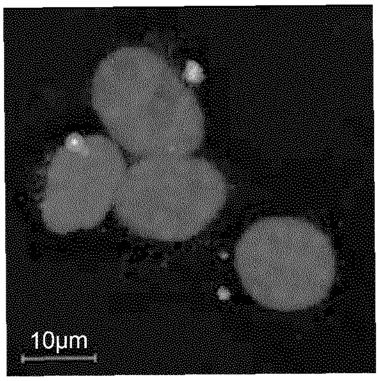
10μm

FIG. 20

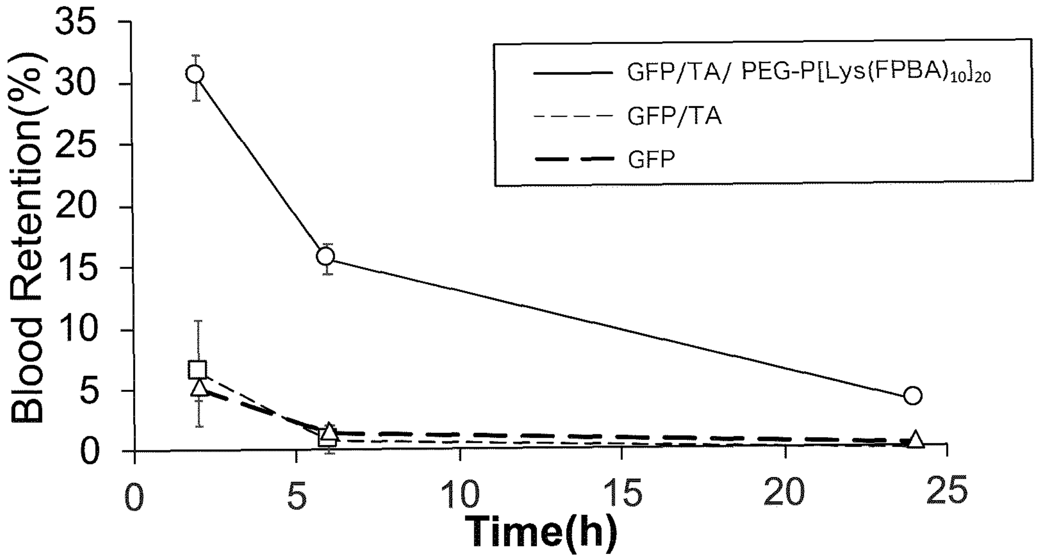
Blood Retention(%)
35
30
25
20
15
10
5
0
0
5
10
15
20
25
Time(h)
GFP/TA/ PEG-P[Lys(FPBA)10]20
GFP/TA
GFP

# FIG. 21

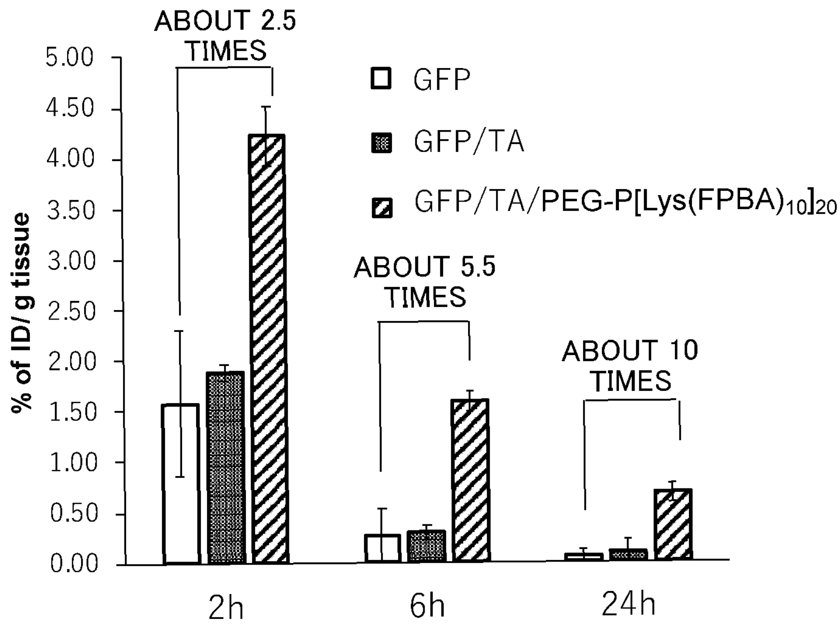
ABOUT 2.5 TIMES
ABOUT 5.5 TIMES
ABOUT 10 TIMES
5.00
4.50
4.00
3.50
3.00
2.50
2.00
1.50
1.00
0.50
0.00
% of ID/ g tissue
GFP
GFP/TA
GFP/TA/PEG-P[Lys(FPBA)10]20
2h
6h
24h

# FIG. 22

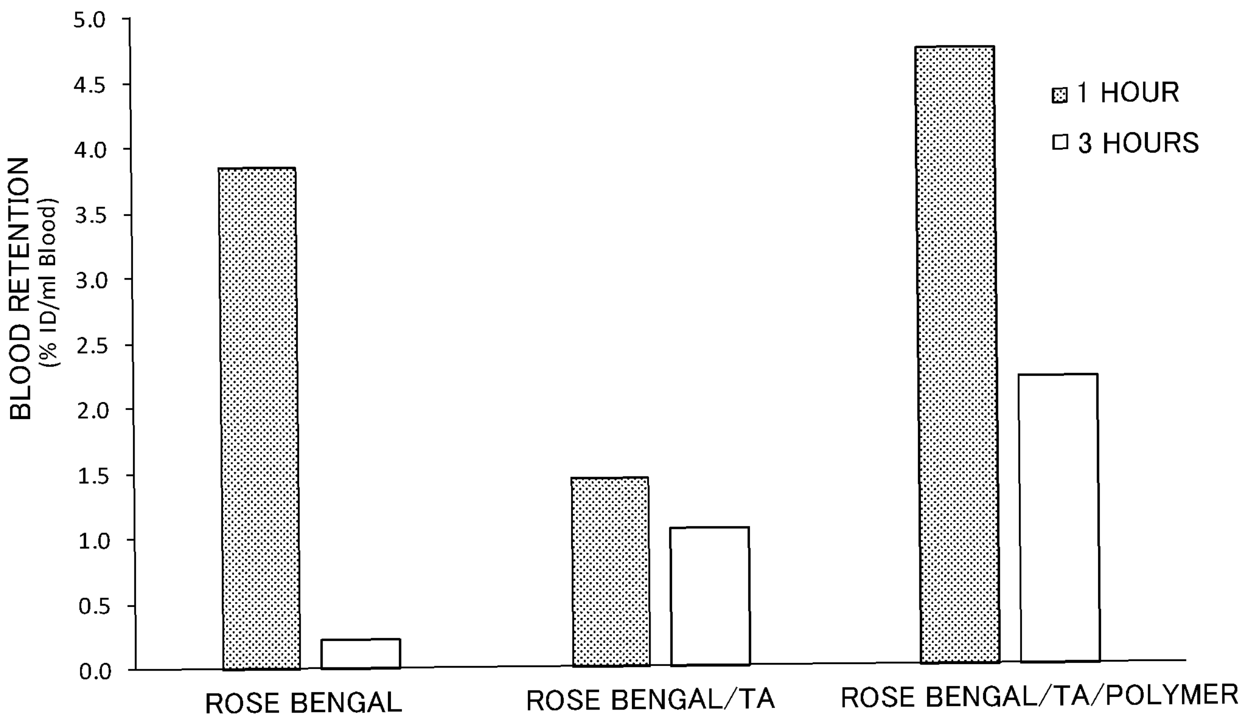
5.0
4.5
4.0
3.5
3.0
2.5
2.0
1.5
1.0
0.5
0.0
BLOOD RETENTION
(% ID/ml Blood)
1 HOUR
3 HOURS
ROSE BENGAL
ROSE BENGAL/TA
ROSE BENGAL/TA/POLYMER

FIG. 23A

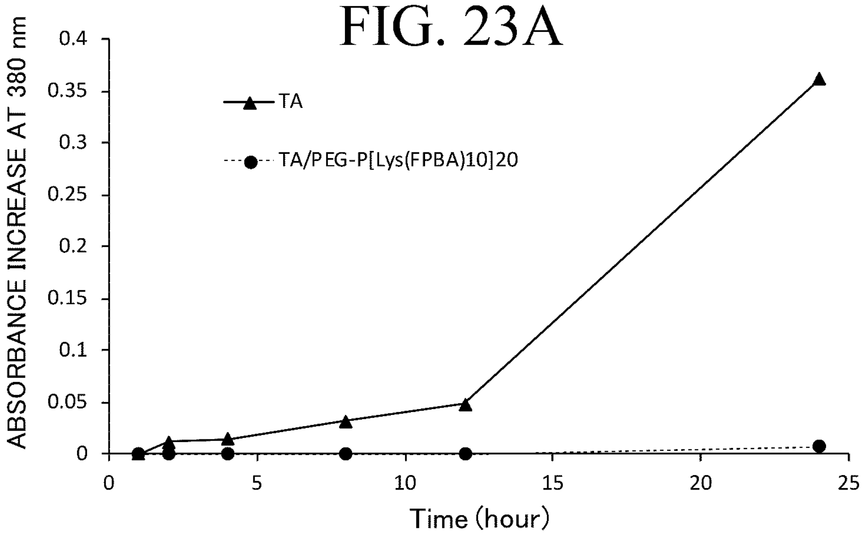


FIG. 23B

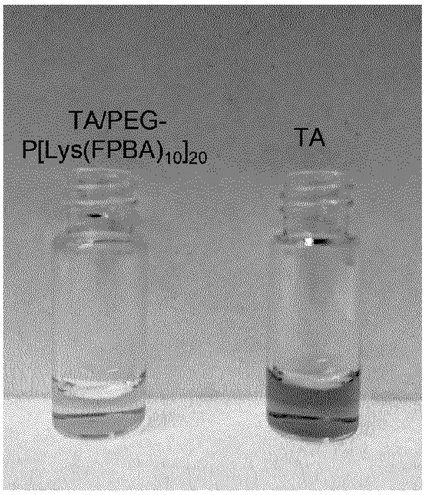


FIG. 23C

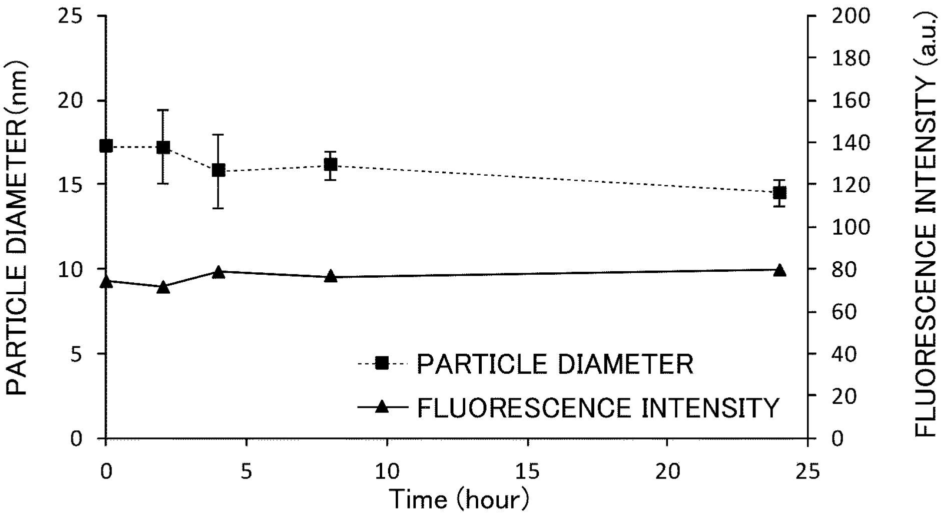

FIG. 24

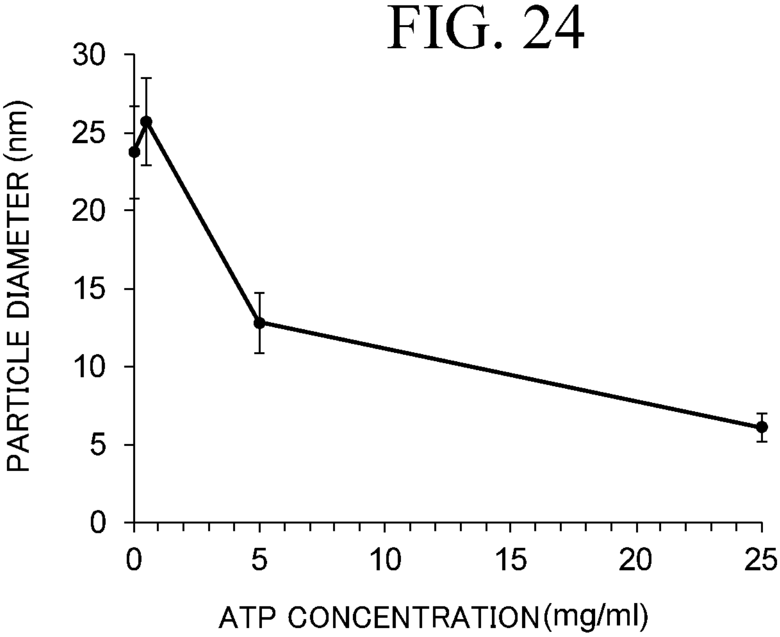
PARTICLE DIAMETER (nm)
30
25
20
15
10
5
0
0
5
10
15
20
25
ATP CONCENTRATION(mg/ml)

FIG. 25A

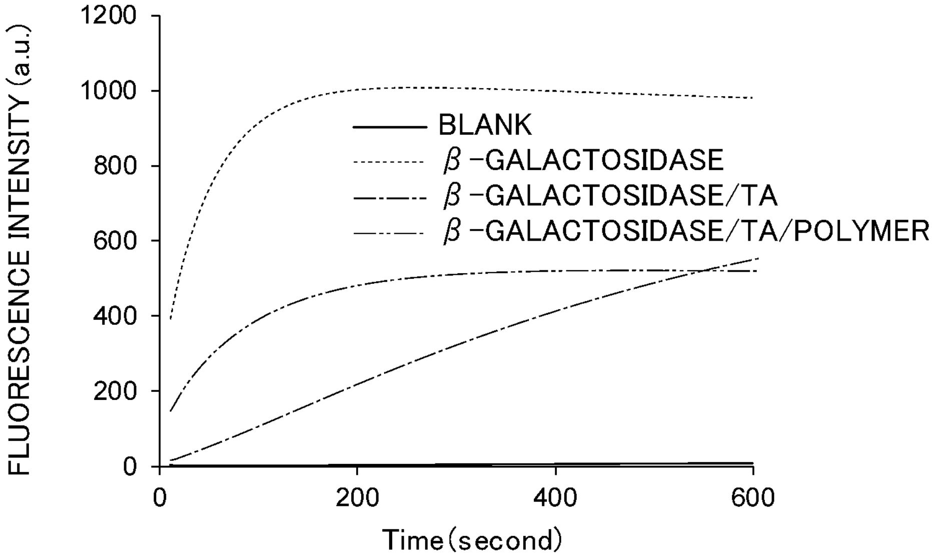


FIG. 25B

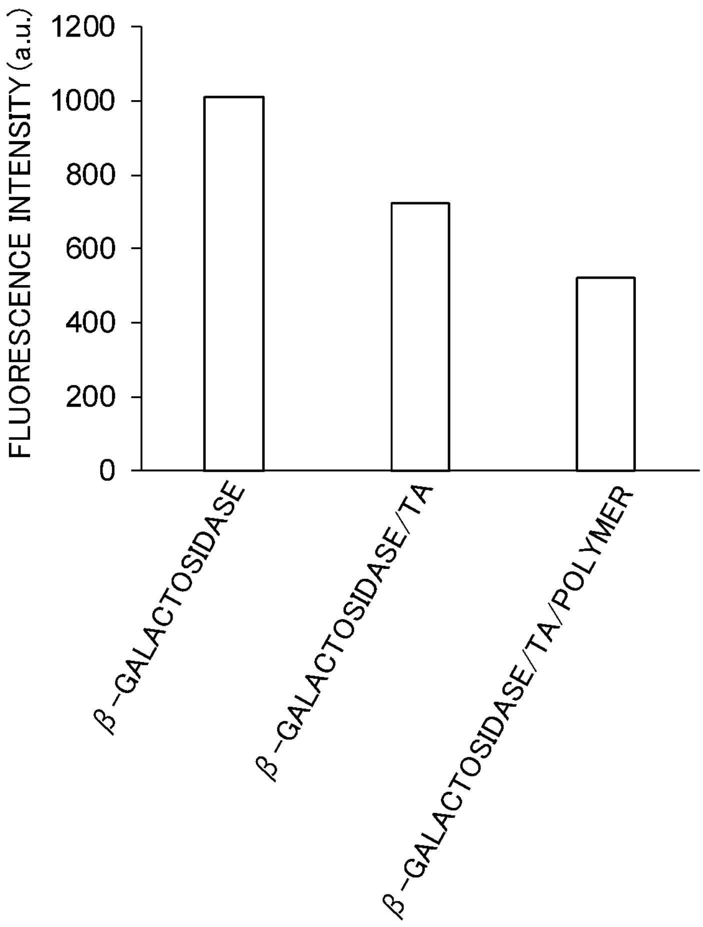

FIG. 26A

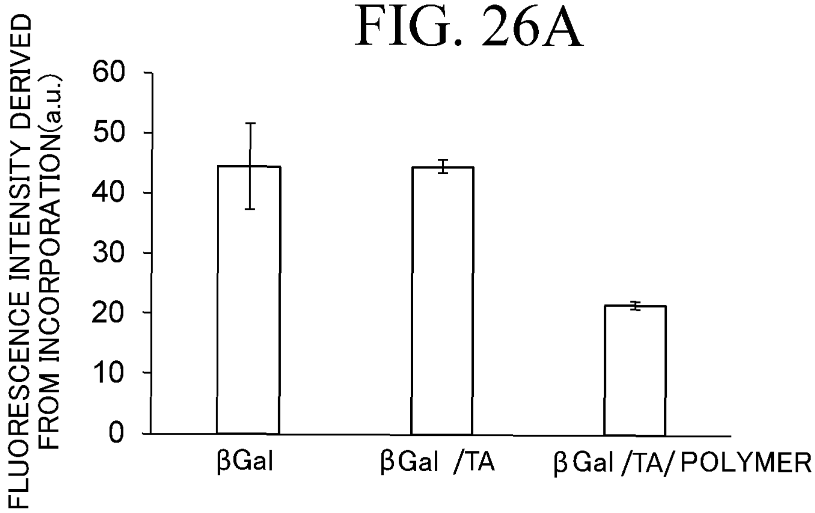


FIG. 26B

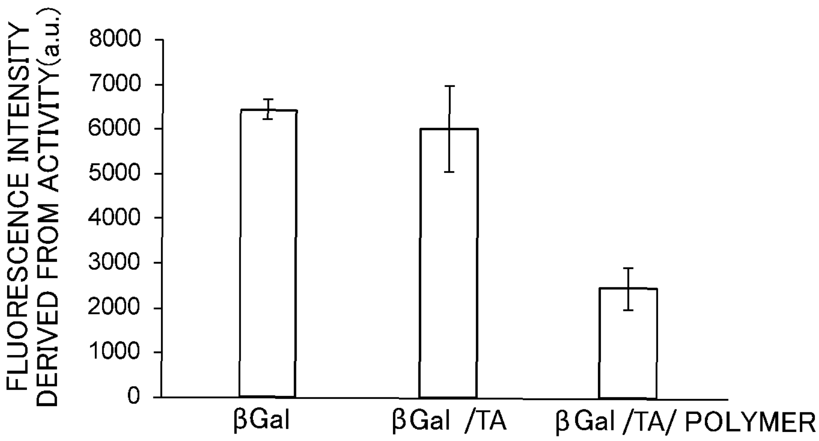


FIG. 26C

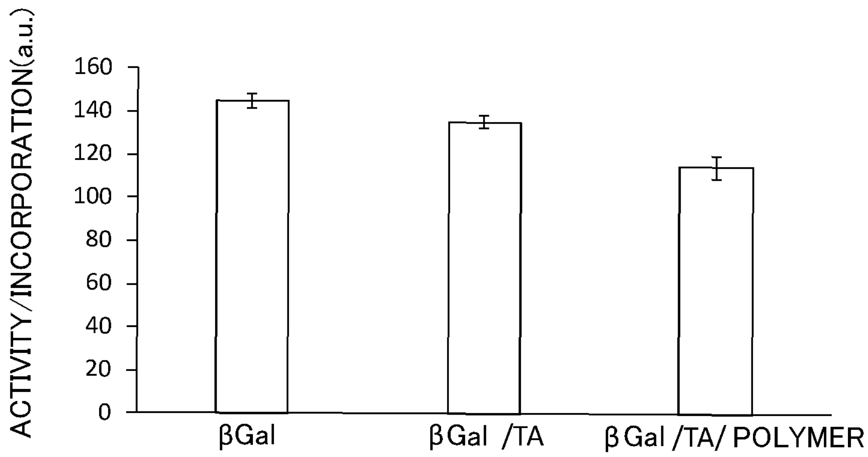

FIG. 27

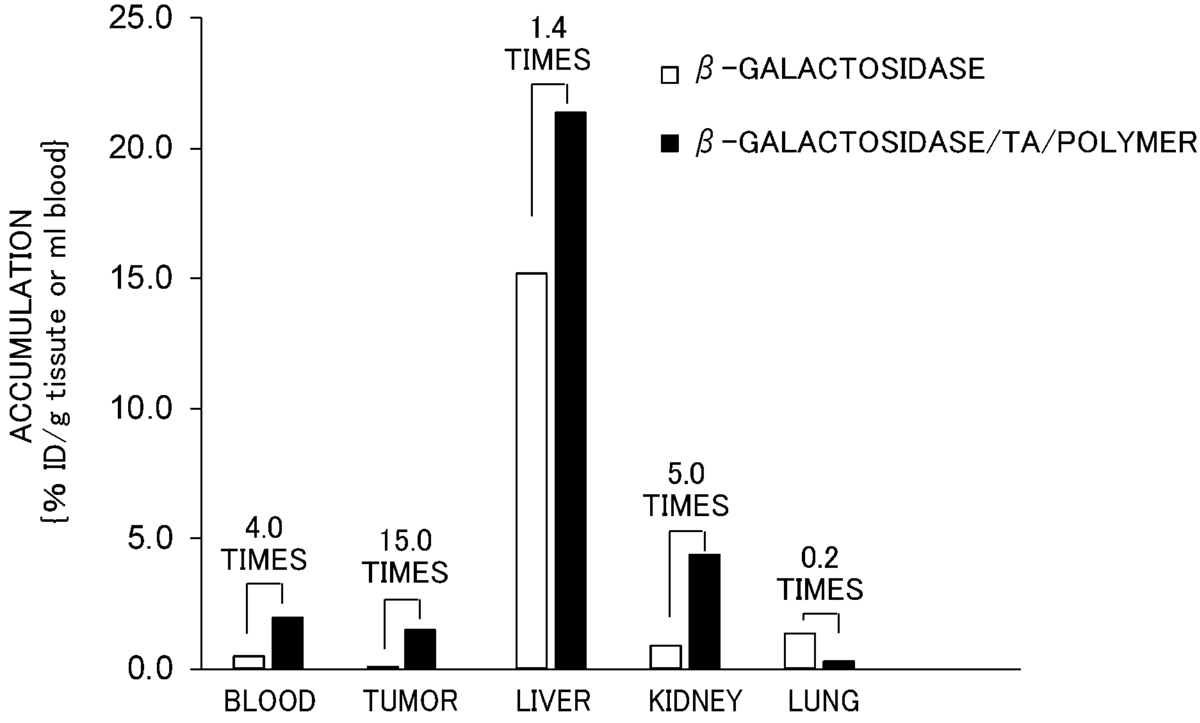
25.0
20.0
15.0
10.0
5.0
0.0
ACCUMULATION
[% ID/g tissute or ml blood]
β-GALACTOSIDASE
β-GALACTOSIDASE/TA/POLYMER
4.0 TIMES
15.0 TIMES
1.4 TIMES
5.0 TIMES
0.2 TIMES
BLOOD
TUMOR
LIVER
KIDNEY
LUNG

FIG. 28

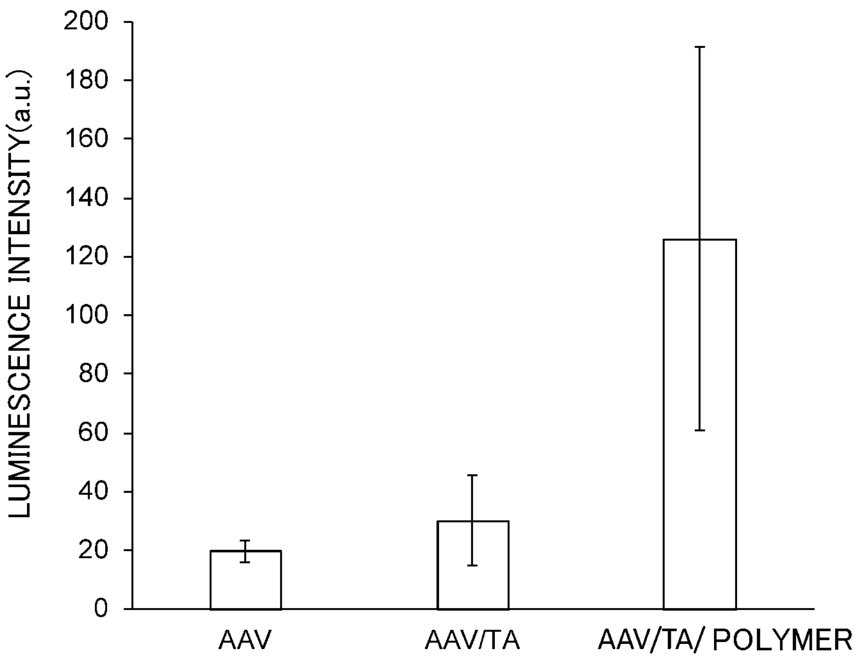
200
180
160
140
120
100
80
60
40
20
0
LUMINESCENCE INTENSITY(a.u.)
AAV
AAV/TA
AAV/TA/ POLYMER

FIG. 29

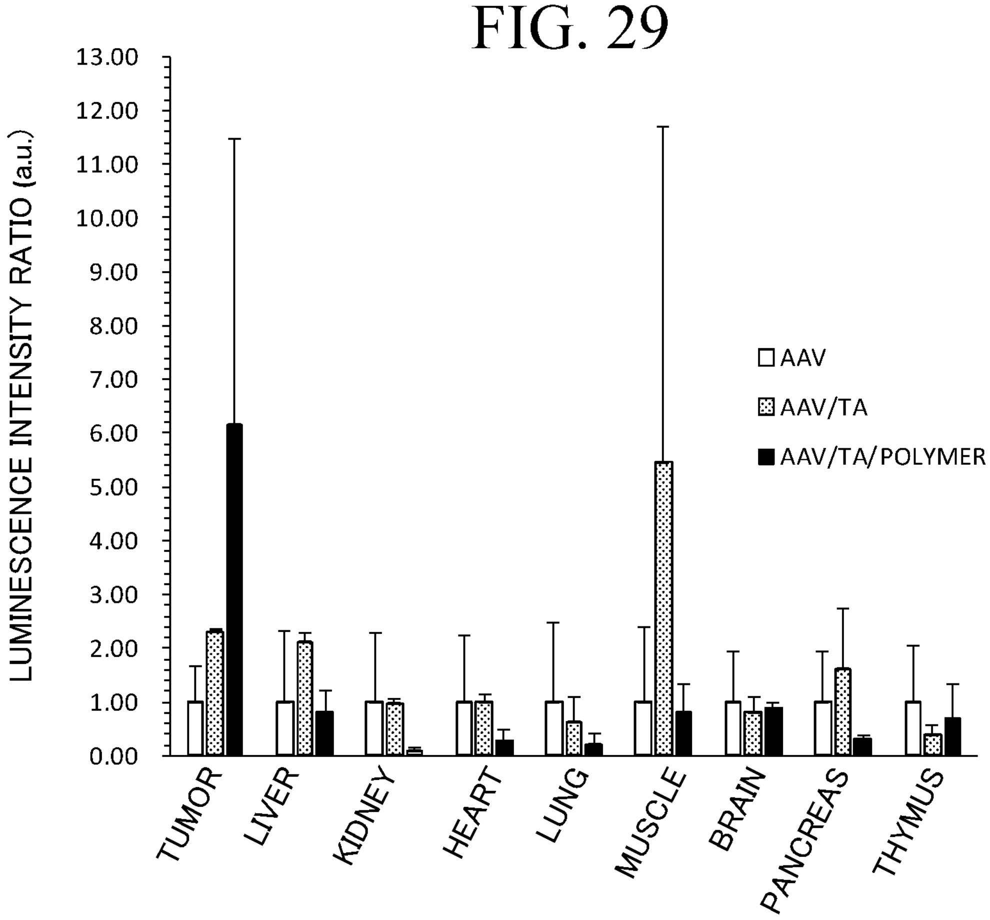
13.00
12.00
11.00
10.00
9.00
8.00
7.00
6.00
5.00
4.00
3.00
2.00
1.00
0.00
LUMINESCENCE INTENSITY RATIO (a.u.)
AAV
AAV/TA
AAV/TA/POLYMER
TUMOR
LIVER
KIDNEY
HEART
LUNG
MUSCLE
BRAIN
PANCREAS
THYMUS

FIG. 30A

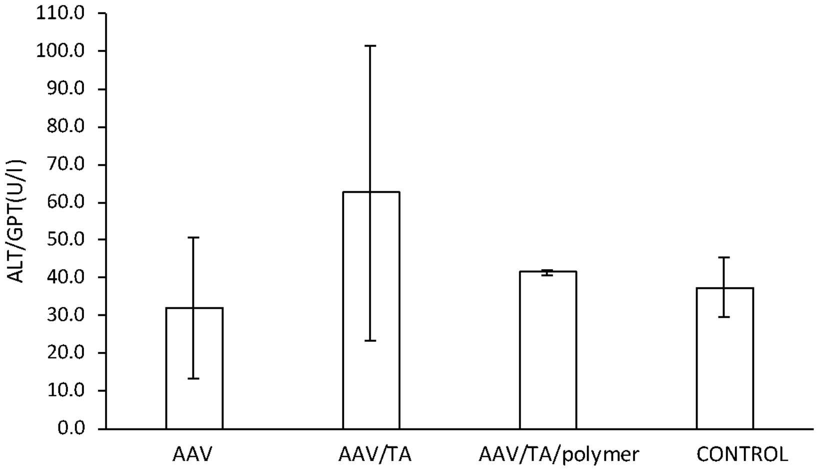


FIG. 30B

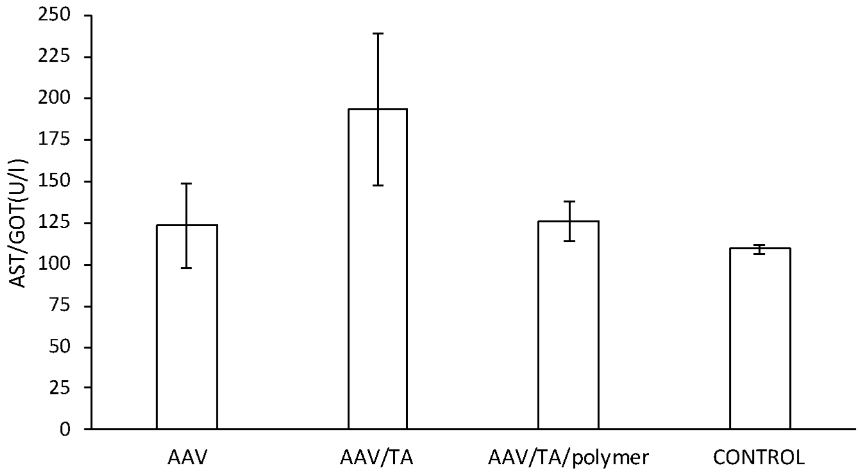

FIG. 31

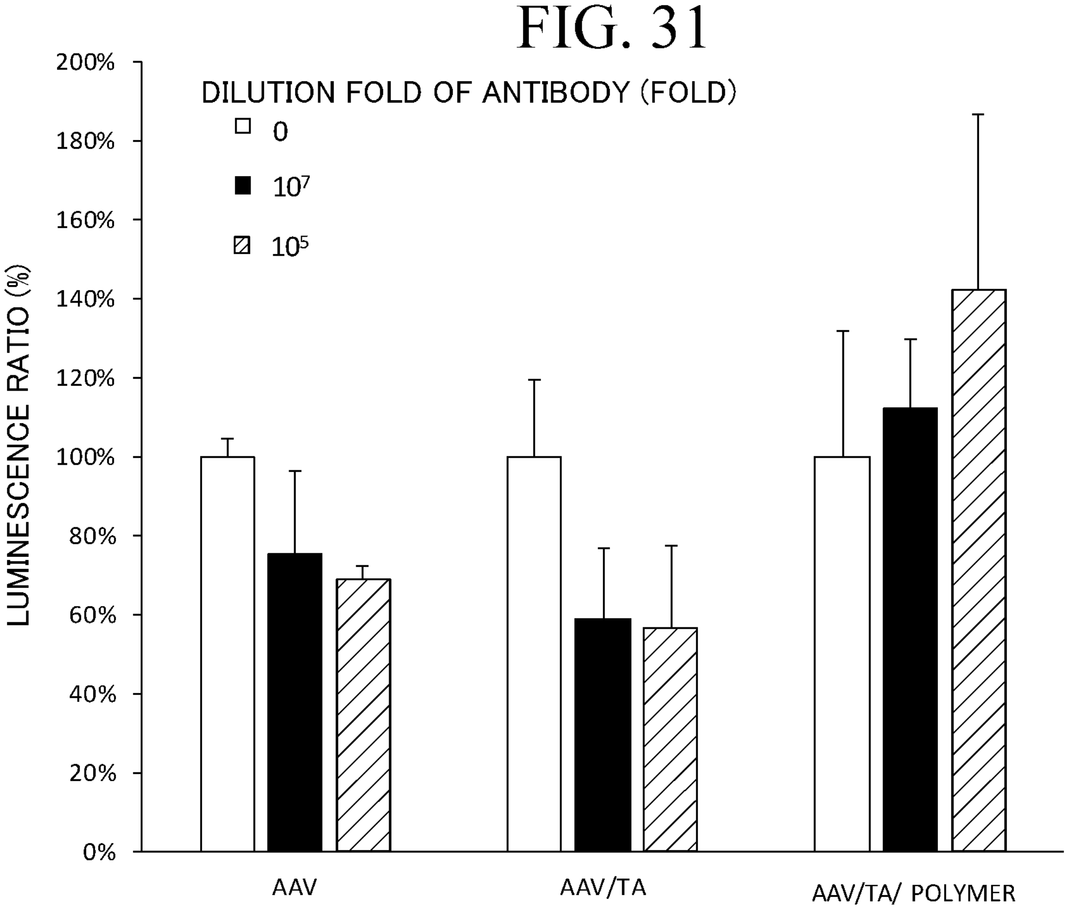
DILUTION FOLD OF ANTIBODY (FOLD)
0
$10^7$
$10^5$
LUMINESCENCE RATIO (%)
200%
180%
160%
140%
120%
100%
80%
60%
40%
20%
0%
AAV
AAV/TA
AAV/TA/ POLYMER

FIG. 32

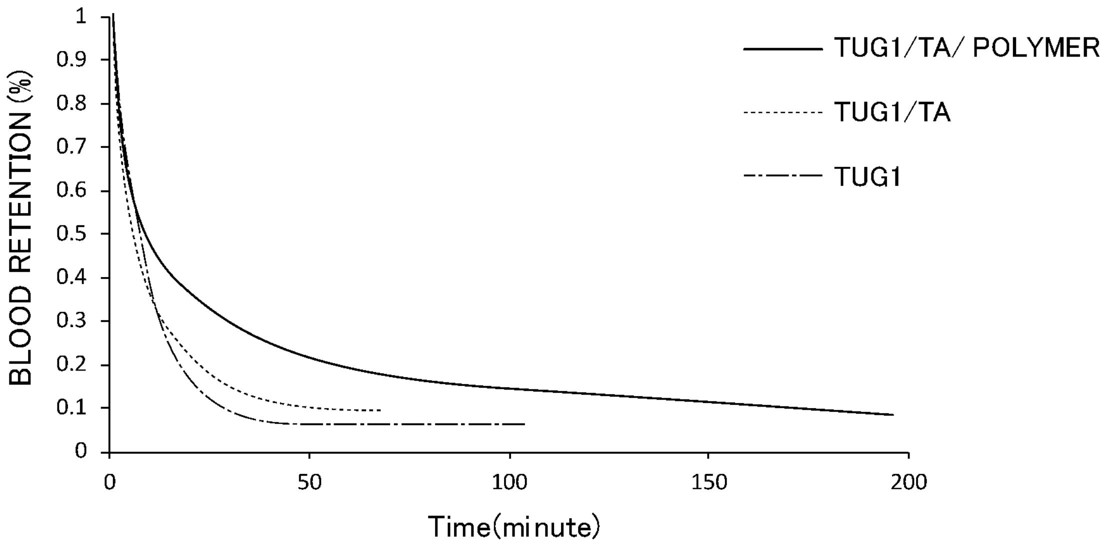
TUG1/TA/ POLYMER
TUG1/TA
TUG1
BLOOD RETENTION (%)
1
0.9
0.8
0.7
0.6
0.5
0.4
0.3
0.2
0.1
0
0
50
100
150
200
Time(minute)

**INTERNATIONAL SEARCH REPORT**

International application No.
PCT/JP2020/021301

A. CLASSIFICATION OF SUBJECT MATTER
A61P 35/00(2006.01)i; A61K 47/10(2006.01)i; A61K 47/24(2006.01)i; A61K 47/65(2017.01)i; A61K 38/16(2006.01)i
FI: A61K47/24; A61K47/65; A61K38/16; A61K47/10; A61P35/00
According to International Patent Classification (IPC) or to both national classification and IPC

B. FIELDS SEARCHED
Minimum documentation searched (classification system followed by classification symbols)
A61P35/00; A61K47/10; A61K47/24; A61K47/65; A61K38/16

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X | JP 2018-145115 A (THE UNIVERSITY OF TOKYO) 20.09.2018 (2018-09-20) examples 2-8, paragraph [0126] | 18, 19 |
| A | entire text, all drawings | 1-17 |
| A | WO 2013/073697 A1 (THE UNIVERSITY OF TOKYO) 23.05.2013 (2013-05-23) entire text, all drawings | 1-19 |
| A | WO 2015/170757 A1 (THE UNIVERSITY OF TOKYO) 12.11.2015 (2015-11-12) entire text, all drawings | 1-19 |
| A | JP 2016-517393 A (CALIFORNIA INSTITUTE OF TECHNOLOGY) 16.06.2016 (2016-06-16) entire text, all drawings | 1-19 |
| A | CHENG, Cui et al., "Phenylboronic acid-containing block copolymers: synthesis, self-assembly, and application for intracellular delivery of proteins", New Journal of Chemistry, 2012, 36, pp. 1413-1421 entire text, all drawings | 1-19 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 July 2020 (06.07.2020) | 14 July 2020 (14.07.2020) |
| Name and mailing address of the ISA/ Japan Patent Office 3-4-3, Kasumigaseki, Chiyoda-ku, Tokyo 100-8915, Japan | Authorized officer<br>Telephone No. |

Form PCT/ISA/210 (second sheet) (January 2015)

**INTERNATIONAL SEARCH REPORT**
Information on patent family members

International application No.
PCT/JP2020/021301

| Patent Documents referred in the Report | Publication Date | Patent Family | Publication Date |
|---|---|---|---|
| JP 2018-145115 A | 20 Sep. 2018 | (Family: none) | |
| WO 2103/073697 A1 | 23 May 2013 | US 2015/0051347 A1<br>entire text, all drawings<br>EP 2781536 A1<br>CN 104093768 A<br>KR 10-2014-0106511 A | |
| WO 2015/170757 A1 | 12 Nov. 2015 | EP 3141243 A1<br>entire text, all drawings | |
| JP 2016-517393 A | 16 Jun. 2016 | WO 2014/133549 A1<br>entire text, all drawings<br>EP 3513812 A1<br>CN 105189614 A<br>CN 110078915 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019100395 A
- JP 2013073697 W
- JP 2018142115 A


### Non-patent literature cited in the description


- **A. KIM ; Y. MIURA ; T. ISHII ; O. F. MUTAF ; N. NISHIYAMA ; H. CABRAL ; K. KATAOKA.** Intracellular Delivery of Charge-Converted Monoclonal Antibodies by Combinatorial Design of Block/Homo Polyion Complex Micelles. *Biomacromolecules,* 2016, vol. 17 (2), 446-453
- **J. E. CHUNG ; S. TAN ; S. J. GAO ; N. YONGVONGSOONTORN ; S. H. KIM ; J. H. LEE ; H. S. CHOI ; H. YANO ; L. ZHUO ; M. KURISAWA.** Self-assembled micellar nanocomplexes comprising green tea catechin derivatives and protein drugs for cancer therapy. *Nat. Nanotech.,* 2014, vol. 9 (11), 907-912
- **J. P. VAN BUREN ; W. B. ROBINSON.** Formation of complexes between protein and tannic acid. *J. Agric. Food Chem.,* 1969, vol. 17 (4), 772-777
- **V. SHARAD ; S. AMIT ; M. ABHA.** Gallic acid: Molecular rival of cancer. *Env. Tox. and pharm.,* 2013, vol. 35 (3), 473-485